(19) Europäisches Patentamt
European Patent Office
Office européen des brevets

(11) Veröffentlichungsnummer : **0 071 568**
**B1**

(12) # EUROPÄISCHE PATENTSCHRIFT

(45) Veröffentlichungstag der Patentschrift : **21.01.87**

(21) Anmeldenummer : **82810234.3**

(22) Anmeldetag : **28.05.82**

(51) Int. Cl.[4] : **C 07 D249/08**, C 07 D233/56, C 07 D403/12, C 07 D401/12, A 01 N 43/653, A 01 N 43/50, A 01 N 43/48

(54) **Mandelsäure-Derivate, Verfahren zu ihrer Herstellung und ihre Verwendung zur Bekämpfung von Mikroorganismen.**

(30) Priorität : **04.06.81 CH 3674/81**
**07.05.82 CH 2840/82**

(43) Veröffentlichungstag der Anmeldung : **09.02.83 Patentblatt 83/06**

(45) Bekanntmachung des Hinweises auf die Patenterteilung : **21.01.87 Patentblatt 87/04**

(84) Benannte Vertragsstaaten : **AT BE CH DE FR IT LI LU NL SE**

(56) Entgegenhaltungen :
**EP-A- 0 010 298**
**EP-A- 0 015 502**
**EP-A- 0 099 165**
**US-A- 4 167 576**

(73) Patentinhaber : **CIBA-GEIGY AG**
**Klybeckstrasse 141**
**CH-4002 Basel (CH)**

(72) Erfinder : **Kunz, Walter, Dr.**
**Im Goldbrunnen 55**
**CH-4104 Oberwil (CH)**
Erfinder : **Eckhardt, Wolfgang, Dr.**
**Breslauerstrasse 16**
**D-7850 Lörrach (DE)**

**EP 0 071 568 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Mandelsäurederivate der nachstehenden Formel I sowie deren pflanzenverträgliche Säureadditionssalze, quaternäre Azoliumsalze und Metallkomplexe. Sie betrifft ferner die Herstellung dieser Verbindungen sowie agrochemische Mittel, die als Wirkstoff mindestens eine der Verbindungen der Formel I enthalten ; sie betrifft auch die Herstellung der Mittel und ein Verfahren zur Bekämpfung oder präventiven Verhütung eines Befalls von Pflanzen durch Mikroorganismen.

Die erfindungsgemässen Verbindungen gehorchen der Formel I

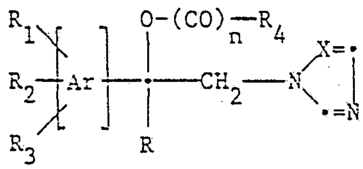

(I)

worin

X für das Brückenglied —CH= oder —N= steht ;

Ar eine Phenyl-, Diphenyl- oder Naphthylgruppe bedeutet ;

$R_1$, $R_2$, $R_3$ unabhängig voneinander für Wasserstoff, Nitro, Halogen, $C_1$-$C_3$-Alkyl, $C_1$-$C_3$-Alkoxy oder $C_1$-$C_3$-Haloalkyl stehen ;

R eine der Gruppen —$COOR_5$, oder —$COSR_6$, darstellt,

n für die Zahlen 0, 1 oder 2 steht,

wobei in den Fällen, bei denen n für die Zahl 0 steht,

$R_4$ Wasserstoff, $C_1$-$C_{12}$-Alkyl, $C_3$-$C_6$-Alkenyl, $C_3$-$C_6$-Haloalkenyl oder ein durch $C_1$-$C_4$-Alkoxy, $C_2$-$C_4$-Alkenyl, $C_2$-$C_4$-Haloalkenyl, $C_2$-$C_4$-Alkinyl, Cyano oder Phenyl substituiertes $C_1$-$C_{12}$-Alkyl bedeutet, wobei jeder $C_2$-$C_{12}$-Alkylsubstituent gegebenenfalls durch eine Carbonylgruppe oder eine Carbamoyloxygruppe (—O—CONH—) unterbrochen sein kann ;

wobei ferner in den Fällen, bei denen n für die Zahl 1 steht,

$R_4$ $C_1$-$C_{12}$-Alkyl, Phenyl, $C_1$-$C_4$-Alkoxy, $C_1$-$C_4$-Alkylthio, $C_2$-$C_6$-Alkenyl, $C_3$-$C_5$-Alkinyl, $C_2$-$C_6$-Haloalkenyl, $C_3$-$C_7$-Cycloalkyl, Furyl, Tetrahydrofuryl, Pyridyl, 1-Imidazolyl, 1-(1,2,4-Triazolyl) oder eine unsubstituierte oder durch Halogen, $C_1$-$C_4$-Alkyl, $C_1$-$C_4$-Alkoxy, —CN oder —$CF_3$ substituierte Phenylgruppe darstellt oder ein durch $C_1$-$C_4$-Alkoxy, $C_1$-$C_4$-Alkylthio, $C_2$-$C_4$-Alkenyl, $C_2$-$C_4$-Haloalkenyl, $C_2$-$C_4$-Alkinyl, Cyano oder Phenyl substituiertes $C_1$-$C_{12}$-Alkyl bedeutet, wobei jeder heterocyclische Substituent unsubstituiert oder durch Halogen und/oder Methyl ein- oder mehrfach substituiert ist und wobei ferner jeder $C_2$-$C_{12}$-Alkylsubstituent gegebenenfalls durch eine Carbonylgruppe oder eine Carbamoyloxygruppe unterbrochen sein kann ; und wobei $R_4$ ferner die Gruppe —N($C_1$-$C_8$-Alkyl)$_2$ darstellen kann ;

wobei ferner in den Fällen, bei denen n für die Zahl 2 steht,

$R_4$ $C_1$-$C_{12}$-Alkyl oder eine unsubstituierte oder durch Halogen, $C_1$-$C_4$-Alkyl, $C_1$-$C_4$-Alkoxy, —CN oder —$CF_3$ substituierte Benzylgruppe bedeutet,

und worin ferner

$R_5$ Wasserstoff, ein durch Halogen substituiertes $C_2$-$C_{10}$ Alkenyl, ein unsubstituiertes oder durch Halogen substituiertes $C_2$-$C_{10}$ Alkinyl, eine $C_3$-$C_8$ Cycloalkylgruppe, eine unsubstituierte oder durch Halogen und/oder Methyl substituierte Phenylgruppe oder eine $C_1$-$C_4$ Alkylgruppe bedeutet, oder aber eine $C_1$-$C_{12}$ Alkylkette darstellt, die ab $C_2$-Alkyl durch Sauerstoff oder Schwefel unterbrochen und/oder die durch eine der folgenden Atome oder Gruppen substituiert ist : Halogen, Phenyl, —COO Alkyl($C_1$-$C_4$), —CO Alkyl($C_1$-$C_4$), —CO Phenyl, einen ungesättigten oder gesättigten 5- oder 6-gliedrigen Ring mit Sauerstoff oder Schwefel als Heteroatom, und

$R_6$ $C_1$-$C_{10}$ Alkyl oder eine unsubstituierte oder durch Halogen, $C_1$-$C_4$-Alkyl, $C_1$-$C_4$ Alkoxy, —CN oder —$CF_3$ substituierte Phenyl- oder Benzylgruppe bedeutet, und

worin Säureadditionssalze, quaternäre Azolium- und Ammoniumsalze sowie Metallkomplexe der Formel I mit eingeschlossen sind.

Unter dem Begriff Alkyl selbst oder als Bestandteil eines anderen Substituenten sind je nach Zahl der angegebenen Kohlenstoffatome beispielsweise folgende Gruppen zu verstehen : Methyl, Ethyl, Propyl, Butyl, Pentyl, Hexyl, Heptyl, Octyl, Nonyl, Decyl, Undecyl oder Dodecyl sowie ihre Isomeren, wie z. B. Isopropyl, Isobutyl, tert.-Butyl, Isopentyl usw. Alkenyl steht z. B. für Vinyl, Propenyl-(1), Allyl, Butenyl-(1), Butenyl-(2), Butenyl-(3) usw., sowie Ketten mit mehreren Doppelbindungen. Alkinyl bedeutet z. B. Propinyl-(1), Propargyl, Butinyl-(1), Butinyl-(2) usw., bevorzugt Propargyl. Haloalkyl steht für einen ein- bis perhalogenierten Alkylsubstituierten, wie z. B. $CHCl_2$, $CH_2Cl$, $CCl_3$, $CF_3$, $CH_2CH_2Cl$, usw. Unter Halogen soll hier und im folgenden Fluor, Chlor, Brom oder Jod, vorzugsweise Chlor oder Fluor verstanden werden. Cycloalkyl seht z. B. für Cyclopropyl, Cyclobutyl, Cyclopentyl, Cyclohexyl oder Cycloheptyl, bevorzugt für Cyclopropyl und Cyclohexyl. Haloalkenyl steht für eine ein- oder mehrfach durch Halogen substituierte Alkenylgruppe, wobei als Halogen Chlor und Brom, insbesondere Chlor bevorzugt sind.

Furyl steht bevorzugt für 2-Furyl, Tetrahydrofuryl, bevorzugt für 2-Tetrahydrofuryl. Pyridyl bedeutet vor allem 3- oder 4-Pyridyl. Naphthyl steht für α- oder β-Naphthyl, insbesondere für α-Naphthyl.

Beispiele salzbildender Säuren sind anorganische Säuren : Halogenwasserstoffsäure wie Fluorwasserstoffsäure, Chlorwasserstoffsäure, Bromwasserstoffsäure oder Jodwasserstoffsäure sowie Schwefelsäure, Phosphorsäure, phosphorige Säure, Salpetersäure und organische Säuren wie Essigsäure, Trifluoressigsäure, Trichloressigsäure, Propionsäure, Glycolsäure, Thiocyansäure, Milchsäure, Bernsteinsäure, Zitronensäure, Benzoesäure, Zimtsäure, Oxalsäure, Ameisensäure, Benzolsulfonsäure, p-Toluolsulfonsäure, Methansulfonsäure, Salicylsäure, p-Aminosalicylsäure, 2-Phenoxybenzoesäure oder 2-Acetoxybenzoesäure.

Metallkomplexe der Formel I bestehen aus dem zugrundeliegenden organischen Molekül und einem anorganischen oder organischen Metallsalz, beispielsweise den Halogeniden, Nitraten, Sulfaten, Phosphaten, Acetaten, Trifluoracetaten, Trichloracetaten, Propionaten, Tartraten, Sulfonaten, Salicylaten, Benzoaten usw. der Elemente der dritten und vierten Hauptgruppe wie Aluminium, Zinn oder Blei sowie der ersten bis achten Nebengruppe wie Chrom, Mangan, Eisen, Kobalt, Nickel, Kupfer, Zink, Silber, Quecksilber usw. Bevorzugt sind die Nebengruppen-Elemente der 4. Periode. Die Metalle können dabei in den verschiedenen ihnen zukommenden Wertigkeiten vorliegen. Die Metallkomplexe der Formel I können ein- oder mehrkernig auftreten, d. h. sie können ein oder mehrere organische Molekülanteile als Liganden enthalten. Komplexe mit den Metallen Kupfer, Zink, Mangan und Zinn sind bevorzugt.

Die Verbindungen der Formel I sind bei Raumtemperatur stabile Oele, Harze oder überwiegend Feststoffe, die sich durch sehr wertvolle mikrobizide Eigenschaften auszeichnen. Sie lassen sich auf dem Agrarsektor oder verwandten Gebieten präventiv und kurativ zur Bekämpfung von pflanzenschädigenden Mikroorganismen einsetzen, dabei sind die Triazolylmethylderivate im Umfang der Formel I (X bedeutet N) bevorzugt. Die erfindungsgemässen Wirkstoffe der Formel I zeichnen sich durch eine sehr gute Pflanzenverträglichkeit aus. Die Entwicklung der Pflanzen wird in keinem Stadium behindert oder verzögert.

Ein Teilbereich vorliegender Erfindung betrifft Verbindungen der Formel I*

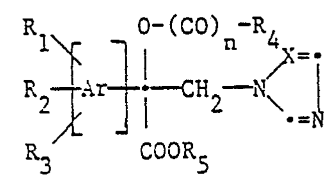

(I*)

worin

X für das Brückenglied —CH= oder —N= steht ;

Ar eine Phenyl-, Diphenyl- oder Naphthylgruppe bedeutet ;

$R_1$, $R_2$, $R_3$ unabhängig voneinander für Wasserstoff, Nitro, Halogen, $C_1$-$C_3$-Alkyl, $C_1$-$C_3$-Alkoxy oder $C_1$-$C_3$-Haloalkyl stehen ;

n für die Zahlen 0 oder 1 steht und in den Fällen, in denen

n für die Zahl 0 steht,

$R_4$ Wasserstoff, $C_1$-$C_{12}$-Alkyl, $C_3$-$C_6$-Alkenyl, $C_3$-$C_6$-Haloalkenyl oder ein durch $C_1$-$C_4$-Alkoxy, $C_2$-$C_4$-Alkenyl, $C_2$-$C_4$-Haloalkenyl, $C_2$-$C_4$-Alkinyl, Cyano oder Phenyl substituiertes $C_1$-$C_{12}$-Alkyl bedeutet, wobei jeder $C_2$-$C_{12}$-Alkylsubstituent gegebenenfalls durch eine Carbonylgruppe unterbrochen ist ;

und in den Fällen, in denen n für die Zahl 1 steht,

$R_4$ $C_1$-$C_{12}$-Alkyl, Phenyl, $C_1$-$C_4$-Alkoxy, $C_2$-$C_6$-Alkenyl, $C_3$-$C_5$-Alkinyl, $C_2$-$C_6$-Haloalkenyl, $C_3$-$C_7$-Cycloalkyl, Furyl, Tetrahydrofuryl, Pyridyl oder ein durch $C_1$-$C_4$-Alkoxy, $C_2$-$C_4$-Alkenyl, $C_2$-$C_4$-Haloalkenyl, $C_2$-$C_4$-Alkinyl, Cyano oder Phenyl substituiertes $C_1$-$C_{12}$-Alkyl bedeutet, wobei jeder cyclische Substituent unsubstituiert oder durch Halogen und/oder Methyl ein- oder mehrfach substituiert ist und wobei ferner jeder $C_2$-$C_{12}$-Alkylsubstituent gegebenenfalls durch eine Carbonylgruppe unterbrochen ist ; und

$R_5$ Wasserstoff, $C_1$-$C_4$-Alkyl, Phenyl, ein durch Nitro, Halogen und/oder Methyl ein- oder mehrfach substituiertes Phenyl oder Benzyl bedeutet ; unter Einschluss der pflanzenverträglichen Säureadditionsalze, quaternären Azoliumsalze sowie Metallkomplexe der Verbindungen der Formel I*.

Eine wichtige Gruppe von Verbindungen sind die an der Hydroxylgruppe unsubstituierten Mandelsäurederivate und Mandelonitrile der Formel I, bei denen X, Ar, R, $R_1$, $R_2$, $R_3$ die für Formel I angegebenen Bedeutungen haben, während n = 0 und $R_4$ = Wasserstoff bedeuten.

Innerhalb dieser letztgenannten Gruppe sind solche Verbindungen von besonderer Bedeutung, bei denen Ar eine Phenylgruppe bedeutet und R eine der Gruppen —$COOR_5$ oder —$COSR_6$ darstellt, wobei X, $R_1$, $R_2$, $R_3$ $R_5$ und $R_6$ die für Formel I gegebene Bedeutung haben.

Unter den letztgenannten Verbindungen sind solche besonders bevorzugt, bei denen Ar Phenyl bedeutet, $R_1$ für Halogen, Methyl, Methoxy oder Trifluormethyl steht und $R_2$ und $R_3$ unabhängig voneinander Wasserstoff, Fluor, Chlor, Brom, Methyl oder Methoxy darstellen, während X und $R_5$ und $R_6$ die unter Formel I gegebene Bedeutung besitzen.

Unter diesen wiederum sind solche Verbindungen bevorzugt, bei denen $R_5$ Wasserstoff ; $C_1$-$C_4$-Alkyl ; eine unsubstituierte oder durch Halogen, $C_1$-$C_4$ Alkyl, $C_1$-$C_4$ Alkoxy, —CN oder —$CF_3$ substituierte Phenylgruppe bedeutet ; und $R_6$ $C_1$-$C_6$ Alkyl oder eine Phenyl- oder Benzylgruppe darstellt. Diese letztgenannte Gruppe soll Untergruppe T genannt werden.

Eine bevorzugte Gruppe von mikrobiziden Wirkstoffen besteht aus Verbindungen der Formel I*, worin X für —CH= oder —N= steht ; Ar Phenyl bedeutet ; $R_1$, $R_2$, $R_3$ unabhängig voneinander für Halogen, Methyl, Methoxy oder Trifluormethyl stehen, n für die Zahl 0 oder 1 steht und $R_4$ sowie $R_5$ die unter Formel I* angegebenen Bedeutungen haben.

Innerhalb dieser Gruppe sind besonders solche Verbindungen der Formel I* bevorzugt, worin X für —N= steht ; $R_1$, $R_2$, $R_3$ unabhängig voneinander für Fluor, Chlor, Brom, Methyl, Methoxy oder Trifluormethyl stehen, n die Zahl 0 bedeutet, $R_4$ für Wasserstoff, $C_1$-$C_6$-Alkyl, $C_3$-$C_6$-Alkenyl oder $C_3$-$C_6$-Haloalkenyl steht und $R_5$ die unter Formel I* angegebenen Bedeutungen hat.

Eine besonders bevorzugte Gruppe bilden jene Verbindungen der Formel I*, worin X für —N= steht ; Ar eine Phenylgruppe bedeutet, zwei der Substituenten $R_1$, $R_2$, $R_3$ für Halogen und/oder Methyl stehen, während der dritte Wasserstoff bedeutet ; $R_4$ für Wasserstoff oder $C_1$-$C_3$-Alkyl steht und $R_5$ Wasserstoff, $C_1$-$C_3$-Alkyl, Phenyl oder Benzyl bedeutet.

Eine weitere bevorzugte Gruppe von Mikrobiziden besteht aus Verbindungen der Formel I*, worin X, Ar, $R_1$, $R_2$, $R_3$ und $R_5$ die unter Formel I* angegebenen Bedeutungen haben, $R_4$ für Wasserstoff oder $C_1$-$C_3$-Alkyl steht und n die Zahl 0 bedeutet.

Eine besonders bevorzugte Gruppe von Mikrobiziden bilden Verbindungen der Formel I*, worin Ar für 2,4-Dihalogenphenyl, 4-Halogenphenyl, 2-($C_1$-$C_2$-Alkyl)-4-halophenyl oder 2-($CF_3$)-4-Halophenyl steht ; n die Zahl 0 oder 1 bedeutet ; X für —N= steht ; $R_4$ Wasserstoff, $C_1$-$C_4$-Alkyl, Cyclopropyl, Cyclohexyl, $C_2$-$C_3$-Alkenyl oder $C_2$-$C_3$-Haloalkenyl bedeutet und $R_5$ für $C_1$-$C_4$-Alkyl, 2,4-Dihalophenyl, 4-Halophenyl oder Phenyl steht. Als Substituenten in den genannten Phenylteilen sind dabei Fluor, Chlor, Brom, Methyl, Methoxy und Trifluormethyl besonders bevorzugt.

Bevorzugt sind auch Mikrobizide der Formel I*, worin X für —N= steht, $R_1$, $R_2$, $R_3$ unabhängig voneinander für Fluor, Chlor, Methyl, Methoxy oder Trifluormethyl stehen, n die Zahl 1 bedeutet, $R_4$ für Wasserstoff, $C_1$-$C_6$-Alkyl, Phenyl, $C_1$-$C_3$-Alkoxy, Benzyl, $C_3$-$C_6$-Cycloalkyl, 2-Furyl, 2-Tetrahydrofuryl, 3-Pyridyl, 4-Pyridyl, 5-Chlor-2-furyl oder Halophenyl steht und Ar und $R_5$ die unter Formel I* angegebenen Bedeutungen haben.

Interessant sind ferner Verbindungen der Formel I*, worin n die Zahl 1 bedeutet, X für —CH= oder —N= steht ; Ar Diphenyl bedeutet ; $R_1$, $R_2$, $R_3$ unabhängig voneinander für Halogen, Methyl oder Methoxy stehen ; n die Zahl 0 oder 1 bedeutet, $R_4$ für Wasserstoff, $C_1$-$C_3$-Alkyl, Phenyl, $C_1$-$C_3$-Alkoxy, $C_3$-$C_6$-Cycloalkyl oder Furyl bedeutet und $R_5$ Wasserstoff, $C_1$-$C_3$-Alkyl, Phenyl oder Halophenyl bedeutet.

Folgende Einzelsubstanzen sind als Mikrobizide besonders bevorzugt :

2-(1H-1,2,4-Triazolylmethyl-1'-yl)-2-hydroxy-2,4-dichlorphenylessigsäuremethylester, (Verb. Nr. 1.1)

2-(1H-1,2,4-Triazolylmethyl-1'-yl)-2-hydroxy-2,4-dichlorphenylessigsäureethylester, (Verb. Nr. 1.9)

2-(1H-1,2,4-Triazolylmethyl-1'-yl)-2-hydroxy-2,4-dichlorphenylessigsäure-n-propylester, (Verb. Nr. 1.10)

2-(1H-1,2,4-Triazolylmethyl-1'-yl)-2-hydroxy-2,4-dichlor-phenylessigsäure-n-butylester, (Verb. Nr. 1.14)

2-(1H-1,2,4-Triazolylmethyl-1'-yl)-2-ethylthiocarbonyloxy-2-(2'-chlor-4'-fluorphenyl)-essigsäureethylester, (Verb. Nr. 2.161)

2-(1H-1,2,4-Triazolylmethyl-1'-yl)-2-hydroxy-2-chlor-4-bromphenylessigsäuremethylester, (Verb. Nr. 1.27)

2-(1H-1,2,4-Triazolylmethyl-1'-yl)-2-hydroxy-2-chlor-4-bromphenylessigsäureethylester, (Verb. Nr. 1.29)

2-(1H-1,2,4-Triazolylmethyl-1'-yl)-2-ethoxy-2,4-dichlorphenylessigsäureethylester, (Verb. Nr. 3.3)

2-(1H-1,2,4-Triazolylmethyl-1'-yl)-2-hydroxy-2,4-dichlorphenylessigsäure-tert. butylester. (Verb. Nr. 1.24)

2-(1H-1,2,4-Triazolylmethyl-1'-yl)-2-hydroxy-2-(2'-chlor-4'-bromphenyl)-essigsäure-β-methoxyethylester, (Verb. Nr. 1.32)

2-(1H-1,2,4-Triazolylmethyl-1'-yl)-2-hydroxy-2,4-dichlorphenylessigsäure-allylester, (Verb. Nr. 1.40)

2-(1H-1,2,4-Triazolylmethyl-1'-yl)-2-hydroxy-2-(2'-chlor-4'-bromphenyl)-essigsäure-isopropylester, (Verb. Nr. 1.55)

2-(1H-1,2,4-Triazolylmethyl-1'-yl)-2-hydroxy-2-(2'-chlor-4'-fluorphenyl)-essigsäure-ethylester, (Verb. Nr. 1.101)

2-(1H-1,2,4-Triazolylmethyl-1'-yl)-2-hydroxy-2,4-dichlorphenylessigsäure-methylthiomethylester, (Verb. Nr. 1.116)

2-(1H-1,2,4-Triazolylmethyl-1'-yl)-2-acetoxy-2,4-dichlorphenylessigsäure-methylester, (Verb. Nr. 2.2)

2-(1H-1,2,4-Triazolylmethyl-1'-yl)-2-acetoxy-2-(2'-chlor-4'-bromphenyl)-essigsäure-ethylester, (Verb. Nr. 2.66)

2-(1H-1,2,4-Triazolylmethyl-1'-yl)-2-ethoxycarbonyloxy-2,4-dichlorphenylessigsäure-methylester, (Verb. Nr. 2.109)

2-(1H-1,2,4-Triazolylmethyl-1'-yl)-2-ethoxycarbonyloxy-2-(2'-chlor-4'-bromphenyl)-essigsäure-ethylester, (Verb. Nr. 2.132)

2-(1H-1,2,4-Triazolylmethyl-1'-yl)-2-ethoxycarbonyloxy-2-(2'-chlor-4'-fluorphenyl)-essigsäure-methylester. (Verb. Nr. 2.157)

Die Verbindungen der Formel I können nach einer ganzen Reihe von Reaktionsvarianten A bis G, wie anschliessend in zwei Reaktionsschemen skiziert und darauffolgend im einzelnen aufgeführt, hergestellt werden. In den Formeln Ia, Ib, Ic, Id, II, III, IV, V, VI, VII, VIII, IX, XI, XII und XIII haben die Substituenten Ar, X, n, $R_1$, $R_2$, $R_3$, $R_4$ und $R_5$ die unter Formel I angegebenen Bedeutungen.

$R_6$* und $R_7$* stehen für Organyl-reste, vorzugsweise für unsubstituiertes oder substituiertes $C_1$-$C_8$-Alkyl oder für Phenyl oder substituiertes Phenyl. Q steht in Formel VI entweder für eine der üblichen Abgangsgruppen, z. B. Halogen, insbesondere Chlor, Brom oder Jod ; für eine Sulfonyloxygruppe, insbesondere Benzolsulfonyloxy, Paratosyloxy oder Niederalkylsulfonyloxy, bevorzugt Mesyloxy ; oder für eine Acyloxygruppe wie Trifluoracetyloxy. Q steht auch für eine Hydroxygruppe oder gemäss « Synthesis » 1979, p. 561-569, für den Rest

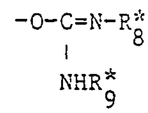

worin $R_8$* und $R_9$* Organylreste, insbesondere Niederalkyl oder gegebenenfalls substituierte Phenylreste repräsentieren. M steht für Wasserstoff oder ein Metallatom, insbesondere für ein Alkalimetallatom, vorzugsweise für Natrium oder Kalium. Hal steht für Halogen, bevorzugt für Chlor oder Brom. Y bedeutet Halogen, bevorzugt Chlor oder Brom oder steht für eine Sulfat- oder Sulfonsäureestergruppe.

Das Symbol α steht stellvertretend für die Gruppierung

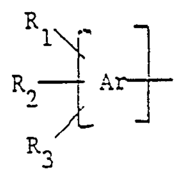

worin die Substituenten $R_1$, $R_2$, $R_3$ und Ar wie unter Formel I definiert sind.

Az repräsentiert die nachfolgende Azolylgruppe

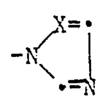

worin X für —CH= oder —N= steht.

(Siehe Schema Seite 6 f.)

Zur Herstellung der Verbindungen der Formel I kann man im einzelnen wie folgt vorgehen :

i) Freie α-Hydroxycarbonsäuren (= Mandelsäuren) der Formel Ia sind besonders bevorzugt und werden dadurch hergestellt, dass man wahlweise, entweder nach Gleichung A ein Dioxolanon der Formel II oder nach Gleichung B ein Cyanhydrin der Formel III (im Umfang der Formel I) basisch oder sauer hydrolysiert.

Die Hydrolysereaktionen A und B werden mit Säuren oder Basen vorteilhafterweise in wässrigen und/oder alkoholischen Lösungen, also in polaren Lösungsmitteln, durchgeführt. Die Reaktionen können auch in zweiphasigen Medien duchgeführt werden. Hierbei ist die Zugabe eines üblichen Phasentransfer-Katalysators vorteilhaft. Es kommen anorganische und organische Säuren in Frage, beispielsweise Mineralsäuren wie Halogenwasserstoffsäuren, Schwefelsäure, Phosphorsäure oder Sulfonsäuren (p-Toluolsulfonsäure, Methansulfonsäure). Als Basen eignen sich organische und anorganische Basen, beispielsweise Oxide, Hydride, Hydroxide, Carbonate, Carbonsäuresalze und Alkoholate der Erdalkali- und der Alkali-Metalle, insbesondere der des Natriums und Kaliums.

Die Reaktionstemperaturen liegen bei der Ringöffnungsreaktion A im allgemeinen zwischen 0° und + 140 °C, bevorzugt zwischen + 30° und + 80 °C und bei der Hydrolyse des Cyanhydrins III zwischen + 60° und + 140 °C, bevorzugt + 80° und + 120 °C oder jeweils am Siedepunkt des Lösungsmittels oder Lösungsmittelgemisches.

Ausgangsverbindungen der Formel II sind grösstenteils aus der EP-OS Nr. 44 276 bekannt. Die noch neuen Verbindungen werden analog den dort angegebenen Methoden hergestellt.

1. Reaktionsschema

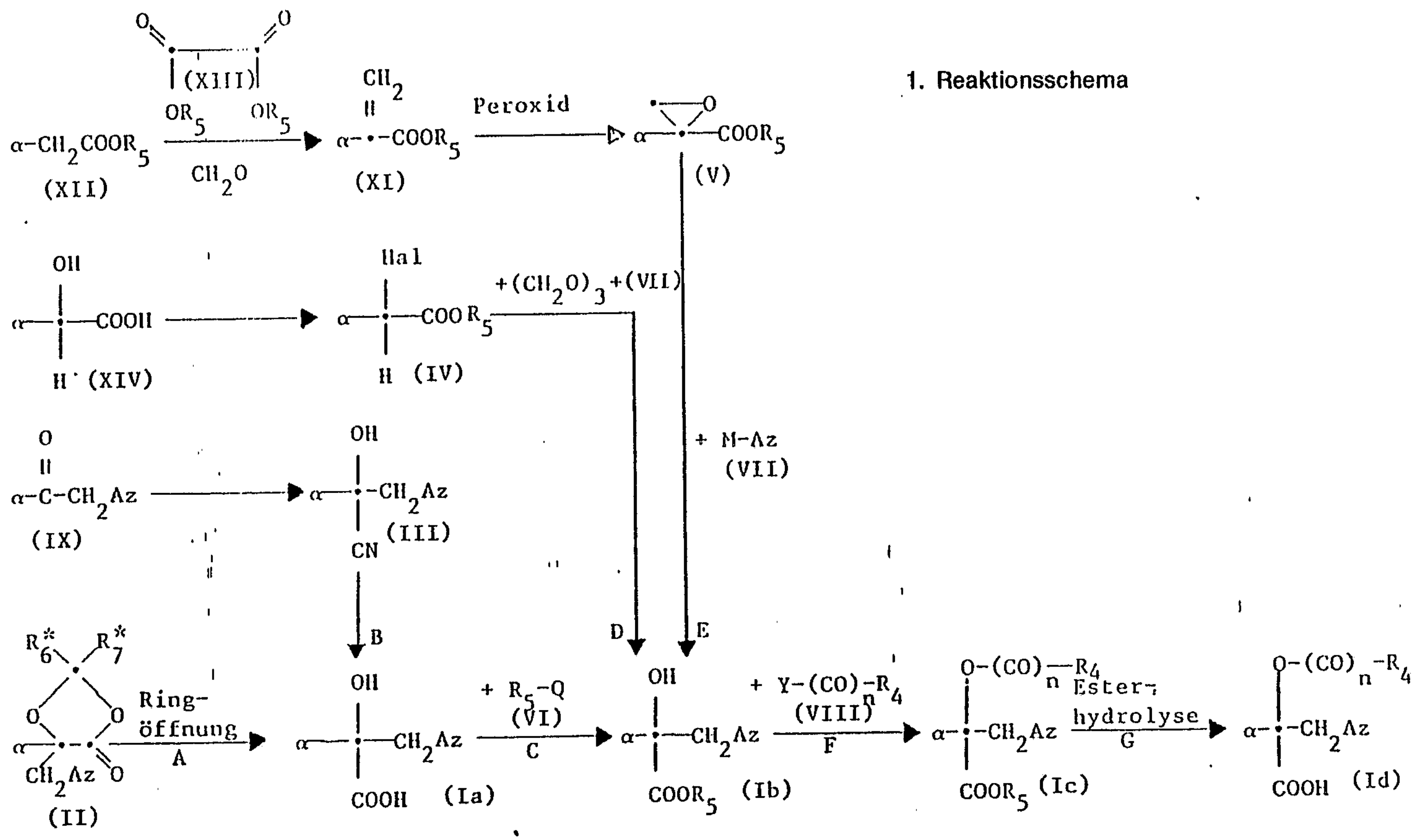


(XII) (XIII) (XI) (V) (XIV) (IV) (IX) (III) (II) (Ia) (Ib) (Ic) (Id)

2. Reaktionsschema zur Herstellung der Thioester
(α und Az sind wie im 1. Reaktionsschema definiert)

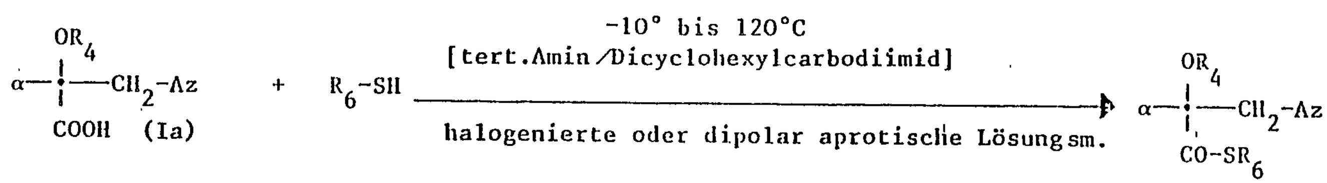


(Ia)

Die Mandelonitrile III (Variante B) lassen sich auf übliche Art aus Aryl-azolylmethylketonen der Formel IX

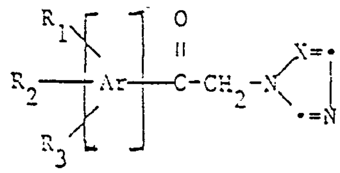

(IX)

nach Art einer Cyanhydrin-Synthese durch Reaktion mit HCN oder Alkalicyanid bei 0° bis 100 °C, vorteilhaft unter Zusatz geringer Mengen einer Base (bevorzugt $NH_4OH$ oder $NH_3$-Gas), oder aber über das entsprechende $NaHSO_3$-Addukt von IX darstellen [Org. Syntheses Coll. Vol. I, p. 336, oder FR-PS 2292706 ; siehe auch Houben-Weyl « Methoden der organischen Chemie », Band 6/3, p. 412].

Mandelonitrile III lassen sich auch gemäss J. Org. Chem. *39*, p. 914 (1974) durch Reaktion von IX mit Trimethylsilylcyanid in Gegenwart katalytischer Mengen $ZnJ_2$ und anschliessende Hydrolyse des Additionsprodukts herstellen.

Sie lassen sich auch durch Reaktion eines Ketons IX mit einem Diniederalkylcyanhydrin der Formel

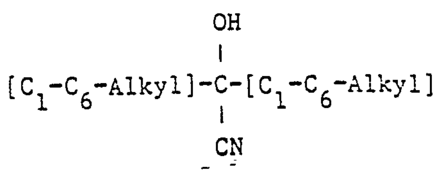

(Alkyl ist besonders Methyl, Ethyl, Propyl) bevorzugt in einem inerten Lösungsmittel oder ohne Lösungsmittel bei 50°-150 °C gewinnen.

Die Hydrolyse der Nitrile III zu Mandelsäurederivaten der Formel Ia kann analog zu bekannten Methoden z. B. mit konz. Chlorwasserstoffsäure erfolgen [Houben-Weyl « Methoden der organischen Chemie, Band VIII, p. 427 ff. (1952)].

Die als Zwischenprodukte dienenden Ketone der Formel IX sind aus der DE-OS 2 431 407 bzw. der GB-PS 1 464 224 zum Teil bekannt geworden. Derartige Ketone lassen sich auch durch Hydrolyse aus entsprechenden Ketalen herstellen, z. B. aus solchen, die in einer der folgenden Publikationen genannt sind : DE-OS 2 610 022 ; 2 602 770 ; 2 930 029 ; 2 930 196 ; 2 940 133.

Noch nicht beschriebene Ketone der Formel IX lassen sich nach einer der vorstehend publizierten Methode erhalten.

ii) Mandelsäureester der Formel Ib sind besonders bevorzugt. Sie lassen sich gemäss Gleichung C auf übliche Art durch Veresterung des entsprechenden Mandelsäure-Derivats Ia (auch in Form seines Alkalimetallsalzes) mit $R_5$-Q (VI) bei — 20° bis + 140 °C herstellen. Für diese Reaktion sind aprotische Lösungsmittel bevorzugt. Die direkte Veresterung wird vorteilhaft mit überschüssigem Alkohol $R_5$-OH bei 0° bis 80° in Gegenwart von Mineralsäuren oder bevorzugt von Lewis-Säuren wie Bortrifluorid-Etherat durchgeführt.

Mandelsäureester der Formel Ib lassen sich auch gemäss Gleichung D aus einem α-Halogenessigester der Formel IV mit Paraformaldehyd bei 0° bis 140 °C, bevorzugt bei 10° bis 80 °C, und a) dem gewünschten Azol der Formel VII (Imidazol oder Triazol) in Gegenwart einer Base (z. B. NaH) oder b) mit dem Alkalisalz des Azols VII in wasserfreien Lösungsmitteln (z. B. Dimethylsulfoxid) darstellen. Diese letztere neuartige Methode ist ein besonderer Bestandteil vorliegender Erfindung.

Ester der Formel Ib lassen sich auch gemäss Gleichung E aus Oxiranen der Formel V mit einem Azol VII (M = H oder Alkalimetall) in einem inerten, bevorzugt polaren Lösungsmittel (DMF, Acetonitril, DMSO und anderen auch in Gemischen mit Kohlenwasserstoffen) bei 20° bis 100 °C herstellen. Dabei können anorganische oder organische Basen zugesetzt werden [vgl. auch EP-OS 15 756].

Wie im 1. Reaktionsschema skizziert, sind Oxirane der Formel V durch übliche Epoxidierung (z. B. $H_2O_2$/wss · NaOH, Peressigsäure u. a.) aus entsprechenden Alkenylverbindungen der Formel XI herstellbar. Verbindungen der Formel XI werden aus Arylessigsäureestern der Formel XII durch Reaktion mit Oxalsäureestern der Formel XIII und Formaldehyd in Gegenwart einer Base hergestellt [vgl. Helvetica Chimica Acta *30*, p. 1 349 (1947) und DE-OS 2 653 189].

Ester der Formel Ib lassen sich auch aus Säuren der Formel Ia und Dimethylformamidacetal (vorzugsweise im Ueberschuss), dessen Acetalkomponente den alkoholischen Teil des Esters bilden soll, in Lösungsmitteln (z. B. ein gleichartiger wasserfreier Alkohol oder aber ein Ether) bei 0° bis 160 °C herstellen [Angew. Chemie *75*, p. 296 (1963) und Helv. Chim. Acta *48*, 1747 (1965)].

iii) Die der Formel Ib entsprechenden und im 2. Reaktionsschema genannten Thioester lassen sich aus den Säuren Ia mit Thioalkoholen in Gegenwart schwacher Basen (tert. Amine) in aprotischen

Lösungsmitteln wie $CHCl_3$, DMF, Dichlormethan, DMSO u. a. bei —10° bis + 120 °C, bevorzugt 0° bis 40° herstellen.

iv) Die freie Hydroxylgruppe in den Verbindungen Ia und Ib ist den üblichen Verätherungs- und Veresterungs-Reaktionen zugänglich, wie sie jedem Fachmann geläufig sind. Für die Acylierung (n = 1 in der Formel I) sind besonders die Gegenwart von Na-hydrid oder aber ein Gemisch aus einem üblichen tert. Amin mit katalytischen Mengen von 4-Dialkylamino-pyridin (z. B. 4-Dimethylaminopyridin) geeignet. Die Acylierung kann dann in der Regel bei Raumtemperatur durchgeführt werden [Angew. Chemie *90*, p. 615 (1978) und « Synthesis » 1972, p. 619].

Umgekehrt lassen sich mit solchen acylierten Verbindungen Hydrolysen zur Bildung der freien OH-Gruppe sowie auch Umesterungen vornehmen.

Sämtliche vorstehend unter i), ii), iii) und iv) geschilderten Herstellungsvarianten sind Teil vorliegender Erfindung.

Die übrigen Ausgangsprodukte der Formel IV, VI, VII, VIII, XI, XII und XIII sind bekannt oder werden nach an sich bekannten Methoden hergestellt.

Die Verbindungen der Formel I besitzen nachbarständig zur aromatischen Gruppe Ar und zu R ein Asymmetriezentrum (*)

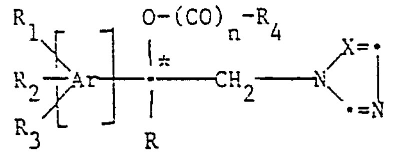

(I)

und können daher in zwei enantiomeren Formen vorliegen. Im allgemeinen entsteht bei der Herstellung dieser Substanzen ein Gemisch beider Enantiomerer, dieses lässt sich auf übliche Weise, z. B. durch fraktionierte Kristallisation, in die optischen Antipoden aufspalten. Optisch reine Antipoden erhält man beispielsweise nach Verfahrensvariante D, indem man eine optisch reine α-Hydroxicarbonsäure der Formel (XIV) in die optisch reine Verbindung der Formel IV überführt und letztere wie unter Variante D zum Ester Ib reagieren lässt.

Sofern nicht speziell genannt, liegt bei der Nennung einer Verbindung der Formel I stets ein Gemisch beider enantiomerer Formen vor. Beide Antipoden zeigen unterschiedliche mikrobizide Aktivität.

Es würde überraschend festgestellt, dass Verbindungen der Formel I ein für praktische Bedürfnisse sehr günstiges Mikrobizid-Spektrum gegen phytopathogene Pilze und Bakterien aufweisen. Sie besitzen sehr vorteilhafte kurative, präventive und systemische Eigenschaften und lassen sich zum Schutz von Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben.

Die Wirkstoffe sind gegen die den folgenden Klassen angehörenden phytopathogenen Pilze wirksam : Ascomyceten (z. B. Venturia, Podosphaera, Erysiphe, Monilinia, Uncinula) ; Basidiomyceten (z. B. die Gattungen Hemileia, Rhizoctonia, Pellicularia, Puccinia) ; Fungi imperfecti (z. B. Botrytis, Helminthosporium, Fusarium, Septoria, Cercospora, Piricularia und Alternaria) und gegen Phycomyceten wie Pythium. Ueberdies wirken die Verbindungen der Formel I systemisch. Sie können ferner als Beizmittel zur Behandlung von Saatgut (Früchte, Knollen, Körner) und Pflanzenstecklingen zum Schutz vor Pilzinfektionen sowie gegen im Erdboden auftretende phytophathogene Pilze eingesetzt werden. Die erfindungsgemässen Wirkstoffe zeichnen sich durch besonders gute Pflanzenverträglichkeit aus.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen.

Darüberhinaus schliesst die vorliegende Erfindung auch die Herstellung agrochemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für die hierin offenbarten Indikationsgebiete gelten im Rahmen dieser Erfindung beispielsweise folgende Pflanzenarten : Getreide : (Weizen, Gerste, Roggen, Hafer, Reis, Sorghum und Verwandte) ; Rüben : (Zucker- und Futterrüben) ; Kern-, Stein- und Beerenobst : (Aepfel, Birnen, Pflaumen, Pfirsiche, Mandeln, Kirschen, Erd-, Him- und Brombeeren) ; Hülsenfrüchte : (Bohnen, Linsen, Erbsen, Soja) ; Oelkulturen : (Raps, Senf, Mohn, Oliven, Sonnenblumen, Kokos, Rizinus, Kakao, Erdnüsse) ; Gurkengewächse : (Kürbis, Gurken, Melonen) ; Fasergewächse : (Baumwolle, Flachs, Hanf, Jute) ; Citrusfrüchte : (Orangen, Zitronen, Pampelmusen, Mandarinen) ; Gemüsesorten : (Spinat, Salat, Spargel, Kohlarten, Möhren, Zwiebeln, Tomaten, Kartoffeln, Paprika) ; Lorbeergewächse : (Avocado,

Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen.

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide, Insektizide, Fungizide, Bakterizide, Nematizide, Molluskizide oder Gemische mehrere dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z. B. natürlichen oder regenerierten mineralischen Stoffen, Lösungs-, Dispergier-, Netz-, Haft-, Verdickungs-, Binde- oder Düngemitteln.

Die Verbindungen der Formel I werden dabei in unveränderter Form oder vorzugsweise zusammen mit den in der Formulierungstechnik üblichen Hilfsmittel eingesetzt und werden daher z. B. zu Emulsionskonzentraten, streichfähigen Pasten, direkt versprühbaren oder verdünnbaren lösungen, verdünnten Emulsionen, Spritzpulvern, löslichen Pulvern, Stäubemitteln, Granulaten, durch Verkapselungen in z. B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha ; bevorzugt 100 g bis 2 kg AS/ha, insbesondere bei 200 g bis 600 g AS/ha.

Die Applikation solcher Mittel kann direkt auf die Pflanze oder Pflanzenteile erfolgen (Blattapplikation) oder auf den Standort der Pflanze (Bodenapplikation) oder auf die Vermehrungsteile, z. B. durch Saatgut-Applikation.

Die Formulierungen d. h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden). Solche Mittel sind gleichfalls Gegenstand vorliegender Erfindung.

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethylenglykolmonomethyl-ether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle oder Sojaöl ; oder Wasser.

Als feste Trägerstoffe, z. B. für Stäubemittel und dispergierbare Pulver, lassen sich Calcit, Talkum, Kaolin, Montmorillonit oder Attapulgit, hochdisperse Kieselsäure oder saugfähige Polymerisate verwenden. Als gekörnte, adsorptive Granulatträger kommen Bimsstein, Ziegelbruch, Sepiolit oder Bentonit, als nicht sorptive Träger z. B. Calcit oder Dolomit in Frage. Es können auch zerkleinerte Pflanzenrückstände verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Die in der Formulierungstechnik gebräuchlichen Tenside sind u. a. in folgenden Publikationen beschrieben :

« Mc Cutcheon's Detergents and Emulsifiers Annual » MC Publishing Corp., Ringwood New Jersey, 1980 Sisely and Wood, « Encyclopedia of Surface Active Agents », Chemical Publishing Co., Inc. New York, 1980.

Die agrochemischen Zubereitungen enthalten in der Regel 0,1 bis 99 %, insbesondere 0,1 bis 95 % Wirkstoff der Formel I, 99,9 bis 1 %, insbesondere 99,8 bis 5 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht. Darüberhinaus werden folgende Symbole verwendet : h = Stunde ; d = Tag ; min = Minute ; RT = Raumtemperatur ; N = Normalität ; abs = absolut, wasserfrei ; DMSO = Dimethylsulfoxid ; DMF = Dimethylformamid.

(Siehe Schema Seite 10 f.)

## Herstellungsbeispiele

### Beispiel 1 (Variante A im 1. Reaktionsschema)

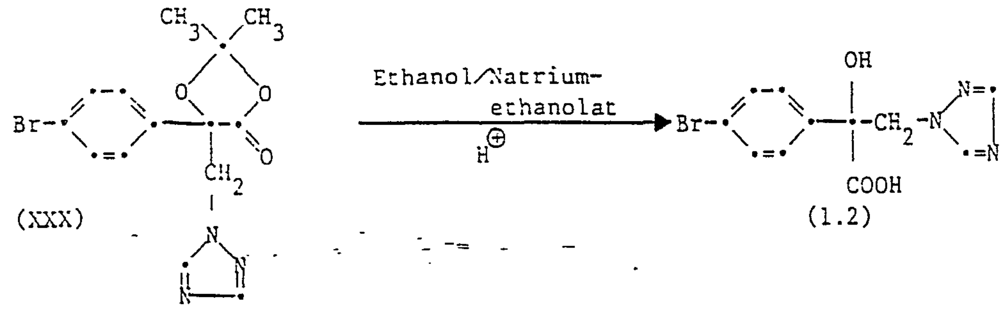


(XXX) (1.2)

Herstellung von 2-(1,2,4-Triazolylmethyl-1'-yl)-2-hydroxy-4-brom-phenylessigsäure (Verb. Nr. 1.2)

7,0 g (0,02 Mol) 4-(1H-1,2,4-Triazolylmethyl)-4-(4-bromphenyl)-2,2-dimethyl-1,3-dioxolan-5-on werden zu einer Lösung von 0,5 (0.022 Mol) Natrium in 50 abs. Ethanol gegeben und unter Rühren 4 h unter Rückfluss erhitzt. Die entstandene Suspension wird mit verdünnter Chlorwasserstoffsäure angesäuert, eingedampft, mit wenig Wasser digeriert, abfiltriert und getrocknet. Ausbeute 5,0 g (80 % d. Th.) Smp. 238-239 °C.

### Beispiel 2 (Variante C)

a)

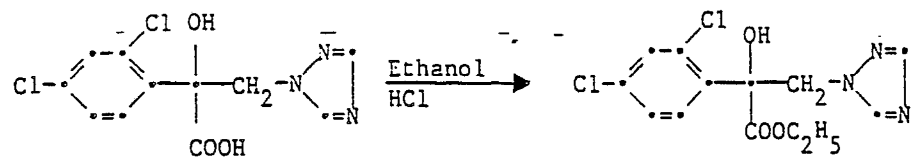


(XXXI) (1.9)

Herstellung von 2-(1,2,4-Triazolylmethyl-1'-yl)-2-hydroxy-2,4-dichlorphenylessigsäure-ethylester (Verb. Nr. 1.9)

50 g (0,165 Mol) 2-(1H-1,2,4-Triazolylmethyl-1'-yl)-2-hydroxy-2,4-dichlorphenylessigsäure werden in 500 ml absolutem Ethanol suspendiert. Die Suspension wird auf einer Temperatur von 20° bis 30 °C gehalten und unter Rühren mit Chlor-Wasserstoff gesättigt. Nach 6 d Rühren bei RT wird das Reaktionsgemisch 4 h unter Rückfluss erhitzt, eingedampft und der Rückstand unter Kühlung mit 10 %iger wässriger Natronlauge schwach alkalisch eingestellt (PH = 8 bis 9). Der anfallende Niederschlag wird abfiltriert, in Methylenchlorid gelöst und mit Wasser gewaschen. Die organische Phase wird über Natriumsulfat getrocknet, filtriert und eingedampft. Der kristalline Rückstand wird aus Methylenchlorid umkristallisiert, Smp. 121-123 °C.

Statt einer Sättigung der Anfangssuspension mit HCl-Gas kann die Reduktion durch Zugabe von 47,2 g (0,33 Mol) Bortrifluorid-Etherat katalysiert werden.

b) Herstellung von 2-(1H-1,2,4-Triazolylmethyl-l'-yl)-2-Hydroxy-4-bromphenylessigsäuremethylester und dessen Methojodid

62,4 g (0,2 Mol) 2-(1H-1,2,4-Triazolylmethyl-1'-yl)-2-hydroxy-4-bromphenylessigsäure werden mit 200 ml 1 N NaOH und 400 ml Methanol verrührt, im Vakuum eingedampft und im Hochvakuum getrocknet. Das entstandene Natriumsalz wird in 500 ml abs. DMF aufgenommen und unter Kühlung tropfenweise mit 42,6 g Methyljodid versetzt und 40 h bei RT gerührt. Dann wird das Reaktionsgemisch im Vakuum eingedampft, der Rückstand zwischen Wasser und Methylenchlorid verteilt. Die organische Phase wird mit Wasser gewaschen, über Natriumsulfat getrocknet, filtriert, eingedampft und der Rückstand mit Diethylether digeriert. Smp. 108-109 °C (Verb. Nr. 1.3).

Die oben anfallende wässrige Phase wird eingedampft, der Rückstand in Chloroform aufgenommen, mit wenig Wasser gewaschen, über Natriumsulfat getrocknet, filtriert und eingedampft. Der neue Rückstand wird in Chloroform gelöst und durch Zugabe von Diethylether zur Kristallisation gebracht. Das ausgefallene Methojodid wird abfiltriert und mit Diethylether gewaschen. Smp. 150-157 °C (Verb. Nr. 6.1).

c) Herstellung von 2-(1H-1,2,4-Triazolylmethyl-1'-yl)-2-hydroxy-2-(2',4'-dichlorphenyl)-essigsäuremethylester (Verb. 1.1)

6,0 g (0.02 Mol) 2-(1H-1,2,4-Triazolylmethyl-1'-yl)-2-hydroxy-2,4-dichlorphenylessigsäure (XXXI) werden in 120 ml abs. Methanol gelöst und tropfenweise mit 10 ml frisch destilliertem Thionylchlorid unter Kühlung innerhalb von 20 min versetzt. Es entsteht ein farbloser Niederschlag. Das Reaktionsgemisch wird 14 h unter Rückfluss erhitzt, eingedampft, der Rückstand wird mit gesättigter, wässriger Natriumhydrogencarbonat lösung unter Kühlung alkalisch eingestellt und mit Methylenchlorid extrahiert. Die Extrakte werden mit Wasser gewaschen, über Natriumsulfat getrocknet, eingedampft und der Rückstand [Smp. 185-188 °C] aus Aceton umkristallisiert. Smp. 188-190 °C. An Stelle von $SOCl_2$ lässt sich 5 ml (0,04 Mol) Bortrifluorid-Etherat vorteilhaft einsetzen.

d) (Variante F-Acylierung)

Herstellung von 2-(1H-1,2,4-Triazolylmethyl-1'-yl)-2-acetoxy-2-(2',4'-dichlorphenyl)-essigsäuremethylester (Verb. Nr. 2.2)

6,4 g (0,02 Mol) der unter c) erhaltenen Verbindung Nr. 1.1 werden zusammen mit 4,1 ml Triäthylamin und 0,5 g 4-Dimethylaminopyridin in 40 ml Dichlormethan vorgelegt. Bei Raumtemperatur werden 3,4 ml Acetanhydrid in 5 ml Dichlormethan zugetropft, wobei sich die Suspension von 22° auf 26° erwärmt und nach beendetem Zutropfen in Lösung geht. Nach 48 Stunden wird mit Eiswasser versetzt und mit Dichlormethan extrahiert. Die Extrakte werden mit Wasser gewaschen, getrocknet und eingedampft. Es verbleibt ein bräunliches Oel, das über Kieselgel (Diethylether) gereinigt wird. 4,8 g der Verbindung Nr. 2.2, Smp. 143-144 °C.

## Beispiel 3 (Variante F-Verätherung)

Herstellung von 2-(1H-1,2,4-Triazolylmethyl-1'-yl)-2-methoxy-4-bromphenylessigsäuremethylester (Verb. Nr. 3.32)

Zu 15,9 g (0,051 Mol) 2-(1H-1,2,4-Triazolylmethyl-1'-yl)-2-hydroxy-4-bromphenylessigsäure (Verb. Nr. 1.2) wird portionsweise eine Lösung von 2,3 g (0,1 Mol) Natrium in 200 ml abs. Methanol eingetragen. Das Reaktionsgemisch wird langsam aufgeheizt, kurze Zeit (5-10 min) unter Rückfluss erhitzt und im Vakuum zur Trockne eingedampft. Der Rückstand wird in 200 ml abs. DMF aufgenommen und bei 0° bis + 5 °C innerhalb von 0,5 h mit 29 g Methyljodid versetzt. Es wird ca. 10 h bei RT gerührt, das Lösungsmittel anschliessend im Vakuum entfernt, der Rückstand mit Eiswasser versetzt und mit Diethylether extrahiert.

Die organische Phase wird mit Wasser gewaschen, über Natriumsulfat getrocknet, abfiltriert und eingedampft. Der Rückstand wird aus Diethylether umkristallisiert. Smp. 80-92 °C.

Aus der wässrigen Phase kann analog zu Beispiel 2b das Methojodid isoliert werden (Verb. Nr. 6.4)

## Beispiel 4 (Variante B)

a) Herstellung des Cyanhydrins von 2,4-Dichlorphenyl-(1H-1,2,4-triazolylmethyl-1'-yl)-keton

38,4 g (0,15 Mol) des Ausgangsketons werden in 80 ml (0,88 Mol) Acetoncyanhydrin bei etwa 40 °C gelöst. Nachdem die Lösung nach etwa 1 h wieder RT aufweist, werden 4 Tropfen konzentrierter wässriger Ammoniaklösung zugegeben, dabei tritt eine Trübung der Reaktionslösung auf. Das Reaktionsgemisch wird ca. 10 h bei Raumtemperatur gerührt und 1 d stehen gelassen. Der gebildete Niederschlag wird abfiltriert und getrocknet : Smp. 145 °C unter Zersetzung.

$^1H$—NMR ($CDCl_3$ + DMSO-$d_6$) [δ in ppm]

4,9 (d, 2H), —CH— ; 7,1-7,6 (m, 3H), Phenyl-H ; 7,7 bzw. 8,3 je (S, 1H), Azol-H.

b) Herstellung von 2-(1H-1,2,4-Triazolylmethyl-1'-yl)-2-hydroxy-2,4-dichlorphenylessigsäure (Verb. Nr. 1.4)

20 g des nach a) hergestellten Cyanhydrins werden mit 200 ml konzentrierter Chlorwasserstoffsäure versetzt, dabei entsteht ein Niederschlag. Das Reaktionsgemisch wird zum Sieden erhitzt, nach 20 h erneut mit 100 ml konzentrierter Chlorwasserstoffsäure versetzt und weitere 24 h unter Rückfluss erhitzt. Dann wird das Gemisch im Vakuum eingedampft, der feste Rückstand in 200 ml Wasser aufgenommen, wobei zunächst eine Lösung und anschliessend wieder ein Niederschlag zu beobachten ist. Durch Zugabe von konzentrierter wässriger Ammoniaklösung wird der PH-Wert der Lösung auf etwa 2 eingestellt und das Gemisch in einem Mixer homogenisiert, der Niederschlag abfiltriert, mit Wasser gewaschen und getrocknet. Das bei 154-165 °C schmelzende Rohprodukt kann entweder weiter umgesetzt oder durch Kristallisation aus Aceton gereinigt werden. Das gereinigte Produkt schmilzt bei 177-181 °C.

Beispiel 5 (Variante D)

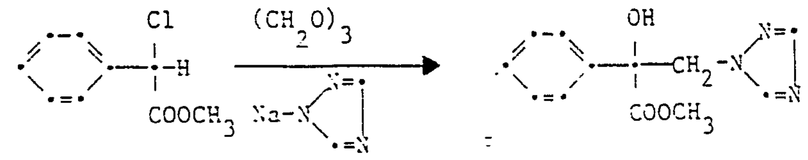


(XXXV) [DMSO] (1.7)

a) Herstellung von 2-(1H-1,2,4-Triazolylmethyl-1'-yl)-2-hydroxyphenylessigsäuremethylester (Verb. Nr. 1.7)

Unter Stickstoffatmosphäre und unter Rühren lässt man 2,4 g Natriumhydrid (55 %ige Oeldispersion) zu 3,8 g (0,055 Mol) 1H-1,2,4-Triazol, in 20 ml abs. DMSO gelöst, tropfen. Nach Abklingen der heftigen Wasserstoffentwicklung wird noch 0,5 h auf 60 °C erwärmt, anschliessend auf RT abgekühlt und zunächst 2 g fester Paraformaldehyd zugegeben, danach bei 15-20 °C unter Eiswasserkühlung eine Lösung von 9,2 g (0,05 Mol) α-Chlorphenylessigsäuremethylester in 5 ml DMSO zugetropfen gelassen. Das Gemisch wird 15 h bei RT gerührt, aufgeheizt und 6 h bei 60 °C gehalten. Nach Zugabe von weiteren 2 g Paraformaldehyd wird noch 5 h auf 80 °C erwärmt, anschliessend abgekühlt, mit Eiswasser versetzt und mit Methylenchlorid extrahiert. Die Extrakte werden mit Wasser gewaschen, über Natriumsulfat getrocknet, filtriert und eingedampft. Ueberschüssiges DMSO wird im Hochvakuum bei etwa 60 °C entfernt. Das Produkt wird chromatographisch [Kieselgel, Chloroform/Diethylether (1 : 1) ; gegen Schluss unter Zusatz von 20 % Methanol] gereinigt. Ausbeute 5,0 g, Smp. 104-106 °C.

Beispiel 6

Herstellung von 2-Hydroxy-2-(2',4'-Dichlorphenyl)-2-(1H-1,2,4-triazolylmethyl-1'-yl)-essigsäureallylester (Verb. Nr. 1.40)

18,0 g (0,06 Mol) der Verbindung Nr. 1.4 werden in 140 ml abs. Dichlormethan aufgeschlämmt und unter Eiskühlung mit einer Lösung von 15,3 g N,N-Dimethylformamid-diallylacetal in 60 ml Dichlormethan versetzt. Die entstehende feste Masse wird 7 Stunden zum Rückfluss erhitzt und eingedampft. Der Rückstand wird über Kieselgel (Chloroform/Diethylether 1 : 1) gereinigt. Aus dem eingedampften Eluat werden mit Hexan 15,0 g des weissen Endprodukts erhalten, Smp. 106-108 °C.

Beispiel 7

a) Herstellung von

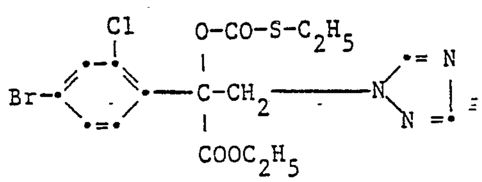

2-(2'-Chlor-4'-bromphenyl)-2-(ethylthiocarbonyl-oxy)-2-(1H-1,2,4-triazolylmethyl-1'-yl)-essigsäureethylester (Verb. Nr. 2.149)

1,3 g (0,03 Mol) 55 %ige Natriumhydriddispersion in Paraffinöl werden in 30 ml Tetrahydrofuran vorgelegt. Dann werden 11,2 g (0,03 Mol) des entsprechenden 2-(2'-Chlor-4'-bromphenyl)-2-hydroxy-2-(1H-1,2,4-triazolylmethyl-1'-yl)-essigsäureethylesters, gelöst in 70 ml Tetrahydrofuran, unter Stickstoffatmosphäre bei Raumtemperatur zugetropft. Nach beendigter Wasserstoffentwicklung werden 4,5 g (0,036 Mol) Chlorthioameisensäure-S-ethylester bei 0-5 °C zugetropft und das Reaktionsgemisch wird über Nacht bei Zimmertemperatur gerührt. Nach dem Einengen am Wasserstrahlvakuum wird der Rückstand in Essigsäureethylester gelöst und mit eisgekühlter Sodalösung und Wasser gewaschen. Nach dem Trocknen mit Natriumsulfat, Filtrieren und Einengen wird der Rückstand zur Abtrennung des Paraffinöls in Acetonitril gelöst und das Oel im Scheidetrichter abgetrennt. Nach dem Einengen am Wasserstrahlvakuum erhält man 8,5 g Endprodukt als gelbes Oel.

b) Verwendet man 3,9 g (0,036 Mol) Dimethylcarbamoylchlorid an Stelle von Chlorthioameisensäure-S-ethylester, so erhält man bei gleicher Arbeitsweise 10,8 g 2-(2'-Chlor-4'-bromphenyl)-2-(N,N-dimethylcarbamoyloxy)-2-(1H-1,2,4-triazolylmethyl-1'-yl)-essigsäureethylester, Smp. 45-66 °C (Verb. Nr. 2.181).

Beispiel 8

a) Herstellung von 2-(2',4'-Dichlorphenyl)-2-hydroxy-2-(1H-1,2,4-triazolylmethyl-1'-yl)-essigsäure-thioethylester (Verb. Nr. 4.3)

12,1 g (0,04 Mol) 2-(1H-1,2,4-Triazolylmethyl-1'-yl)-2-hydroxy-2,4-dichlorphenylessigsäure werden zusammen mit 3,0 ml (0,040 6 Mol) Ethylmercaptan und 0,5 g 4-Dimethylaminopyridin in 60 ml abs. DMF bei 0° vorgelegt. Dazu werden unter Rühren 8,2 g Dicyclohexylcarbodiimid, in 25 ml abs. DMF gelöst, zugetropft, 15 Stunden gerührt, filtriert, und der Niederschlag mit DMF gewaschen. Das Filtrat wird mit Eiswasser versetzt, und die ausgefallene, klebrige Masse in Dichlormethan aufgenommen und die Wasserphase weiter mit Dichlormethan extrahiert. Die vereinigten Extrakte werden mit Wasser gewaschen, getrocknet und eingedampft. Der feste Rückstand wird aus Tetrahydrofuran/Ethylacetat umkristallisiert : 7,5 g (55 %) Endprodukt, Smp. 180-182 °C.

b) Verwendet man 4,5 ml Thiophenol an Stelle des Ethylmercaptans erhält man bei gleicher Arbeitsweise umd Umkristallisation des Endprodukts aus Tetrahydrofuran/Hexan 5,5 g 2-(2',4'-Dichlorphenyl)-2-hydroxy-2-(1H-1,2,4-triazolylmethyl-1'-yl)-essigsäure-thiophenylester (Verb. Nr. 4.7) als weisse Kristalle, Smp. 164-166 °C.

Nach Art der vorstehenden Beispiele und der einleitend angegebenen Herstellungsvarianten lassen sich folgende Verbindungen gemäss vorliegender Erfindung herstellen :

(Siehe Tabellen Seite 14 ff.)

Tabelle 1 : Verbindungen der Formel

(wobei nicht genannte Substituenten $R_1$, $R_2$, $R_3$ stets Wasserstoff bedeuten.)

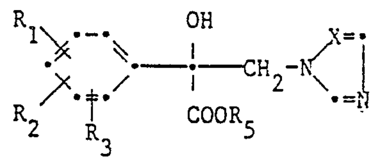

| Verb. Nr. | $R_1$, $R_2$, $R_3$ | $R_5$ | X | Physik. Daten [Schmelzpunkt] |
|---|---|---|---|---|
| 1.1 | 2,4-Di-Cl | $CH_3$ | N | Fp. 188-190°C |
| 1.2 | 4-Br | H | N | 238-239°C |
| 1.3 | 4-Br | $CH_3$ | N | 108-109°C |
| 1.4 | 2,4-Di-Cl | H | N | 177-181°C |
| 1.5 | 4-Br | $C_4H_9$-n | N | 74-76°C |
| 1.6 | 4-Br | $C_2H_5$ | N | 108-112°C |
| 1.7 | H | $CH_3$ | N | 104-6°C |
| 1.8 | 2,4-Di-Cl | $C_2H_5$ | CH | 136-9°C |
| 1.9 | 2,4-Di-Cl | $C_2H_5$ | N | 121-123°C |
| 1.10 | 2,4-Di-Cl | $C_3H_7$-n | N | 115-116°C |
| 1.11 | 2,4-Di-Cl | $C_3H_7$-n | CH | |
| 1.12 | 2,4-Di-Cl | $C_3H_7$-i | N | 146-149°C |
| 1.13 | 2,4-Di-Cl | $C_4H_9$-n | CH | |
| 1.14 | 2,4-Di-Cl | $C_4H_9$-n | N | 77-80°C |
| 1.15 | 4-Cl | $C_3H_7$-n | N | |
| 1.16 | 2,4-Di-Cl | $-CH(CH_3)C_3H_7$-n | N | Oel |
| 1.17 | 2,4-Di-Cl | 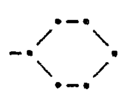 | N | halbfeste Masse |
| 1.18 | 2,4-Di-Cl | 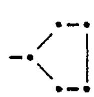 | N | Fp. 92-97°C |
| 1.19 | 2,4-Di-Cl | $-CH_2-C_6H_5$ | N | Fp. 151-152°C |
| 1.20 | 2,4-Di-Cl | $-CH_2-C_6H_5$ | CH | |
| 1.21 | 2,4-Di-Cl | $-C_6H_4Cl(4)$ | N | |

Tabelle 1 (Fortsetzung)

| Verb. Nr. | $R_1$, $R_2$, $R_3$ | $R_5$ | X | Physik. Daten |
|---|---|---|---|---|
| 1.22 | $3-NO_2$ | $CH_3$ | N | |
| 1.23 | 2,4-Di-Cl | $C_6H_3Cl_2(2,4)$ | N | |
| 1.24 | 2,4-Di-Cl | $-C(CH_3)_3$ | N | Fp. 126-128°C |
| 1.25 | 2,4-Di-Cl | $C_6H_3Cl_2(2,4)$ | CH | |
| 1.26 | $3-CF_3$ | $CH_3$ | N | |
| 1.27 | 2-Cl, 4-Br | $CH_3$ | N | Fp. 165-172°C |
| 1.28 | 2-Cl, 4-Br | $C_2H_5$ | CH | |
| 1.29 | 2-Cl, 4-Br | $C_2H_5$ | N | Fp. 132-139°C |
| 1.30 | 2,3,4 Tri-Cl | $CH_3$ | N | |
| 1.31 | 4-Cl | $C_2H_5$ | N | Fp. 60-4°C |
| 1.32 | 2-Cl-4-Br | $CH_2CH_2OCH_3$ | N | Fp. 167-171°C |
| 1.33 | 2-Cl-4-Br | $CH_2CH_2OCH_3$ | CH | |
| 1.34 | 2,4-Di-Cl | $CH_2CH_2OCH_3$ | N | |
| 1.35 | 2-Cl, 4-F | $CH_2CH_2OCH_3$ | N | |
| 1.36 | 2,4-Di-Cl | $CH_2OCH_3$ | N | |
| 1.37 | 2-Cl, 4-Br | $CH_2OCH_3$ | CH | |
| 1.38 | 2-Cl, 4-F | $CH_2SCH_3$ | N | |
| 1.39 | 2-Cl, 4-Br | $CH_2CH{=}CH_2$ | N | |
| 1.40 | 2,4-Di-Cl | $CH_2CH{=}CH_2$ | N | Fp. 106-108°C |
| 1.41 | 2,4-Di-Cl | $CH_2C{\equiv}CH$ | N | |
| 1.42 | 2-Cl, 4-Br | $CH_2C{\equiv}CH$ | N | |
| 1.43 | 2,4-Di-Cl | $-CH_2-$ 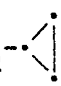 | N | Fp. 98-100°C |
| 1.44 | 2,4,6-Tri-Cl | $CH_3$ | N | |
| 1.45 | 2-F | $CH_3$ | N | |
| 1.46 | 4-Cl | $C_2H_5$ | CH | |
| 1.47 | 3,4-Di-Cl | $C_2H_5$ | N | |
| 1.48 | $2,5-Di-CH_3$ | $CH_3$ | N | Fp. 135-139°C |
| 1.49 | $2,4-Di-CH_3$ | $CH_3$ | N | |
| 1.50 | 2-Cl, 4-F | $CH_2OCH_3$ | N | |

Harz

Tabelle 1 (Fortsetzung)

| Verb. Nr. | $R_1$, $R_2$, $R_3$ | $R_5$ | X | Physik. Daten |
|---|---|---|---|---|
| 1.51 | 2-Cl, 4-F | $C(CH_3)_3$ | N | |
| 1.52 | 2-Cl, 4-Br | $C(CH_3)_3$ | N | |
| 1.53 | 2,4-Di-Cl | $-CH(CH_3)C_2H_5$ | CH | |
| 1.54 | 2,4-Di-Br | $-CH(CH_3)_2$ | N | |
| 1.55 | 2-Cl, 4-Br | $CH(CH_3)_2$ | N | Fp. 150-155°C |
| 1.56 | 2-Cl, 4-F | $C_3H_{7-n}$ | N | |
| 1.57 | 2,4-Di-Cl | $C_5H_{11-n}$ | N | |
| 1.58 | " | $C_6H_{13-n}$ | N | Fp. 81-82°C |
| 1.59 | 2,4-Di-Br | $CH(CH_3)C_5H_{11-n}$ | N | |
| 1.60 | 2,4-Di-Cl | $CH(CH_3)COOCH_3$ | N | Fp. 79-81°C |
| 1.61 | " | $CH_2COOCH_3$ | N | |
| 1.62 | " | $CH_2CH_2SCH_3$ | N | |
| 1.63 | " | $CH_2CH_2Br$ | N | Fp. 180-182°C |
| 1.64 | " | $C_6H_5$ | N | |
| 1.65 | 2-Cl, 4-Br | $C_6H_3Cl_2(2,4)$ | N | |
| 1.66 | 2-Cl, 4-F | $C_6H_3(CH_3)_2(2,4)$ | N | |
| 1.67 | 2,4-Di-Cl | $C_6H_2(CH_3)_2(3,5)Cl(4)$ | N | |
| 1.68 | " | $C_6H_4(OCH_3)(4)$ | N | |
| 1.69 | " | $C_6H_4Br(3)$ | CH | |
| 1.70 | " | $C_6H_4-C(CH_3)_3(4)$ | N | |
| 1.71 | " | $C_6H_4-CF_3(3)$ | CH | |
| 1.72 | 2-Cl, 4-Br | $-CH_2-$ 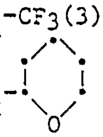 | N | Oel |
| 1.73 | 2,4-Di-Br | $-CH_2-$ 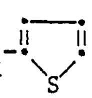 | N | |
| 1.74 | 2,4-Di-Cl | $-CH_2COOC_2H_5$ | N | |
| 1.75 | " | $-CH_2CCl_3$ | N | |

Tabelle 1 (Fortsetzung)

| Verb. Nr. | $R_1$, $R_2$, $R_3$ | $R_5$ | X | Physik. Daten |
|---|---|---|---|---|
| 1.76 | 2,4-Di-Cl | $-CH_2-C(CH_3)_3$ | N | Fp. 158-160°C |
| 1.77 | " | $-C_8H_{17}-n$ | N | Harz |
| 1.78 | " | $-C_{12}H_{25}-n$ | N | Oel |
| 1.79 | " | $-CH_2-CO-C(CH_3)_3$ | N | Fp.70-73°C |
| 1.80 | " | $-CH_2-CO-$ 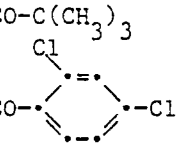 | N | |
| 1.81 | 2-Cl, 4-Br | $-CH_2\overset{O}{\overset{\|}{C}}-C(CH_3)_3$ | N | |
| 1.82 | 2-Cl, 4-Br | $-C_8H_{17}-n$ | CH | |
| 1.83 | 2-Cl, 4-Br | $-CH_2-C_6H_5$ | N | Fp. 138-141°C |
| 1.84 | 2-Cl, 4-F | $-CH_2-C_6H_5$ | N | |
| 1.85 | " | $-CH_2CH=CH_2$ | N | |
| 1.86 | " | $-CH_2-$ 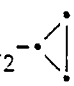 | CH | Harz |
| 1.87 | " | 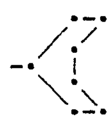 | N | |
| 1.88 | 2,4-Di-Cl | 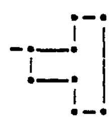 | N | |
| 1.89 | 4-Br | $-CH_2-CCl_3$ | N | |
| 1.90 | 2-Cl, 4-F | $-CH_2COOCH_3$ | N | |
| 1.91 | 2,4-Di-$CH_3$ | $-CH(CH_3)-COOCH_3$ | N | |
| 1.92 | 2-Cl, 4-Br | $-CH_2CH_2SCH_3$ | N | |
| 1.93 | 4-$NO_2$ | $-C_2H_5$ | N | |
| 1.94 | 4-F | $-CH_3$ | CH | |
| 1.95 | 4-F | $-CH_2CH_2OCH_3$ | N | |

Tabelle 1 (Fortsetzung)

| Verb. Nr. | $R_1$, $R_2$, $R_3$ | $R_5$ | X | Physik. Daten |
|---|---|---|---|---|
| 1.96 | 2,4-Di-Br | H | N | |
| 1.97 | 2-Cl, 4-Br | H | N | Fp. 226-233°C (Hydrochlorid) |
| 1.98 | 2-Cl, 4-Br | $-C(CH_3)_2-C_2H_5$ | N | |
| 1.99 | 2,4-Di-Br | $CH_3$ | N | |
| 1.100 | 2-Cl, 4-F | $CH_3$ | N | Fp. 168-172°C |
| 1.101 | " | $C_2H_5$ | N | Fp. 173-177°C |
| 1.102 | " | $C_2H_5$ | CH | |
| 1.103 | " | $C_3H_7$-i | N | |
| 1.104 | " | 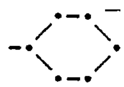 | N | |
| 1.105 | 2-Cl, 4-Br | 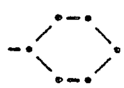 | N | |
| 1.106 | " | 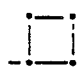 | N | |
| 1.107 | 2,4-Di-Cl | 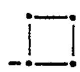 | N | |
| 1.108 | " | 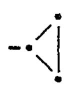 | N | |
| 1.109 | " | $-CH_2-$ 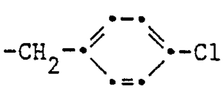 | N | |
| 1.110 | 2-Cl, 4-Br | $-CH_2CH_2Cl$ | N | Fp. 113-116°C |
| 1.111 | 2,4-Di-Br | $C_2H_5$ | N | Fp. 151-153°C |
| 1.112 | 2-Cl, 4-Br | $-CH_2-$ 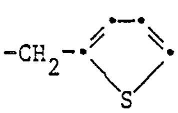 | N | |

Tabelle 1 (Fortsetzung)

| Verb. Nr. | $R_1$, $R_2$, $R_3$ | $R_5$ | X | Physik. Daten |
|---|---|---|---|---|
| 1.113 | 4-F | $C_2H_5$ | N | |
| 1.114 | 2,6-Di-Cl | $C_2H_5$ | N | |
| 1.115 | 4-F | $-C(CH_3)_3$ | N | |
| 1.116 | 2,4-Di-Cl | $-CH_2SCH_3$ | N | 145-147°C |
| 1.117 | 2,4-Di-Cl | $-CH_2CH_2Cl$ | N | |
| 1.118 | " | $-CH_2-CO-CH_3$ | N | |
| 1.119 | " | $-CH_2SCH_3$ | CH | |
| 1.120 | 2-Cl, 4-F | $-CH_2SCH_3$ | N | |
| 1.121 | " | $-CH_2-COOC_2H_5$ | N | |
| 1.122 | " | $-CH_2-CO-C(CH_3)_3$ | N | |
| 1.123 | " | $-CH_2CH_2Cl$ | N | |
| 1.124 | " | $-C_6H_4(CF_3)-4$ | N | |
| 1.125 | | | | |
| 1.125 | 2-Cl, 4-Br | $-CH_2CO-$ 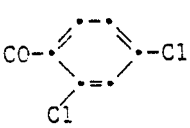 | N | |
| 1.126 | 2,4-Di-Cl | $-CH_2CH_2F$ | N | |

Tabelle 2 : Verbindungen der Formel I mit Ar = Phenyl, worin n = 1 bedeutet (Acyl- und Oxalyl-Derivate) und R = $COOR_5$

| Verb. Nr. | $R_1$, $R_2$, $R_3$ | $R_4$ | $R_5$ | N | Physik. Daten |
|---|---|---|---|---|---|
| 2.1 | H | $-CH_3$ | $-CH_3$ | N | |
| 2.2 | 2,4-Di-Cl | $-CH_3$ | $-CH_3$ | N | Fp. 143-144°C |
| 2.3 | " | $-CH_3$ | $-C_2H_5$ | N | Fp. 149-151°C |
| 2.4 | " | $-CH_3$ | $-C_3H_7(i)$ | N | Fp. 144-150°C |
| 2.5 | " | $-CH_3$ | $-CH_3$ | CH | Fp. 138-141°C |
| 2.6 | " | $-CH_3$ | $-C_4H_9(n)$ | N | Oel |
| 2.7 | " | $-CH_3$ | $-C_3H_7(n)$ | N | |
| 2.8 | " | $-CH_3$ | $-C_4H_9(t)$ | N | |
| 2.9 | " | $-CH_3$ | $-CH_2CH{=}CH_2$ | N | Fp. 152-156°C |
| 2.10 | " | $-CH_3$ | $-CH_2CH_2OCH_3$ | N | |
| 2.11 | " | $-CH_3$ | $-CH_2CH_2Cl$ | N | |
| 2.12 | " | $-CH_3$ | $-CH_2OCH_3$ | N | |
| 2.13 | " | $-CH_3$ | $-CH_2-C_6H_5$ | N | |
| 2.14 | " | $-CH_3$ | 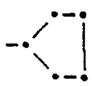 | N | |
| 2.15 | " | $-CH_3$ | 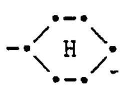 | N | |
| 2.16 | " | $-CH_3$ | $-CH_2-$ 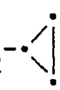 | N | |
| 2.17 | " | $-CH_3$ | $-CH_2C{\equiv}CH$ | N | |
| 2.18 | " | $-CH_3$ | $-C_{12}H_{25}(n)$ | N | |
| 2.19 | " | $-CH_3$ | $-CH_2-$ 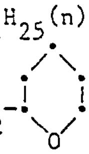 | N | |
| 2.20 | " | $-CH_3$ | $-CH_2-$ 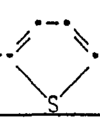 | N | |

Tabelle 2 (Fortsetzung)

| Verb. Nr. | $R_1$, $R_2$, $R_3$ | $R_4$ | $R_5$ | N | Physik. Daten |
|---|---|---|---|---|---|
| 2.21 | 2,4-Di-Cl | $-CH_3$ | $-CH_2COOC_2H_5$ | N | |
| 2.22 | " | $-CH_3$ | $-CH(CH_3)COOCH_3$ | N | |
| 2.23 | " | $-C_2H_5$ | $-CH_3$ | N | |
| 2.24 | " | $-C_2H_5$ | $-C_2H_5$ | N | |
| 2.25 | " | $-C_2H_5$ | $-C_2H_5$ | CH | |
| 2.26 | " | $-C_2H_5$ | $-C_3H_7(n)$ | N | |
| 2.27 | " | $-C_2H_5$ | $-C_3H_7(i)$ | N | |
| 2.28 | " | $-C_2H_5$ | $-C_4H_9(n)$ | N | |
| 2.29 | " | $-C_2H_5$ | $-CH_2CH=CH_2$ | N | |
| 2.30 | " | $-C_2H_5$ | $-CH_2CH_2OCH_3$ | N | |
| 2.31 | " | $-C_2H_5$ | $-CH_2OCH_3$ | N | |
| 2.32 | " | $-C_2H_5$ | $-C_4H_9(t)$ | N | |
| 2.33 | " | $-CH_2OCH_3$ | $-CH_3$ | N | |
| 2.34 | " | $-CH_2OCH_3$ | $-CH_3$ | CH | |
| 2.35 | " | $-CH_2OCH_3$ | $-C_2H_5$ | N | Oel |
| 2.36 | " | $-CH_2OCH_3$ | $-C_3H_7(n)$ | N | |
| 2.37 | " | $-CH_2OCH_3$ | $-C_3H_7(i)$ | N | |
| 2.38 | " | $-CH_2OCH_3$ | $-C_4H_9(n)$ | N | |
| 2.39 | " | $-CH_2OCH_3$ | $-C_4H_9(t)$ | N | |
| 2.40 | " | $-CH_2OCH_3$ | $-CH_2CH=CH_2$ | N | |
| 2.41 | " | " | $-CH_2CH_2OCH_3$ | N | |
| 2.42 | " | $-CCl=CCl_2$ | $-CH_3$ | N | |
| 2.43 | " | " | $-C_2H_5$ | N | |
| 2.44 | " | $-CH_2Cl$ | $-CH_3$ | N | |
| 2.45 | " | $-CH_2Cl$ | $-C_2H_5$ | N | |
| 2.46 | " | 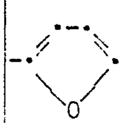 | $-CH_3$ | N | |

Tabelle 2 (Fortsetzung)

| Verb. Nr. | $R_1$, $R_2$, $R_3$ | $R_4$ | $R_5$ | N | Physik. Daten |
|---|---|---|---|---|---|
| 2.47 | 2,4-Di-Cl | 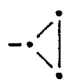 | $-CH_3$ | N | |
| 2.48 | " | $-C_6H_3Cl_2(3,4)$ | $-C_2H_5$ | N | |
| 2.49 | " | 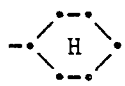 | $-C_2H_5$ | N | Oel |
| 2.50 | " | 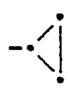 | $-CH_3$ | CH | |
| 2.51 | " | $-CH_2OC_2H_5$ | $-C_4H_9(n)$ | CH | |
| 2.52 | " | $-CH_2-C_6H_3Cl_2(2,4)$ | $-C_2H_5$ | N | |
| 2.53 | " | $-CH_2-C_6H_3Cl_2(2,4)$ | $-CH_3$ | N | |
| 2.54 | " | $-CH_2OC_2H_5$ | $-C_6H_4Cl(4)$ | N | |
| 2.55 | " | 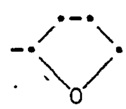 | $-CH_3$ | N | Oel |
| 2.56 | " | 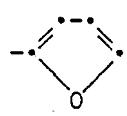 | $-C_2H_5$ | CH | |
| 2.57 | " | 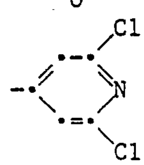 | $-C_6H_4-CH_3(4)$ | N | |
| 2.58 | " | $-C_{12}H_{25}(n)$ | $-CH_3$ | N | |
| 2.59 | $4-CH_3$ | $-CCl=CCl_2$ | $-CH_3$ | N | |
| 2.60 | 2-Cl | $-CCl=CCl_2$ | $-C_6H_4Cl(4)$ | N | |
| 2.61 | 4-Cl | $-CCl=CCl_2$ | $-CH_3$ | CH | |
| 2.62 | 4-Br | $-CCl=CCl_2$ | $-C_2H_5$ | N | |

Tabelle 2 (Fortsetzung)

| Verb. Nr. | $R_1$, $R_2$, $R_3$ | $R_4$ | $R_5$ | N | Physik. Daten |
|---|---|---|---|---|---|
| 2.63 | 3-$NO_2$ | 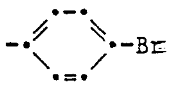 | $-CH_3$ | N | |
| 2.64 | 4-Cl | $-CH_3$ | $-C_2H_5$ | N | Oel |
| 2.65 | 4-Cl | $-C_2H_5$ | $-C_2H_5$ | N | Harz |
| 2.66 | 2-Cl, 4-Br | $-CH_3$ | $-C_2H_5$ | N | Fp. 85-87°C |
| 2.67 | " | $-CH_3$ | $-CH_3$ | N | Fp. 146-148°C |
| 2.68 | " | $-CH_3$ | $-C_3H_7$(i) | N | Oel |
| 2.69 | " | $-CH_3$ | $-C_4H_9$(n) | N | |
| 2.70 | " | $-CH_3$ | $-C_4H_9$(t) | N | |
| 2.71 | " | $-CH_3$ | $-C_2H_5$ | CH | |
| 2.72 | " | -CH3 | $-CH_2CH_2OCH_3$ | N | |
| 2.73 | " | $-CH_3$ | $-CH_2CH_2Cl$ | N | |
| 2.74 | " | $-CH_3$ | $-CH_2CH=CH_2$ | N | |
| 2.75 | " | $-CH_3$ | $-CH_2OCH_3$ | N | |
| 2.76 | " | $-CH_3$ | $-CH_2-C_6H_5$ | N | |
| 2.77 | " | $-CH_3$ | $-CH_2C\equiv CH$ | N | |
| 2.78 | " | $-CH_3$ | $-CH_2-$ 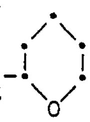 | N | |
| 2.79 | " | $-CH_3$ | $-CH_2-$ 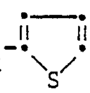 | N | |
| 2.80 | " | $-C_2H_5$ | $-C_2H_5$ | N | |
| 2.81 | " | $-C_2H_5$ | $-CH_3$ | N | |
| 2.82 | " | $-CH_2OCH_3$ | $-C_2H_5$ | N | |
| 2.83 | " | $-CH_2Cl$ | $-C_2H_5$ | N | |
| 2.84 | " | $-CCl=CCl_2$ | $C_2H_5$ | N | Oel |
| 2.85 | " | $-CH_2OC_2H_5$ | $C_6H_4Cl(4)$ | N | |
| 2.86 | " | 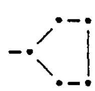 | $CH_3$ | N | |

Tabelle 2 (Fortsetzung)

| Verb. Nr. | $R_1$, $R_2$, $R_3$ | $R_4$ | $R_5$ | X | Physik. Daten |
|---|---|---|---|---|---|
| 2.87 | 2-Cl, 4-Br | $-C_6H_3Cl_2(2,4)$ | $C_2H_5$ | N | |
| 2.88 | " | 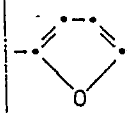 | $C_2H_5$ | N | |
| 2.89 | " | $-CH_3$ | 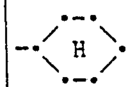 | N | |
| 2.90 | " | $-CH_3$ | 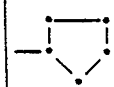 | N | |
| 2.91 | " | $-CH_3$ | $-C_6H_5$ | N | |
| 2.92 | 2-Cl, 4-F | $-CH_3$ | $-CH_3$ | N | Fp. 165-171°C |
| 2.93 | " | $-CH_3$ | $-CH_3$ | CH | |
| 2.94 | " | $-CH_3$ | $-C_2H_5$ | N | |
| 2.95 | " | $-CH_2OCH_3$ | $-C_2H_5$ | N | |
| 2.96 | " | $-CCl=CCl_2$ | $-C_2H_5$ | N | |
| 2.97 | " | $-CH_3$ | $-C_3H_7(i)$ | N | |
| 2.98 | " | $-CH_3$ | $-C_4H_9(n)$ | N | |
| 2.99 | " | $-CH_3$ | $-C_4H_9(t)$ | N | |
| 2.100 | " | $-CH_3$ | $-CH_2-C_6H_5$ | N | |
| 2.101 | " | $-CH_3$ | $-CH_2CH=CH_2$ | N | |
| 2.102 | 2,4-Di-Br | $-CH_3$ | $-CH_3$ | N | |
| 2.103 | " | $-CH_3$ | $-C_2H_5$ | N | Harz |
| 2.104 | " | $-CH_3$ | $-C_3H_7(i)$ | N | |
| 2.105 | " | $-CH_3$ | $-C_4H_9(t)$ | N | |
| 2.106 | 2,3,4-Tri-Cl | $-CH_3$ | $-CH_3$ | N | |
| 2.107 | 3,4-Di-Cl | $-CH_3$ | $-C_2H_5$ | N | |
| 2.108 | 2,4-Di-Cl | $-OCH_3$ | $-CH_3$ | N | |
| 2.109 | " | $-OC_2H_5$ | $-CH_3$ | N | Fp. 98-100° C |

Tabelle 2 (Fortsetzung)

| Verb. Nr. | $R_1$, $R_2$, $R_3$ | $R_4$ | $R_5$ | X | Physik. Daten |
|---|---|---|---|---|---|
| 2.110 | 2,4-Di-Cl | $-OC_2H_5$ | $-C_2H_5$ | N | Harz |
| 2.111 | " | $-OC_2H_5$ | $-C_2H_5$ | CH | Fp. 165°C (Zers.) |
| 2.112 | " | $-OC_2H_5$ | $-C_3H_7$(n) | N | |
| 2.113 | " | " | $-C_3H_7$(i) | N | |
| 2.114 | " | " | $-C_4H_9$(n) | N | |
| 2.115 | " | " | $-C_4H_9$(t) | N | |
| 2.116 | " | " | $-CH_2CH_2OCH_3$ | N | |
| 2.117 | " | " | $-CH_2CH_2Cl$ | N | |
| 2.118 | " | " | $-CH_2OCH_3$ | N | |
| 2.119 | " | " | $-CH_2-C_6H_5$ | N | |
| 2.120 | " | " | $-C_6H_5$ | N | |
| 2.121 | " | $-O-CH_2-C_6H_5$ | $-C_2H_5$ | N | |
| 2.122 | " | $-O-C_6H_5$ | $-C_2H_5$ | N | |
| 2.123 | " | -O- 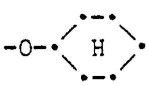 | $-C_2H_5$ | N | |
| 2.124 | " | $-OCH_2CH=CH_2$ | $-C_2H_5$ | N | |
| 2.125 | " | $-SC_2H_5$ | $-CH_3$ | N | Fp. 85-87° C |
| 2.126 | " | $-SC_2H_5$ | $-C_2H_5$ | N | |
| 2.127 | " | $-SC_2H_5$ | $-C_2H_5$ | CH | |
| 2.128 | " | $-S-CH_2-C_6H_5$ | $-C_2H_5$ | N | |
| 2.129 | " | $-SCH_3$ | $-CH_3$ | N | |
| 2.130 | " | $-SC_3H_7$(i) | $-C_2H_5$ | N | |
| 2.131 | 2-Cl, 4-Br | $-OCH_3$ | $-CH_3$ | N | |
| 2.132 | " | $-OC_2H_5$ | $-C_2H_5$ | N | Oel |
| 2.133 | " | $-OC_2H_5$ | $-C_2H_5$ | CH | |
| 2.134 | " | -O- 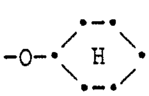 | $-C_2H_5$ | N | |

Tabelle 2 (Fortsetzung)

| Verb. Nr. | $R_1$, $R_2$, $R_3$ | $R_4$ | $R_5$ | X | Physik. Daten |
|---|---|---|---|---|---|
| 2.135 | 2-Cl, 4-Br | $-O-C_6H_5$ | $-C_2H_5$ | N | |
| 2.136 | " | $-O-CH_2-C_6H_5$ | $-C_2H_5$ | N | |
| 2.137 | " | $-OCH_2CH=CH_2$ | $-C_2H_5$ | N | |
| 2.138 | " | $-OC_3H_7(n)$ | $-C_2H_5$ | N | |
| 2.139 | " | $-OC_4H_9(n)$ | $-C_2H_5$ | N | |
| 2.140 | " | $-OC_2H_5$ | $-C_3H_7(n)$ | N | |
| 2.141 | " | $-OC_2H_5$ | $-C_3H_7(i)$ | N | Oel |
| 2.142 | " | $-OC_2H_5$ | $-C_4H_9(n)$ | N | |
| 2.143 | " | $-OC_2H_5$ | $-CH_2CH=CH_2$ | N | |
| 2.144 | " | $-OC_2H_5$ | $-CH_2CH_2OCH_3$ | N | |
| 2.145 | " | " | $-CH_2CH_2Cl$ | N | |
| 2.146 | " | " | $-CH_2-C_6H_5$ | N | |
| 2.147 | " | " | $-CH_2OCH_3$ | N | |
| 2.148 | " | $-SCH_3$ | $-C_2H_5$ | N | |
| 2.149 | " | $-SC_2H_5$ | $-C_2H_5$ | N | gelbes Oel |
| 2.150 | " | $-SCH_2-C_6H_5$ | $-C_2H_5$ | N | |
| 2.151 | " | $-SC_2H_5$ | $-C_3H_7(n)$ | N | |
| 2.152 | " | $-SC_2H_5$ | $-C_4H_9(t)$ | N | |
| 2.153 | " | $-SC_2H_5$ | $-CH_2CH=CH_2$ | N | |
| 2.154 | 2,4-Di-Br | $-SC_2H_5$ | $-C_2H_5$ | N | Harz |
| 2.155 | " | $-OC_2H_5$ | $-C_2H_5$ | N | Harz |
| 2.156 | " | $-OCH_3$ | $-CH_3$ | N | |
| 2.157 | 2-Cl, 4-F | $-OC_2H_5$ | $-CH_3$ | N | Viskos |
| 2.158 | " | $-OC_2H_5$ | $-C_2H_5$ | N | |
| 2.159 | " | $-OC_2H_5$ | $-C_3H_7(i)$ | N | |
| 2.160 | " | $-OC_2H_5$ | $-C_4H_9(t)$ | N | |
| 2.161 | " | $-SC_2H_5$ | $-C_2H_5$ | N | Harz |
| 2.162 | " | $-SCH_3$ | $-CH_3$ | N | |
| 2.163 | " | $-SCH_2-C_6H_5$ | $-C_2H_5$ | N | |

Tabelle 2 (Fortsetzung)

| Verb. Nr. | $R_1$, $R_2$, $R_3$ | $R_4$ | $R_5$ | X | Physik. Daten |
|---|---|---|---|---|---|
| 2.164 | 2-Cl, 4-F | $-OCH_3$ | $-CH_3$ | N | |
| 2.165 | " | $-OCH_2-C_6H_5$ | $-C_2H_5$ | N | |
| 2.166 | " | $-OC_2H_5$ | $-CH_2CH=CH_2$ | N | |
| 2.167 | " | $-OC_2H_5$ | $-C_2H_5$ | CH | |
| 2.168 | 3,4-Di-Cl | $-OC_2H_5$ | $-C_2H$ | N | |
| 2.169 | 3,4-Di-Cl | $-SC_2H_5$ | $-C_2H_5$ | N | |
| 2.170 | 2-Cl, 4-Br | $-OC_2H_5$ | $-CH_3$ | N | Harz |
| 2.177 | " | $-N(CH_3)_2$ | $-CH_3$ | N | |
| 2.178 | " | $-N(C_2H_5)_2$ | $-C_2H_5$ | N | |
| 2.179 | " | -1-imidazolyl | $-C_2H_5$ | N | |
| 2.180 | " | $-N(C_4H_9sec)_2$ | $-C_2H_5$ | N | |
| 2.181 | 2-Cl, 4-Br | $-N(CH_3)_2$ | $-C_2H_5$ | N | Smp. 45 - 66° |
| 2.182 | " | $-N(C_2H_5)_2$ | $-C_2H_5$ | N | |
| 2.183 | " | $-N(C_4H_9sec)_2$ | $-C_2H_5$ | N | |
| 2.184 | " | 1-(1,2,4-triazolyl | $-C_2H_5$ | N | |
| 2.185 | 2,4-Di-Cl | $-N(CH_3)_2$ | $-C_3H_7$(i) | N | |
| 2.186 | " | $-N(CH_3)_2$ | $-C_4H_9$(n) | N | |
| 2.187 | " | $-N(C_2H_5)_2$ | $-CH_2C-C_6H_5$ | N | |
| 2.193 | 4-Cl | $-OC_2H_5$ | -C H | N | Oel |

Tabelle 3 : Verbindungen der Formel I mit Ar = Phenyl, worin n = 0 bedeutet

| Verb. Nr. | $R_1$, $R_2$ $R_3$ | $R_4$ | $R_5$ | X | Physik. Daten |
|---|---|---|---|---|---|
| 3.1 | 2,4-Di-Cl | $-CH_2CH=CH_2$ | $-CH_3$ | N | |
| 3.2 | " | $-CH_3$ | $-CH_3$ | N | |
| 3.3 | " | $-C_2H_5$ | $-C_2H_5$ | N | Harz |
| 3.4 | " | $-CH_2CH=CH_2$ | $-C_2H_5$ | N | Oel |
| 3.5 | " | $-CH_2CH=CH_2$ | $-C_2H_5$ | CH | |
| 3.6 | " | $-CH_2-C_6H_5$ | $-C_2H_5$ | N | |
| 3.7 | " | $-CH_2COC(CH_3)_3$ | $-C_2H_5$ | N | |
| 3.8 | " | $-CH_2-CO-$ 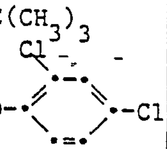 | $-CH_3-$ | N | |
| 3.9 | " | $-CH_2OCH_3$ | $-C_2H_5$ | N | |
| 3.12 | " | $-CH_3$ | $-C_4H_9(n)$ | N | Oel |
| 3.13 | " | $-C_2H_5$ | $-C_4H_9(i)$ | N | |
| 3.14 | " | $-C_4H_9(n)$ | $-C_4H_9(n)$ | N | |
| 3.15 | " | $-C_3H_7(n)$ | $-C_3H_7(n)$ | CH | |
| 3.16 | " | $-CH_2-C_6H_5$ | $-CH_2-C_6H_5$ | N | |
| 3.17 | " | $-CH_2CH=CH_2$ | $-CH_2CH=CH_2$ | N | |
| 3.18 | " | $-CH_2CH=CH_2$ | $-CH_2CH=CH_2$ | CH | |
| 3.19 | " | $-C_4H_9(n)$ | $C_6H_3Cl_2(2,4)$ | N | |
| 3.20 | " | $-C_2H_5$ | $-C(CH_3)_3$ | N | |
| 3.21 | " | $-CH_2CH_2CN$ | $-CH_3$ | N | |
| 3.23 | " | $-CH_2COC_6H_3Cl_2(2,4)$ | $-CH_3$ | CH | |
| 3.24 | " | $-CH_2COCH_3$ | $-C_2H_5$ | N | |
| 3.25 | " | $-C_3H_7(n)$ | $-CH_3$ | N | Harz |
| 3.26 | " | $-C_4H_9(n)$ | $-CH_2CH=CH_2$ | N | Harz |
| 3.27 | " | $-C_{12}H_{25}(n)$ | $-CH_3$ | N | Harz |

Tabelle 3 (Fortsetzung)

| Verb. Nr. | $R_1$, $R_2$, $R_3$ | $R_4$ | $R_5$ | X | Physik. Daten |
|---|---|---|---|---|---|
| 3.28 | 4-Cl | $-C_3H_7$ | $-C_3H_7(n)$ | N | |
| 3.29 | " | $-CH_2CH=CH_2$ | $-CH_3$ | N | |
| 3.30 | " | $-CH_2COC_6H_4Cl(4)$ | $-C_6H_4Cl(4)$ | N | |
| 3.31 | 2,3,4-Tri-Cl | $-CH_2CH=CH_2$ | $-CH_2CH=CH_2$ | N | |
| 3.32 | 4-Br | $-CH_3$ | $-CH_3$ | N | Fp. 80-92°C |
| 3.33 | $2-CH_3$ | $-CH_3$ | $-CH_3$ | N | |
| 3.34 | 2-Cl, 4-Br | $-CH_3$ | $-CH_3$ | N | |
| 3.35 | " | $-CH_2CH=CH_2$ | $-C_2H_5$ | N | |
| 3.36 | " | $-CH_2COC_6H_4Cl(4)$ | $-C_6H_4Cl(4)$ | N | |
| 3.37 | " | $-CH_2-C_6H_5$ | $-CH_2C_6H_5$ | N | |
| 3.38 | " | $-CH_2COC(CH_3)_3$ | $-C_2H_5$ | N | |
| 3.39 | " | $-CH_2CH=CH_2$ | $-C_2H_5$ | CH | |
| 3.40 | 2-Cl, 4-F | $-CH_2CH=CH_2$ | $-C_2H_5$ | N | |
| 3.41 | " | $-CH_3$ | $-CH_3$ | N | |
| 3.42 | 2-Br, 4-Br | $-CH_2CH=CH_2$ | $-C_2H_5$ | N | |
| 3.43 | 2,4-Di-Cl | $-CH_2CH=CH_2$ | $-C_4H_9(n)$ | N | Oel |
| 3.44 | 4-Br | $-CH_3$ | $-C_2H_5$ | N | Fp. 65-67°C |

Tabelle 4 : Verbindungen der Formel I, worin Ar = Phenyl und R = —$COSR_6$ bedeuten

| Verb. Nr. | $R_1$, $R_2$, $R_3$ | $R_4$ | $R_6$ | X | n | Physik. Daten |
|---|---|---|---|---|---|---|
| 4.1 | H | H | $-C_2H_5$ | N | 0 | |
| 4.2 | 2,4-Di-Cl | H | $-CH_3$ | N | 0 | |
| 4.3 | " | H | $-C_2H_5$ | N | 0 | Fp. 180-182°C |
| 4.4 | " | H | $-C_2H_5$ | CH | 0 | |
| 4.5 | " | H | $-C_3H_7(n)$ | N | 0 | |
| 4.6 | " | H | $-CH_2-C_6H_5$ | N | 0 | Fp. 171-173°C |
| 4.7 | " | H | $-C_6H_5$ | N | 0 | Fp.164-166° |
| 4.8 | " | $-CH_2CH=CH_2$ | $-C_2H_5$ | N | 0 | |
| 4.9 | " | $-CH_2-C_6H_5$ | $-C_2H_5$ | N | 0 | |
| 4.10 | " | $-CH_3$ | $-C_2H_5$ | N | 1 | |
| 4.11 | " | $-CH_3$ | $-C_2H_5$ | CH | 1 | |
| 4.12 | " | $-CH_2OCH_3$ | $-C_2H_5$ | N | 1 | |
| 4.13 | " | $-C_2H_5$ | $-C_2H_5$ | N | 1 | |
| 4.14 | 2-Cl, 4-Br | H | $-CH_3$ | N | 0 | |
| 4.15 | " | H | $-C_2H_5$ | N | 0 | |
| 4.16 | " | H | $-CH_2-C_6H_5$ | N | 0 | |
| 4.17 | " | H | $-C_6H_5$ | N | 0 | |
| 4.18 | " | H | $-C_2H_5$ | N | 0 | |
| 4.19 | " | $-CH_2CH=CH_2$ | $-C_2H_5$ | N | 0 | |
| 4.20 | " | $-CH_3$ | $-C_2H_5$ | N | 1 | |
| 4.21 | " | $-C_2H_5$ | $-C_2H_5$ | N | 1 | |
| 4.22 | " | $-CH_2OCH_3$ | $-C_2H_5$ | N | 1 | |
| 4.23 | 4-Cl | H | $-C_2H_5$ | N | 0 | |
| 4.24 | 2-Cl, 4-F | H | $-C_2H_5$ | N | 0 | |
| 4.25 | " | H | $-CH_3$ | N | 0 | |
| 4.26 | " | H | $-C_2H_5$ | CH | 0 | |
| 4.27 | " | $-CH_3$ | $-C_2H_5$ | N | 1 | |
| 4.28 | 2,4-Di-Br | H | $-C_2H_5$ | N | 0 | |
| 4.29 | " | $-CH_3$ | $-C_2H_5$ | N | 1 | |

Tabelle 4 (Fortsetzung)

| Verb. Nr. | $R_1$, $R_2$, $R_3$ | $R_4$ | $R_6$ | X | n | Physik. Daten |
|---|---|---|---|---|---|---|
| 4.30 | 2,4-Di-Br | $-OC_2H_5$ | $-C_2H_5$ | N | 1 | |
| 4.31 | " | $-SC_2H_5$ | $-C_2H_5$ | N | 1 | |
| 4.32 | 2,4-Di-Cl | H | $-CH_2CH=CH_2$ | N | 0 | |
| 4.33 | " | $-CH_3$ | $-CH_2CH=CH_2$ | N | 1 | |
| 4.34 | " | $-OCH_3$ | $-CH_3$ | N | 1 | |
| 4.35 | " | $-OC_2H_5$ | $-C_2H_5$ | N | 1 | |
| 4.36 | " | $-OCH_2-C_6H_5$ | $-C_2H_5$ | N | 1 | |
| 4.37 | " | $-O-C_6H_5$ | $-C_2H_5$ | N | 1 | |
| 4.38 | " | -O- 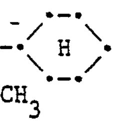 | $-C_2H_5$ | N | 1 | |
| 4.39 | " | $-SCH_3$ | $-CH_3$ | N | 1 | |
| 4.40 | " | $-SC_2H_5$ | $-C_2H_5$ | N | 1 | |
| 4.41 | " | $-SCH_2-C_6H_5$ | $-CH_2-C_6H_5$ | N | 1 | |
| 4.42 | 2-Cl, 4-F | $-OC_2H_5$ | $-C_2H_5$ | N | 1 | |
| 4.43 | " | $-OCH_3$ | $-CH_3$ | N | 1 | |
| 4.44 | " | $-OCH_3$ | $-CH_3$ | N | 1 | |
| 4.45 | " | $-SC_2H_5$ | $-C_2H_5$ | N | 1 | |
| 4.46 | 2-Cl, 4-Cl | $-OCH_3$ | $-CH_3$ | N | 2 | |
| 4.47 | " | $-OC_2H_5$ | $-CH_3$ | N | 2 | |
| 4.48 | " | $-OC_2H_5$ | $-C_2H_5$ | N | 2 | |
| 4.49 | 2-Cl, 4-Br | $-OCH_3$ | $-C_2H_5$ | N | 2 | |
| 4.50 | " | $-OC_2H_5$ | $-C_2H_5$ | N | 2 | |
| 4.51 | " | $-N(CH_3)_2$ | $-C_2H_5$ | N | 1 | |
| 4.52 | " | $-N(C_2H_5)_2$ | $-C_2H_5$ | N | 1 | |
| 4.53 | 2-Cl, 4-Cl | $-N(CH_3)_2$ | $-CH_3$ | N | 1 | |
| 4.54 | 2-Cl, 4-Cl | $-N(C_2H_5)_2$ | $-CH_3$ | N | 1 | |
| 4.55 | " | $-N(C_4H_9sec)_2$ | $-CH_3$ | N | 1 | |
| 4.56 | " | 1-(1,2,4-triazolyl) | $-CH_3$ | N | 1 | |
| 4.57 | " | -1-imidazolyl | $-CH_3$ | N | 1 | |
| 4.58 | " | $-N(CH_3)_2$ | $-C_2H_5$ | N | 1 | |

Tabelle 6 : Azoliumsalze der Formel

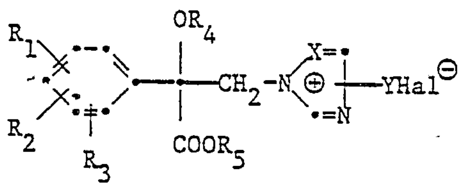

(XXXVII)

(wobei nicht genannte Substituenten $R_1$, $R_2$, $R_3$ stets Wasserstoff bedeuten.)

| Verb. Nr. | $R_1$, $R_2$, $R_3$ | $R_4$ | $R_5$ | X | Y Hal | Physik. Konstante |
|---|---|---|---|---|---|---|
| 6.1 | 4-Br | H | $CH_3$ | N | $CH_3J$ | Fp. 150-157°C (Zers.) |
| 6.2 | 2,4-Di-Cl | H | $CH_3$ | N | $CH_3J$ | |
| 6.3 | 4-Cl | $CH_3$ | $CH_3$ | N | $CH_3J$ | Fp. |
| 6.4 | 4-Br | $CH_3$ | $CH_3$ | N | $CH_3J$ | 88-96°C |
| 6.5 | 2,4-Di-Cl | H | $CH_3$ | N | $BrCH_2COCH_3$ | |
| 6.6 | 2,4-Di-Cl | H | $CH_3$ | N | $ClCH_2C_6H_5$ | |
| 6.7 | 4-Br | H | $CH_3$ | N | $BrCH_2COC_6H_5$ | |
| 6.8 | 4-Cl | $CH_3$ | $CH_3$ | N | $ClCH_2CH_2SO_2CH_3$ | |
| 6.9 | 4-Cl | H | $CH_3$ | N | $BrCH(C_6H_5)_2$ | |
| 6.10 | 2,4-Di-Cl | H | $CH_3$ | N | $BrCH(CH_3)COOCH_3$ | |
| 6.11 | 2,4-Di-Cl | H | $CH_3$ | N | $BrCH_2COC_6H_3Cl_2(2,4)$ | |
| 6.12 | 2-Cl, 4-Br | H | $C_2H_5$ | N | $BrCH_2COC_6H_3Cl_2(2,4)$ | Fp. 173-176°C |
| 6.13 | 4-Br | $C_2H_5$ | $C_2H_5$ | N | $JC_4H_9$-n | |
| 6.14 | 4-Cl | $CH_3$ | $CH_3$ | N | $ClCH_2C_6H_5Cl(4)$ | |
| 6.15 | 2-Cl, 4-Br | H | $CH_3$ | N | $ClCH_2CN$ | |
| 6.16 | 2,3,4-Tri-Cl | H | $CH_3$ | N | $JC_3H_7$-n | |
| 6.17 | 4-Br | H | $CH_3$ | CH | $CH_3J$ | |
| 6.18 | 2,4-Di-Cl | H | $CH_3$ | N | $ClCH_2SCH_3$ | |
| 6.19 | 2,4-Di-Cl | $CH_3$ | $CH_3$ | N | $JCH_2CH_3$ | |
| 6.20 | 2,4-Di-Cl | H | $CH_3$ | CH | $JCH_2CH_3$ | |
| 6.21 | 2,4-Di-Cl | $CH_3$ | $CH_3$ | CH | $JCH_2CH_3$ | Fp. |
| 6.22 | 2-Cl, 4-Br | H | $C_2H_5$ | N | 1/2 $CuCl_2$ | 171-177°C |

Tabelle 7 : Verbindungen der Formel

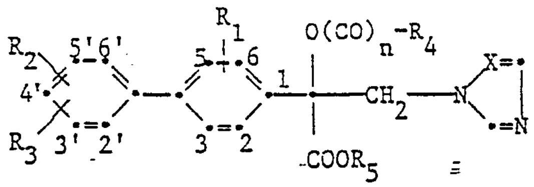

(XXXVIII)

| Verb. Nr. | $R_1$ | $R_2$ | $R_3$ | n | $R_4$ | $R_5$ | X | Physik. Konst. |
|---|---|---|---|---|---|---|---|---|
| 7.1 | H | H | H | 0 | $CH_3$ | $CH_3$ | N | |
| 7.2 | H | H | H | 0 | H | H | N | Fp. >200°C |
| 7.3 | H | H | H | 0 | H | $CH_3$ | N | Fp. >182°C |
| 7.4 | H | H | H | 0 | H | $CH_3$ | CH | |
| 7.5 | H | H | H | 0 | H | $C_2H_5$ | N | |
| 7.6 | H | H | H | 0 | H | $C_4H_9$-n | N | |
| 7.7 | H | 4'-Cl | H | 0 | H | $CH_3$ | N | |
| 7.8 | H | 4'-Br | H | 0 | H | $C_2H_5$ | N | |
| 7.9 | 2-$OCH_3$ | H | H | 0 | $CH_3$ | $C_3H_7$-n | N | |
| 7.10 | H | 2'-$OCH_3$ | 3'-Cl | 0 | H | $C_2H_5$ | N | |
| 7.11 | H | 4'-$CH_3$ | H | 0 | H | $CH_3$ | N | |
| 7.12 | H | 4'-$CH_3$ | H | 0 | $C_2H_5$ | $CH_3$ | CH | |
| 7.13 | H | 4'-Cl | H | 1 | $CH_3$ | $CH_3$ | N | |
| 7.14 | H | 4'-Cl | H | 1 | $CH_2CH=CH_2$ | $CH_3$ | N | |
| 7.15 | H | 4'-Cl | H | 1 | $CH_2CH_2CN$ | $CH_3$ | N | |
| 7.16 | H | 4'-Cl | H | 1 | $CCl=CCl_2$ | $CH_3$ | N | |
| 7.17 | H | 4'-Cl | H | 1 | 2-Tetrahydro-furyl | $C_2H_5$ | N | |
| 7.18 | H | 4'-Br | H | 1 | 2-Tetrahydro-furyl | $CH_3$ | CH | |
| 7.19 | H | 4'-Br | H | 0 | H | H | N | |
| 7.20 | H | H | H | 0 | H | $C_6H_3Cl_2$(2,4) | N | |
| 7.21 | H | 4'-Cl | H | 1 | $CH_3$ | $C_6H_3Cl_2$(2,4) | N | |
| 7.22 | H | 4'-Cl | H | 1 | Cyclopropyl | $C_6H_4Cl$(4) | N | |
| 7.23 | 2-Cl | H | H | 0 | H | $CH_3$ | N | Fp. 174-182°C |

Tabelle 8 : Verbindungen der Formel

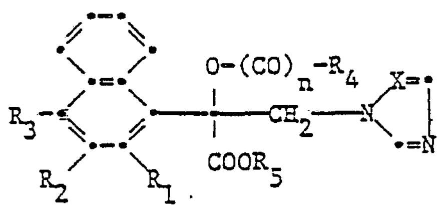

(XXXIX)

| Verb. Nr. | $R_1$ | $R_2$ | $R_3$ | n | $R_4$ | $R_5$ | X | Physik. Konst. |
|---|---|---|---|---|---|---|---|---|
| 8.1 | H | H | H | 0 | H | H | N | |
| 8.2 | H | H | H | 0 | H | H | CH | |
| 8.3 | H | H | H | 0 | $CH_3$ | $CH_3$ | N | |
| 8.4 | H | H | H | 0 | $CH_3$ | $CH_3$ | CH | |
| 8.5 | H | H | H | 0 | H | $CH_3$ | N | Fp. >200°C |
| 8.6 | H | H | H | 0 | H | $CH_3$ | CH | |
| 8.7 | H | H | H | 0 | H | $C_2H_5$ | N | |
| 8.8 | H | H | H | 1 | $CH_3$ | $CH_3$ | N | |
| 8.9 | H | H | H | 1 | $C_2H_5$ | $C_2H_5$ | N | |
| 8.10 | H | H | H | 1 | $CCl=CCl_2$ | $CH_3$ | N | |
| 8.11 | H | H | H | 1 | Cyclopropyl | $CH_3$ | N | |
| 8.12 | Cl | H | H | 0 | H | $CH_3$ | N | Fp. 195°C (Zers.) |
| 8.13 | Cl | H | H | 0 | H | H | N | |
| 8.14 | Br | Cl | H | 0 | $CH_3$ | $CH_3$ | N | |
| 8.15 | H | H | Cl | 1 | $CH_2OCH_3$ | $C_6H_4Cl(4)$ | N | |
| 8.16 | $CH_3$ | H | H | 0 | H | H | N | |
| 8.17 | H | $CH_3$ | $CH_3$ | 0 | $CH_3$ | $CH_3$ | N | |
| 8.18 | H | H | $OCH_3$ | 0 | $CH_2CH=CH_2$ | $CH_2CH=CH_2$ | N | |
| 8.19 | H | $CH_3$ | H | 0 | $CH_2CH=CH_2$ | $CH_2CH=CH_2$ | N | |
| 8.20 | H | H | H | 0 | H | $CH_2C_6H_5$ | CH | |
| 8.21 | H | H | H | 0 | H | $CH_3$ | N | |
| 8.22 | H | H | H | 0 | H | $CH_2C_6H_5$ | N | |

Tabelle 9 : Verbindungen der Formel

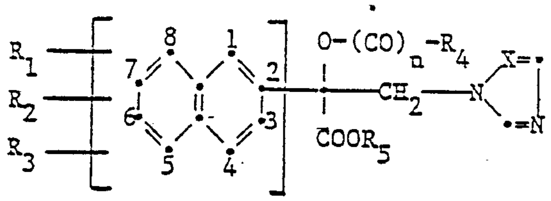

(XXXX)

| Verb. Nr. | $R_1$ | $R_2$ | $R_3$ | n | $R_4$ | $R_5$ | X | Physik. Konst. |
|---|---|---|---|---|---|---|---|---|
| 9.1 | H | H | H | 0 | H | H | N | |
| 9.2 | H | H | H | 0 | H | H | CH | |
| 9.3 | H | H | H | 0 | $CH_3$ | $CH_3$ | N | – |
| 9.4 | H | H | H | 0 | $CH_3$ | $CH_3$ | CH | |
| 9.5 | H | H | H | 0 | H | $CH_3$ | N | Fp. 154-158°C |
| 9.6 | H | H | H | 0 | H | $CH_3$ | CH | |
| 9.7 | H | H | H | 0 | H | $C_2H_5$ | N | Fp. 165°C (Zers.) |
| 9.8 | H | H | H | 1 | $CH_3$ | $CH_3$ | N | |
| 9.9 | H | H | H | 1 | $C_2H_5$ | $C_2H_5$ | N | |
| 9.10 | H | H | H | 1 | $CCl=CCl_2$ | $CH_3$ | N | |
| 9.11 | H | H | H | 1 | Cyclopropyl | $CH_3$ | N | Fp. 181-186°C |
| 9.12 | 6-Cl | H | H | 0 | H | $CH_3$ | N | |
| 9.13 | 6-Cl | H | H | 0 | H | H | N | |
| 9.14 | 6-Cl | 7-Cl | H | 0 | $CH_3$ | $CH_3$ | N | |
| 9.15 | 5-F | 7-F | H | 1 | $CH_2OCH_3$ | $C_6H_4Cl(4)$ | N | |
| 9.16 | 5-$CH_3$ | H | H | 0 | H | H | N | |
| 9.17 | 1-$CH_3$ | 8-$CH_3$ | 4-$CH_3$ | 0 | $CH_3$ | $CH_3$ | N | |
| 9.18 | 5-Cl | 6-$OCH_3$ | H | 0 | $CH_2CH=CH_2$ | $CH_2CH=CH_2$ | N | |
| 9.19 | 6-Br | H | H | 0 | $CH_2CH=CH_2$ | $CH_2CH=CH_2$ | N | |
| 9.20 | 5-F | 7-F | H | 0 | H | $CH_3$ | N | |
| 9.21 | 1-$OCH_3$ | 6-$OCH_3$ | H | 0 | H | $CH_3$ | N | |
| 9.22 | 6-J | H | H | 1 | $CCl=CCl_2$ | $CH_3$ | N | |
| 9.23 | H | H | H | 1 | Cyclohexyl | $CH_3$ | N | |
| 9.24 | 6-Cl | 7-Cl | H | 1 | $CCl=CCl_2$ | $CH_3$ | N | |

Formulierungsbeispiele für Wirkstoffe der Formel I (% = Gewichtsprozent)

| A. Emulsions-Konzentrate | a) | b) | c) |
|---|---|---|---|
| Wirkstoff aus den Tabellen 1 bis 9 | 25% | 40% | 50% |
| Ca-Dodecylbenzolsulfonat | 5% | 8% | 6% |
| Ricinusöl-polyethylenglykolether (36 Mol Ethylenoxid) | 5% | - | - |
| Tributylphenoyl-polyethylenglykol-ehter (30 Mol Ethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| B. Lösungen | a) | b) | c) | d) |
|---|---|---|---|---|
| Wirkstoff aus den Tabellen 1 bis 9 | 80% | 10% | 5% | 95% |
| Ethylenglykol-monomethyl-ether | 20% | - | - | - |
| Polyethylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

(MG = Molekulargewicht)

Die Lösungen sind zur Anwendung in Form kleinster Tropfen geeignet.

| C. Granulate | a) | b) |
|---|---|---|
| Wirkstoff aus den Tabellen 1 bis 9 | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| D. Stäubemittel | a) | b) |
|---|---|---|
| Wirkstoff aus den Tabellen 1 bis 9 | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

| E. Spritzpulver | a) | b) | c) |
|---|---|---|---|
| Wirkstoff aus den Tabellen 1 bis 9 | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyethylenglykolether (7-8 Mol Ethylenoxid) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

## Biologische Beispiele

### Beispiel B1 : Wirkung gegen Puccinia graminis auf Weizen

a) Residual-protektive Wirkung

Weizenpflanzen wurden 6 Tage nach der Aussaat mit einer aus Spritzpulver des Wirkstoffes hergestellten Spritzbrühe (0,06 % Aktivsubstanz) besprüht. Nach 24 Stunden wurden die behandelten Pflanzen mit einer Uredosporensuspension des Pilzes infiziert. Nach einer Inkubation während 48 Stunden bei 95-100 % relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

b) Systemische Wirkung

Zu Weizenpflanzen wurde 5 Tage nach der Aussaat eine aus Spritzpulver des Wirkstoffes hergestellte Spritzbrühe gegossen (0,006 % Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95-100 % relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

Unbehandelte, aber infizierte Kontrollpflanzen zeigten einen Befall von 100 %. Pflanzen, die mit Mitteln enthaltend Verbindungen der Formel I behandelt waren, zeigten nur geringen (< 20 %) oder keinen Befall. Verbindungen der Untergruppe T sowie ihre Derivate mit n = 0 und $R_4$ = $C_1$-$C_4$ Alkyl und n = 1 und $R_4$ = $C_1$-$C_4$ Alkyl, $C_1$-$C_4$ Alkoxy und $C_1$-$C_4$ Alkylthio (insgesamt Untergruppe $T_2$ genannt) und andere Verbindungen hemmten den Befall auf weniger als 10 %. Die Verbindungen Nr. 1.1, 1.4-1.6, 1.8-1.14, 1.24, 1.27, 1.32, 1.40, 1.55, 1.100, 1.101, 1.116, 2.2-2.6, 2.66-2.68, 2.92, 2.109, 2.132, 2.149, 2.157, 2.161 unterdrückten den Pilzbefall vollständig (0-5 %). Verbindung Nr. 1.1 verhinderte den Pilzbefall auch noch bei einer Konzentration von 0,002 % vollständig.

### Beispiel B2 : Wirkung gegen Cercospora arachidicola auf Erdnusspflanzen

a) Residual-protektive Wirkung

10-15 cm hohe Erdnusspflanzen wurden mit einer aus Spritzpulver der Wirksubstanz hergestellten Spritzbrühe (0,006 Aktivsubstanz) besprüht und 48 Stunden später mit einer Konidiensuspension des Pilzes infiziert. Die infizierten Pflanzen wurden während 72 Stunden bei ca. 21 °C und hoher Luftfeuchtigkeit inkubiert und anschliessend bis zum Auftreten der typischen Blattflecken in einem Gewächshaus aufgestellt. Die Beurteilung der fungiziden Wirkung erfolgte 12 Tage nach der Infektion basierend auf Anzahl und Grösse der auftretenden Flecken.

b) Systemische Wirkung

Zu 10-15 cm hohen Erdnusspflanzen wurde eine aus Spritzpulver des Wirkstoffes hergestellte Spritzbrühe gegossen (0,06 % Aktivsubstanz bezogen auf das Erdvolumen). Nach 48 Stunden wurden die behandelten Pflanzen mit einer Konidiensuspension des Pilzes infiziert und 72 Stunden bei ca. 21 °C und hoher Luftfeuchtigkeit inkubiert. Anschliessend wurden die Pflanzen im Gewächshaus aufgestellt und nach 11 Tagen der Pilzbefall beurteilt.

Im Vergleich zu unbehandelten, aber infizierten Kontrollpflanzen (Anzahl und Grösse der Flecken = 100 %), zeigten Erdnusspflanzen, die mit Wirkstoffen aus den Tabellen 1 bis 13 behandelt wurden, einen stark reduzierten Cercospora-Befall. So verhinderten die Verbindungen Nr. 1.1 bis 1.15, 2.2-2.4, 2.66, 2.92, 2.161, 7.1-7.3, 7.23, 8.3, 8.5, 9.5 und andere in obigen Versuchen das Auftreten von Flecken fast vollständig (0-10 %).

### Beispiel B3 : Wirkung gegen Erysiphe graminis auf Gerste

a) Residual-protektive Wirkung

Ca. 8 cm hohe Gerstenpflanzen wurden mit einer aus Spritzpulver des Wirkstoffes hergestellten Spritzbrühe (0,02 % Aktivsubstanz) besprüht. Nach 3-4 Stunden wurden die behandelten Pflanzen mit Konidien des Pilzes bestäubt. Die infizierten Gerstenpflanzen wurden in einem Gewächshaus bei ca.

22 °C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,006 % Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

Verbindungen der Formel I bzw. Verbindungen aus den Tabellen 1 bis 13 reduzierten den Pilzbefall unter 20 %, während unbehandelte aber infizierte Kontrollpflanzen zu 100 % befallen waren. Verbindungen der Untergruppen $T_2$ und T des Beispiels B1 hemmten den Befall auf weniger als 10 %. Eine vollständige Hemmung des Pilzbefalls (0-5 %) wurde unter anderen mit den Verbindungen Nr. 1.1-1.19, 1.24-1.27, 1.29, 1.32, 1.34, 1.40, 1.43, 1.55, 1.58, 1.109-1.111, 1.116, 2.2, 2.6, 2.66, 2.92, 2.109-2.112, 2.132, 2.149, 2.157, 2.161-2.170, 3.2-3.4, 4.1-4.3, 4.40, 6.22, 7.23, 8.5, 9.11 erzielt.

Beispiel B4 : Residual-protektive Wirkung gegen Venturia inaequalis auf Apfeltrieben

Apfelstecklinge mit 10-20 cm langen Frischtrieben wurden mit einer aus Spritzpulver des Wirkstoffes hergestellten Spritzbrühe (0,06 % Aktivsubstanz) besprüht. Nach 24 Stunden wurden die behandelten Pflanzen mit einer Konidiensuspension des Pilzes infiziert. Die Pflanzen wurden dann während 5 Tagen bei 90-100 % relativer Luftfeuchtigkeit inkubiert und während 10 weiteren Tagen in einem Gewächshaus bei 20-24 °C aufgestellt. Der Schorfbefall wurde 15 Tage nach der Infektion beurteilt. Verbindungen 1.1 bis 1.10, 1.14, 1.40, 1.55, 1.83, 1.110, 1.111, 1.116, 2.2, 2.109, 2.116 und andere hemmten den Krankheitsbefall auf weniger als 10 % oder zeigten keinen Krankheitsbefall (z. B. Verb. 1.1 und 7.3).

Triebe an Apfelbäumen werden im Freiland in gleichem Masse geschützt, ohne in ihrer Weiterentwicklung gehemmt zu werden.

Beispiel B5 : Wirkung gegen Botrytis cinerea auf Bohnen

Residual-protektive Wirkung

Ca. 10 cm hohe Bohnen-Pflanzen wurden mit einer aus Spritzpulver des Wirkstoffes hergestellten Spritzbrühe (0,02 % Aktivsubstanz) besprüht. Nach 48 Stunden wurden die behandelten Pflanzen mit einer Konidiensuspension des Pilzes infiziert. Nach einer Inkubation der infizierten Pflanzen während 3 Tagen bei 95-100 % relativer Luftfeuchtigkeit und 21 °C erfolgte die Beurteilung des Pilzbefalls. Die Verbindungen aus den Tabellen 1 bis 9 hemmten in vielen Fällen die Pilzinfektion sehr stark. Bei einer Konzentration von 0,02 % erwiesen sich z. B. die Verbindungen Nr. 1.1, 1.6 und 6.1 als voll wirksam (Krankheitsbefall 0 bis 5 %).

Beispiel B6 : Wirkung gegen Piricularia oryzae auf Reis

Residual-protektive Wirkung

Reispflanzen wurden nach zweiwöchiger Anzucht mit einer aus Spritzpulver des Wirkstoffes hergestellten Spritzbrühe (0,02 % Aktivsubstanz) besprüht. Nach 48 Stunden wurden die behandelten Pflanzen mit einer Konidiensuspension des Pilzes infiziert. Nach 5 Tagen Inkubation bei 95-100 % relativer Luftfeuchtigkeit und 24 °C wurde der Pilzbefall beurteilt.

Gegenüber 100 % Befall bei ungeschützten Pflanzen hemmten die Verbindungen Nr. 1.27 und 2.68 den Pilzbefall vollständig (0-5 % Befall).

**Patentansprüche** (für die Vertragsstaaten : BE, CH, DE, FR, IT, LI, LU, NL, SE)

1. Mandelsäure-Derivate der Formel I

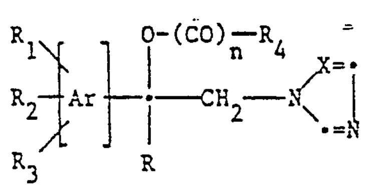

(I)

worin

X für das Brückenglied —CH= oder —N= steht ;

Ar eine Phenyl-, Diphenyl- oder Naphthylgruppe bedeutet ;

$R_1$, $R_2$, $R_3$ unabhängig voneinander für Wasserstoff, Nitro, Halogen, $C_1$-$C_3$-Alkyl, $C_1$-$C_3$-Alkoxy oder $C_1$-$C_3$-Haloalkyl stehen ;

R eine der Gruppen $—COOR_5$ oder $—COSR_6$ darstellt,

n für die Zahlen 0, 1 oder 2 steht,

wobei in den Fällen, bei denen n für die Zahl 0 steht,

$R_4$ Wasserstoff, $C_1$-$C_{12}$-Alkyl, $C_3$-$C_6$-Alkenyl, $C_3$-$C_6$-Haloalkenyl oder ein durch $C_1$-$C_4$-Alkoxy, $C_2$-$C_4$-Alkenyl, $C_2$-$C_4$-Haloalkenyl, $C_2$-$C_4$-Alkinyl, Cyano oder Phenyl substituiertes $C_1$-$C_{12}$-Alkyl bedeutet, wobei jeder $C_2$-$C_{12}$-Alkylsubstituent gegebenenfalls durch eine Carbonylgruppe oder eine Carbamoyloxygruppe (—O—CONH—) unterbrochen sein kann ;

wobei ferner in den Fällen, bei denen n für die Zahl 1 steht,

$R_4$ $C_1$-$C_{12}$-Alkyl, Phenyl, $C_1$-$C_4$-Alkoxy, $C_1$-$C_4$-Alkylthio, $C_2$-$C_6$-Alkenyl, $C_3$-$C_5$-Alkinyl, $C_2$-$C_6$-Haloalkenyl, $C_3$-$C_7$-Cycloalkyl, Furyl, Tetrahydrofuryl, Pyridyl, 1-Imidazolyl, 1-(1,2,4-Triazolyl) oder eine unsubstituierte oder durch Halogen, $C_1$-$C_4$-Alkyl, $C_1$-$C_4$-Alkoxy, —CN oder $—CF_3$ substituierte Phenylgruppe darstellt oder ein durch $C_1$-$C_4$-Alkoxy, $C_1$-$C_4$-Alkylthio, $C_2$-$C_4$-Alkenyl, $C_2$-$C_4$-Haloalkenyl, $C_2$-$C_4$-Alkinyl, Cyano oder Phenyl substituiertes $C_1$-$C_{12}$-Alkyl bedeutet, wobei jeder heterocyclische Substituent unsubstituiert oder durch Halogen und/oder Methyl ein- oder mehrfach substituiert ist und wobei ferner jeder $C_2$-$C_{12}$-Alkylsubstituent gegebenenfalls durch eine Carbonylgruppe oder eine Carbamoyloxygruppe unterbrochen sein kann ; und wobei $R_4$ ferner die Gruppe $—N(C_1$-$C_8$-$Alkyl)_2$ darstellen kann ;

wobei ferner in den Fällen, bei denen n für die Zahl 2 steht,

$R_4$ $C_1$-$C_{12}$-Alkyl oder eine unsubstituierte oder durch Halogen, $C_1$-$C_4$-Alkyl, $C_1$-$C_4$-Alkoxy, —CN oder $—CF_3$ substituierte Benzylgruppe bedeutet,

und worin ferner

$R_5$ Wasserstoff, ein durch Halogen substituiertes $C_2$-$C_{10}$ Alkenyl, ein unsubstituiertes oder durch Halogen substituiertes $C_2$-$C_{10}$ Alkinyl, eine $C_3$-$C_8$ Cycloalkylgruppe, eine unsubstituierte oder durch Halogen und/oder Methyl substituierte Phenylgruppe oder eine $C_1$-$C_4$ Alkylgruppe bedeutet, oder aber eine $C_1$-$C_{12}$ Alkylkette darstellt, die ab $C_2$-Alkyl durch Sauerstoff oder Schwefel unterbrochen und/oder die durch eine der folgenden Atome oder Gruppen substituiert ist : Halogen, Phenyl, —COO Alkyl($C_1$-$C_4$), —CO Alkyl($C_1$-$C_4$), —CO Phenyl, einen ungesättigten oder gesättigten 5- oder 6-gliedrigen Ring mit Sauerstoff oder Schwefel als Heteroatom, und

$R_6$ $C_1$-$C_{10}$ Alkyl oder eine unsubstituierte oder durch Halogen, $C_1$-$C_4$-Alkyl, $C_1$-$C_4$ Alkoxy, —CN oder $—CF_3$ substituierte Phenyl- oder Benzylgruppe bedeutet, und

worin Säureadditionssalze, quaternäre Azolium- und Ammoniumsalze sowie Metallkomplexe der Formel I mit eingeschlossen sind.

2. Verbindungen der Formel I gemäss Anspruch 1, bei denen X, Ar, R, $R_1$, $R_2$, $R_3$ die für die Formel I angegebenen Bedeutungen haben, während n = 0 und $R_4$ = Wasserstoff bedeuten.

3. Verbindungen der Formel I gemäss Anspruch 2, worin Ar eine Phenylgruppe bedeutet und R eine der Gruppen $—COOR_5$, oder $—COSR_6$ darstellt, wobei X, $R_1$, $R_2$, $R_3$, $R_5$ und $R_6$ die für Formel I gegebene Bedeutung haben.

4. Verbindungen der Formel I gemäss Anspruch 3, worin Ar Phenyl bedeutet, $R_1$ für Halogen, Methyl, Methoxy oder $CF_3$ steht und $R_2$ und $R_3$ unabhängig voneinander Wasserstoff, Fluor, Chlor, Brom, Methyl oder Methoxy darstellen, während X, $R_5$ und $R_6$ die unter Formel I gegebene Bedeutung besitzen.

5. Verbindungen der Formel I gemäss Anspruch 4, worin $R_5$ Wasserstoff, $C_1$-$C_4$ Alkyl, eine unsubstituierte oder durch Halogen, $C_1$-$C_4$ Alkyl, $C_1$-$C_4$ Alkoxy, —CN oder $—CF_3$ substituierte Phenylgruppe bedeutet ; und $R_6$ $C_1$-$C_6$ Alkyl oder eine Phenyl- oder Benzylgruppe darstellt.

6. Mandelsäurederivate der Formel I*

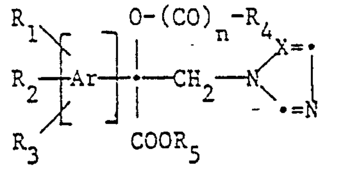

(I*)

worin

X für das Brückenglied —CH= oder —N= steht ;

Ar eine Phenyl-, Diphenyl- oder Naphthylgruppe bedeutet ;

$R_1$, $R_2$, $R_3$ unabhängig voneinander für Wasserstoff, Nitro, Halogen, $C_1$-$C_3$-Alkyl, $C_1$-$C_3$-Alkoxy oder $C_1$-$C_3$-Haloalkyl stehen ;

n für die Zahlen 0 oder 1 steht und in den Fällen,

in denen n für die Zahl 0 steht,

$R_4$ Wasserstoff, $C_1$-$C_{12}$-Alkyl, $C_3$-$C_6$-Alkenyl, $C_3$-$C_6$-Haloalkenyl oder ein durch $C_1$-$C_4$-Alkoxy, $C_2$-$C_4$-Alkenyl, $C_2$-$C_4$-Haloalkenyl, $C_2$-$C_4$-Alkinyl, Cyano oder Phenyl substituiertes $C_1$-$C_{12}$-Alkyl bedeutet, wobei jeder $C_2$-$C_{12}$-Alkylsubstituent gegebenenfalls durch eine Carbonylgruppe unterbrochen ist ;

und in den Fällen, in denen n für die Zahl 1 steht,

$R_4$ $C_1$-$C_{12}$-Alkyl, Phenyl, $C_1$-$C_4$-Alkoxy, $C_2$-$C_6$-Alkenyl, $C_3$-$C_5$-Alkinyl, $C_2$-$C_6$-Haloalkenyl, $C_3$-$C_7$-Cycloalkyl, Furyl, Tetrahydrofuryl, Pyridyl oder ein durch $C_1$-$C_4$-Alkoxy, $C_2$-$C_4$-Alkenyl, $C_2$-$C_4$-Haloalkenyl, $C_2$-$C_4$-Alkinyl, Cyano oder Phenyl substituiertes $C_1$-$C_{12}$-Alkyl bedeutet, wobei jeder cyclische Substituent unsubstituiert oder durch Halogen und/oder Methyl ein- oder mehrfach substituiert ist und wobei ferner jeder $C_2$-$C_{12}$-Alkylsubstituent gegebenenfalls durch eine Carbonylgruppe unterbrochen ist ; und

$R_5$ Wasserstoff, $C_1$-$C_4$-Alkyl, Phenyl, ein durch Nitro, Halogen und/oder Methyl ein- oder mehrfach substituiertes Phenyl oder Benzyl bedeutet ; unter Einschluss der pflanzenverträglichen Säureadditionssalze, quaternären Azoliumsalze sowie Metallkomplexe der Verbindungen der Formel I*.

7. Verbindungen der Formel I* gemäss Anspruch 6, worin X für CH oder N steht ; Ar Phenyl bedeutet ; $R_1$, $R_2$, $R_3$ unabhängig voneinander für Halogen, Methyl, Methoxy oder $CF_3$ stehen, n = 0 oder 1 bedeutet und $R_4$ und $R_5$ die für Formel I* angegebenen Bedeutungen haben.

8. Verbindungen der Formel I* gemäss Anspruch 7, worin X für N steht ; $R_1$, $R_2$, $R_3$ unabhängig voneinander für Fluor, Chlor, Brom, $CH_3$, $CH_3O$ oder $CF_3$ stehen, n die Zahl 0 bedeutet, $R_4$ Wasserstoff, $C_1$-$C_6$ Alkyl, $C_3$-$C_6$ Alkenyl oder $C_3$-$C_6$ Haloalkenyl steht und $R_5$ die unter Formel I* angegebenen Bedeutungen hat.

9. Verbindungen der Formel I* gemäss Anspruch 6, worin X, Ar, $R_1$, $R_2$, $R_3$ und $R_5$ die unter Formel I* angegebenen Bedeutungen haben, $R_4$ für Wasserstoff oder $C_1$-$C_3$ Alkyl steht und n die Zahl 0 bedeutet.

10. 2-(1H-1,2,4-Triazolylmethyl-1'-yl)-2-hydroxy-2,4-dichlorphenylessigsäureester gemäss Anspruch 6 der Formel

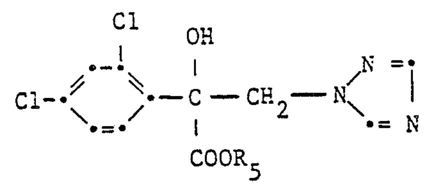

worin $R_5$ Methyl, Ethyl, n-Propyl, Isopropyl, n-Butyl oder tert.-Butyl bedeutet.

11. Die Metallkomplexe im Umfang der Formel I* nach Anspruch 6 mit den Metallen Kupfer, Zink, Mangan oder Zinn.

12. Verfahren zur Herstellung von Mandelsäure-Derivaten der Formel I gemäss Anspruch 1, dadurch gekennzeichnet, dass man Aryl-azolylmethylketone der Formel IX

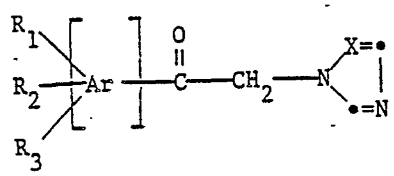

(IX)

mit HCN oder Alkalicyanid bei 0° bis 100 °C reagieren lässt, oder aber Verbindungen der Formel IX in Form ihres $NaHSO_3$-Addukts mit HCN oder Alkylicyanid bei 0° bis 100 °C zur Reaktion bringt und das so gewonnene Mandelonitril der Formel III

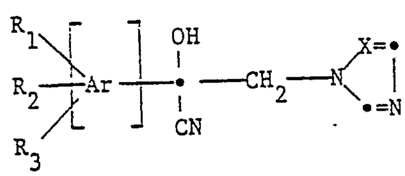

(III)

in einem basischen oder sauren Medium zu Mandelsäure-Derivaten der Formel Ia hydrolysiert

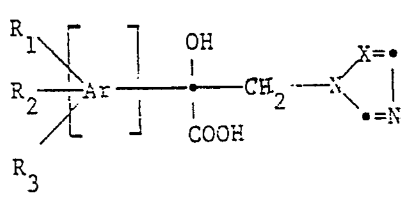

(Ia)

wobei in Formel IX, III und Ia die Substituenten X, Ar, $R_1$, $R_2$ und $R_3$ die unter Formel I angegebenen Bedeutungen haben, und, sofern gewünscht, weitere Derivatisierung der Verbindungen der Formel Ia an

der OH-Gruppe oder an der COOH-Gruppe, und/oder, sofern gewünscht, weitere Ueberführung in Säureadditionssalze, quaternäre Azolium- und Ammoniumsalze oder Metallkomplexe.

13. Verfahren zur Herstellung von Mandelsäureestern der Formel Ib

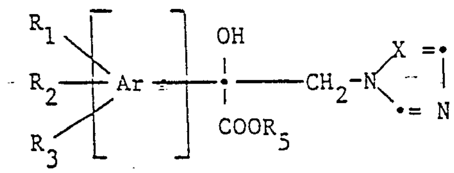

(Ib)

dadurch gekennzeichnet, dass man einen entsprechenden α-Halogenessigester der Formel IV

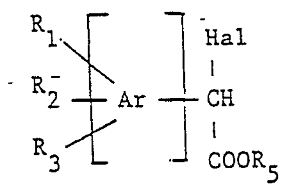

(IV)

mit Paraformaldehyd bei 0° bis 140 °C und a) dem gewünschten Azol der Formel VII

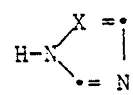

(VII)

in Gegenwart einer Base oder b) mit dem Alkalisalz des Azols in einem wasserfreien Lösungsmittel reagieren lässt, wobei in den Formeln Ib, IV und VII, Ar, $R_1$, $R_2$, $R_3$, X und $R_5$ die für Formel I gemäss Anspruch 1 gegebene Bedeutung haben, und Hal für ein Halogenatom steht.

14. Schädlingsbekämpfungsmittel zur Bekämpfung oder Verhütung eines Befalls durch Mikroorganismen, dadurch gekennzeichnet, dass es als mindestens eine aktive Komponente eine Verbindung der Formel I bzw. der Formel I* gemäss einem der Ansprüche 1 bis 11 enthält, zusammen mit Trägerstoffen.

15. Verfahren zur Bekämpfung oder Verhütung eines Befalls von Kulturpflanzen durch phytopathogene Mikroorganismen, dadurch gekennzeichnet, dass man eine Verbindung der Formel I bzw. der Formel I* gemäss einen der Ansprüche 1 bis 11 auf die Pflanze oder deren Standort appliziert.

**Patentansprüche** (für den Vertragsstaat AT)

1. Schädlingsbekämpfungsmittel zur Bekämpfung oder Verhütung eines Befalls durch Mikroorganismen, dadurch gekennzeichnet, dass es als mindestens eine aktive Komponente ein Mandelsäure derivat der Formel I enthält,

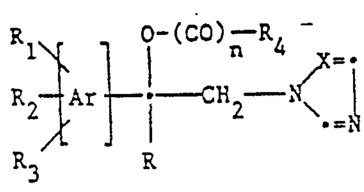

(I)

worin

X für das Brückenglied —CH= oder —N= steht ;

Ar eine Phenyl-, Diphenyl- oder Naphthylgruppe bedeutet ;

$R_1$, $R_2$, $R_3$ unabhängig voneinander für Wasserstoff, Nitro, Halogen, $C_1$-$C_3$-Alkyl, $C_1$-$C_3$-Alkoxy oder $C_1$-$C_3$-Haloalkyl stehen ;

R eine der Gruppen —$COOR_5$ oder —$COSR_6$ darstellt,

n für die Zahlen 0, 1 oder 2 steht,

wobei in den Fällen, bei denen n für die Zahl 0 steht,

$R_4$ Wasserstoff, $C_1$-$C_{12}$-Alkyl, $C_3$-$C_6$-Alkenyl, $C_3$-$C_6$-Haloalkenyl oder ein durch $C_1$-$C_4$-Alkoxy, $C_2$-$C_4$-Alkenyl, $C_2$-$C_4$-Haloalkenyl, $C_2$-$C_4$-Alkinyl, Cyano oder Phenyl substituiertes $C_1$-$C_{12}$-Alkyl bedeutet, wobei jeder $C_2$-$C_{12}$-Alkylsubstituent gegebenenfalls durch eine Carbonylgruppe oder eine Carbamoyloxygruppe (—O—CONH—) unterbrochen sein kann ;

wobei ferner in den Fällen, bei denen n für die Zahl 1 steht,

$R_4$ $C_1$-$C_{12}$-Alkyl, Phenyl, $C_1$-$C_4$-Alkoxy, $C_1$-$C_4$-Alkylthio, $C_2$-$C_6$-Alkenyl, $C_3$-$C_5$-Alkinyl, $C_2$-$C_6$-Haloalkenyl, $C_3$-$C_7$-Cycloalkyl, Furyl, Tetrahydrofuryl, Pyridyl, 1-Imidazolyl, 1-(1,2,4-Triazolyl) oder eine unsubstituierte oder durch Halogen, $C_1$-$C_4$-Alkyl, $C_1$-$C_4$-Alkoxy, —CN oder —$CF_3$ substituierte Phenylgruppe darstellt oder ein durch $C_1$-$C_4$-Alkoxy, $C_1$-$C_4$-Alkylthio, $C_2$-$C_4$-Alkenyl, $C_2$-$C_4$-Haloalkenyl, $C_2$-$C_4$-Alkinyl, Cyano oder Phenyl substituiertes $C_1$-$C_{12}$-Alkyl bedeutet, wobei jeder heterocyclische Substituent unsubstituiert oder durch Halogen und/oder Methyl ein- oder mehrfach substituiert ist und wobei ferner jeder $C_2$-$C_{12}$-Alkylsubstituent gegebenenfalls durch eine Carbonylgruppe oder eine Carbamoyloxygruppe unterbrochen sein kann ; und wobei $R_4$ ferner die Gruppe —N($C_1$-$C_8$-Alkyl)$_2$ darstellen kann ; wobei ferner in den Fällen, bei denen n für die Zahl 2 steht,

$R_4$ $C_1$-$C_{12}$-Alkyl oder eine unsubstituierte oder durch Halogen, $C_1$-$C_4$-Alkyl, $C_1$-$C_4$-Alkoxy, —CN oder —$CF_3$ substituierte Benzylgruppe bedeutet,
und worin ferner

$R_5$ Wasserstoff, ein durch Halogen substituiertes $C_2$-$C_{10}$ Alkenyl, ein unsubstituiertes oder durch Halogen substituiertes $C_2$-$C_{10}$ Alkinyl, eine $C_3$-$C_8$ Cycloalkylgruppe, eine unsubstituierte oder durch Halogen und/oder Methyl substituierte Phenylgruppe oder eine $C_1$-$C_4$ Alkylgruppe bedeutet, oder aber eine $C_1$-$C_{12}$ Alkylkette darstellt, die ab $C_2$-Alkyl durch Sauerstoff oder Schwefel unterbrochen und/oder die durch eine der folgenden Atome oder Gruppen substituiert ist : Halogen, Phenyl, —COOAlkyl($C_1$-$C_4$), —COAlkyl($C_1$-$C_4$), —COPhenyl, einen ungesättigten oder gesättigten 5- oder 6-gliedrigen Ring mit Sauerstoff oder Schwefel als Heteroatom, und

$R_6$ $C_1$-$C_{10}$ Alkyl oder eine unsubstituierte oder durch Halogen, $C_1$-$C_4$-Alkyl, $C_1$-$C_4$ Alkoxy, —CN oder —$CF_3$ substituierte Phenyl- oder Benzylgruppe bedeutet, und
worin Säureadditionssalze, quaternäre Azolium- und Ammoniumsalze sowie Metallkomplexe der Formel I mit eingeschlossen sind ; zusammen mit Trägerstoffen.

2. Mittel gemäß Anspruch 1 enthaltend eine Verbindung der Formel I, bei der X, Ar, R, $R_1$, $R_2$, $R_3$ die für die Formel I angegebenen Bedeutungen haben, während n = 0 und $R_4$ = Wasserstoff bedeuten.

3. Mittel gemäß Anspruch 1 enthaltend eine Verbindung der Formel I gemäss Anspruch 2, worin Ar eine Phenylgruppe bedeutet und R eine der Gruppen —$COOR_5$, oder —$COSR_6$ darstellt, wobei X, $R_1$, $R_2$, $R_3$, $R_5$ und $R_6$ die für Formel I gegebene Bedeutung haben.

4. Mittel gemäß Anspruch 1 enthaltend eine Verbindung der Formel I gemäss Anspruch 3, worin Ar Phenyl bedeutet, $R_1$ für Halogen, Methyl, Methoxy oder $CF_3$ steht und $R_2$ und $R_3$ unabhängig voneinander Wasserstoff, Fluor, Chlor, Brom, Methyl oder Methoxy darstellen, während X, $R_5$ und $R_6$ die unter Formel I gegebene Bedeutung besitzen.

5. Mittel gemäß Anspruch 1 enthaltend eine Verbindung der Formel I gemäss Anspruch 4, worin $R_5$ Wasserstoff, $C_1$-$C_4$ Alkyl, eine unsubstituierte oder durch Halogen, $C_1$-$C_4$ Alkyl, $C_1$-$C_4$ Alkoxy, —CN oder —$CF_3$ substituierte Phenylgruppe bedeutet ; und $R_6$ $C_1$-$C_6$ Alkyl oder eine Phenyl- oder Benzylgruppe darstellt.

6. Mittel gemäß Anspruch 1 enthaltend eine Verbindung der Formel I*

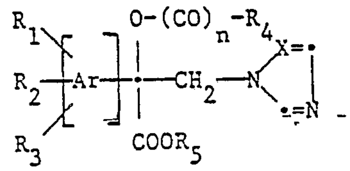

(I*)

worin

X für das Brückenglied —CH= oder —N= steht ;

Ar eine Phenyl-, Diphenyl- oder Naphthylgruppe bedeutet ;

$R_1$, $R_2$, $R_3$ unabhängig voneinander für Wasserstoff, Nitro, Halogen, $C_1$-$C_3$-Alkyl, $C_1$-$C_3$-Alkoxy oder $C_1$-$C_3$-Haloalkyl stehen ;

n für die Zahlen 0 oder 1 steht und in den Fällen,
in denen n für die Zahl 0 steht,

$R_4$ Wasserstoff, $C_1$-$C_{12}$-Alkyl, $C_3$-$C_6$-Alkenyl, $C_3$-$C_6$-Haloalkenyl oder ein durch $C_1$-$C_4$-Alkoxy, $C_2$-$C_4$-Alkenyl, $C_2$-$C_4$-Haloalkenyl, $C_2$-$C_4$-Alkinyl, Cyano oder Phenyl substituiertes $C_1$-$C_{12}$-Alkyl bedeutet, wobei jeder $C_2$-$C_{12}$-Alkylsubstituent gegebenenfalls durch eine Carbonylgruppe unterbrochen ist ;
und die den Fällen, in denen n für die Zahl 1 steht,

$R_4$ $C_1$-$C_{12}$-Alkyl, Phenyl, $C_1$-$C_4$-Alkoxy, $C_2$-$C_6$-Alkenyl, $C_3$-$C_5$-Alkiryl, $C_2$-$C_6$-Haloalkenyl, $C_3$-$C_7$-Cycloalkyl, Furyl, Tetrahydrofuryl, Pyridyl oder ein durch $C_1$-$C_4$-Alkoxy, $C_2$-$C_4$-Alkenyl, $C_2$-$C_4$-Haloalkenyl, $C_2$-$C_4$-Alkinyl, Cyano oder Phenyl substituiertes $C_1$-$C_{12}$-Alkyl bedeutet, wobei jeder cyclische Substituent unsubstituiert oder durch Halogen und/oder Methyl ein- oder mehrfach substituiert ist und wobei ferner jeder $C_2$-$C_{12}$-Alkylsubstituent gegebenenfalls durch eine Carbonylgruppe unterbrochen ist ;
und

$R_5$ Wasserstoff, $C_1$-$C_4$-Alkyl, Phenyl, ein durch Nitro, Halogen und/oder Methyl ein- oder mehrfach substituiertes Phenyl oder Benzyl bedeutet ; unter Einschluss der pflanzenverträglichen Säureadditionssalze, quaternären Azoliumsalze sowie Metallkomplexe der Verbindungen der Formel I*.

7. Mittel gemäß Anspruch 1 enthaltend eine Verbindung der Formel I* gemäss Anspruch 6, worin X für CH oder N steht ; Ar Phenyl bedeutet ; $R_1$, $R_2$, $R_3$ unabhängig voneinander für Halogen, Methyl, Methoxy oder $CF_3$ stehen, n = 0 oder 1 bedeutet und $R_4$ und $R_5$ die für Formel I* angegebenen Bedeutungen haben.

8. Mittel gemäß Anspruch 1 enthaltend eine Verbindung der Formel I* gemäss Anspruch 7, worin X für N steht ; $R_1$, $R_2$, $R_3$ unabhängig voneinander für Fluor, Chlor, Brom, $CH_3$, $CH_3O$ oder $CF_3$ stehen, n die Zahl 0 bedeutet, $R_4$ Wasserstoff, $C_1$-$C_6$ Alkyl, $C_3$-$C_6$ Alkenyl oder $C_3$-$C_6$ Haloalkenyl steht und $R_5$ die unter Formel I* angegebenen Bedeutungen hat.

9. Mittel gemäß Anspruch 1 enthaltend eine Verbindung der Formel I* gemäss Anspruch 6, worin X, Ar, $R_1$, $R_2$, $R_3$ und $R_5$ die unter Formel I* angegebenen Bedeutungen haben, $R_4$ für Wasserstoff oder $C_1$-$C_3$ Alkyl steht und n die Zahl 0 bedeutet.

10. Mittel gemäß Anspruch 1 enthaltend einen 2-(1H-1,2,4-Triazolylmethyl-1'-yl)-2-hydroxy-2,4-dichlorphenylessigsäureester gemäss Anspruch 6 der Formel

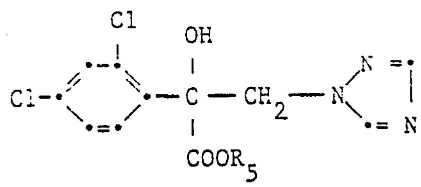

worin $R_5$ Methyl, Ethyl, n-Propyl, Isopropyl, n-Butyl oder tert.-Butyl bedeutet.

11. Mittel gemäß Anspruch 1 enthaltend einen der Metallkomplexe im Umfang der Formel I* nach Anspruch 6 mit den Metallen Kupfer, Zink, Mangan oder Zinn.

12. Verfahren zur Herstellung von Mandelsäure-Derivaten der Formel I gemäss Anspruch 1, dadurch gekennzeichnet, dass man Aryl-azolylmethylketone der Formel IX

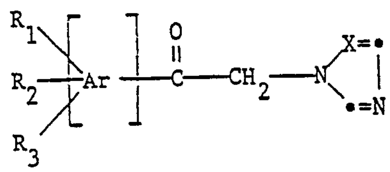

(IX)

mit HCN oder Alkalicyanid bei 0° bis 100 °C reagieren lässt, oder aber Verbindungen der Formel IX in Form ihres $NaHSO_3$-Addukts mit HCN oder Alkylicyanid bei 0° bis 100 °C zur Reaktion bringt und das so gewonnene Mandelonitril der Formel III

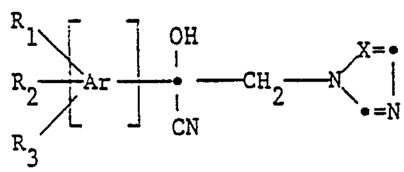

(III)

in einem basischen oder sauren Medium zu Mandelsäure-Derivaten der Formel Ia hydrolysiert (Ia)

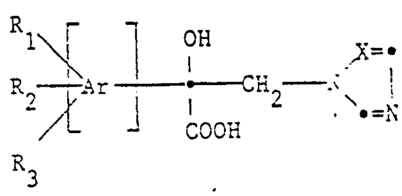

(Ia)

wobei in Formel IX, III und Ia die Substituenten X, Ar, $R_1$, $R_2$ und $R_3$ die unter Formel I angegebenen Bedeutungen haben, und, sofern gewünscht, weitere Derivatisierung der Verbindungen der Formel Ia an der OH-Gruppe oder an der COOH-Gruppe, und/oder, sofern gewünscht, weitere Ueberführung in Säureadditionssalze, quaternäre Azolium- und Ammoniumsalze oder Metallkomplexe.

13. Verfahren zur Herstellung von Mandelsäureestern der Formel Ib

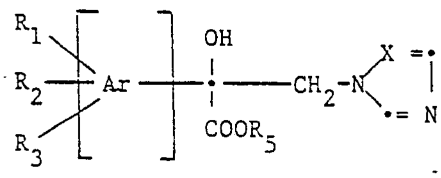

(Ib)

dadurch gekennzeichnet, dass man einen entsprechenden α-Halogenessigester der Formel IV

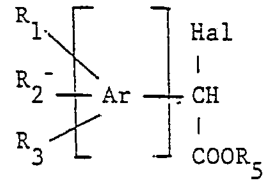

(IV)

mit Paraformaldehyd bei 0° bis 140 °C und a) dem gewünschten Azol der Formel VII

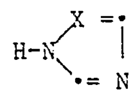

(VII)

in Gegenwart einer Base oder b) mit dem Alkalisalz des Azols in einem wasserfreien Lösungsmittel reagieren lässt, wobei in den Formeln Ib, IV und VII Ar, $R_1$, $R_2$, $R_3$, X und $R_5$ die für Formel I gemäss Anspruch 1 gegebene Bedeutung haben, und Hal für ein Halogenatom steht.

**Claims** (for the Contracting States : DE, FR, CH, LI, IT, NL, BE, SE, LU)

1. A mandelic acid derivative of the formula I

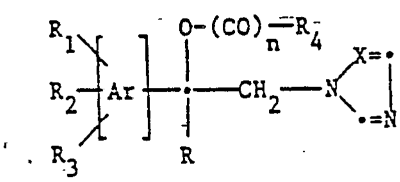

(I)

wherein

X is the bridge member —CH= or —N= ;

Ar is a phenyl, diphenyl or naphthyl group ;

$R_1$, $R_2$ and $R_3$ are each independently hydrogen, nitro, halogen, $C_1$-$C_3$-alkyl, $C_1$-$C_3$-alkoxy or $C_1$-$C_3$-haloalkyl ;

R is one of the groups $—COOR_5$ or $—COSR_6$ ;

n is 0, 1 or 2,

where in the cases in which n is 0

$R_4$ is hydrogen, $C_1$-$C_{12}$-alkyl, $C_3$-$C_6$-alkenyl, $C_3$-$C_6$-haloalkenyl, or $C_1$-$C_{12}$-alkyl substituted by $C_1$-$C_4$-alkoxy, $C_2$-$C_4$-alkenyl, $C_2$-$C_4$-haloalkenyl, $C_2$-$C_4$-alkynyl, cyano or phenyl, and each $C_2$-$C_{12}$-alkyl substituent may be interrupted by a carbonyl group or a carbamoyloxy group (—O—CONH—),

in the cases in which n is 1

$R_4$ is $C_1$-$C_{12}$-alkyl, phenyl, $C_1$-$C_4$-alkoxy, $C_1$-$C_4$-alkylthio, $C_2$-$C_6$-alkenyl, $C_3$-$C_5$-alkynyl, $C_2$-$C_6$-haloalkenyl, $C_3$-$C_7$-cycloalkyl, furyl, tetrahydrofuryl, pyridyl, 1-imidazolyl or 1-(1,2,4-triazolyl), or a phenyl group which is unsubstituted or substituted by halogen, $C_1$-$C_4$-alkyl, $C_1$-$C_4$-alkoxy, —CN or $—CF_3$, or a $C_1$-$C_{12}$-alkyl group substituted by $C_1$-$C_4$-alkoxy, $C_1$-$C_4$-alkylthio, $C_2$-$C_4$-alkenyl, $C_2$-$C_4$-haloalkenyl, $C_2$-$C_4$-alkynyl, cyano or phenyl, each heterocyclic substituent being unsubstituted or mono- or polysubstituted by halogen and/or methyl, and each $C_2$-$C_{12}$-alkyl substituent may be interrupted by a carbonyl group or a carbamoyloxy group, and $R_4$ may also be the group $—N(C_1$-$C_8$-alkyl$)_2$,

and in the cases in which n is 2

$R_4$ is $C_1$-$C_{12}$-alkyl, or a benzyl group which is unsubstituted or substituted by halogen, $C_1$-$C_4$-alkyl, $C_1$-$C_4$-alkoxy, —CN or $—CF_3$ ;

and wherein

$R_5$ is hydrogen, a $C_2$-$C_{10}$-alkenyl group which is substituted by halogen, a $C_2$-$C_{10}$-alkynyl group which is unsubstituted or substituted by halogen, or it is a $C_3$-$C_8$-cycloalkyl group, or a phenyl group which is unsubstituted or substituted by halogen and/or methyl, or it is a $C_1$-$C_4$-alkyl group, or a $C_1$-$C_{12}$-alkyl chain which from $C_2$-alkyl may be interrupted by oxygen or sulfur, and/or which is substituted by one of the following atoms or groups : halogen, phenyl, —COO—($C_1$-$C_4$)-alkyl, —CO—($C_1$-$C_4$)-alkyl, —CO-phenyl, or an unsaturated or saturated 5- or 6-membered ring with oxygen or sulfur as hetero atom, and

$R_6$ is $C_1$-$C_{10}$-alkyl, or a phenyl or benzyl group each unsubstituted or substituted by halogen, $C_1$-$C_4$-alkyl, $C_1$-$C_4$-alkoxy, —CN or $—CF_3$ ;

or an acid addition salt, quaternary azolium or ammonium salt, or metal complex of the formula I.

2. A compound of the formula I according to claim 1, wherein X, Ar, R, $R_1$, $R_2$ and $R_3$ are as defined for formula I, n is 0, and $R_4$ is hydrogen.

3. A compound of the formula I according to claim 2, wherein Ar is a phenyl group, and R is any one of the groups —$COOR_5$ or —$COSR_6$, and X, $R_1$, $R_2$, $R_3$, $R_5$ and $R_6$ are as defined for formula I.

4. A compound of the formula I according to claim 3, wherein Ar is phenyl, $R_1$ is halogen, methyl, methoxy or $CF_3$, $R_2$ and $R_3$ are each independently of the other hydrogen, fluorine, chlorine, bromine, methyl or methoxy, and X, $R_5$ and $R_6$ are as defined for formula I.

5. A compound of the formula I according to claim 4, wherein $R_5$ is hydrogen, $C_1$-$C_4$-alkyl, a phenyl group which is unsubstituted or substituted by halogen, $C_1$-$C_4$-alkyl, $C_1$-$C_4$-alkoxy, —CN or —$CF_3$ ; and $R_6$ is $C_1$-$C_6$-alkyl or a phenyl or benzyl group.

6. A mandelic acid derivative of the formula I*

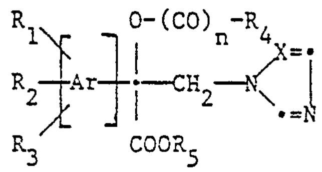

(I*)

wherein

X is the bridge member —CH= or —N= ;

Ar is a phenyl, diphenyl or naphthyl group ;

$R_1$, $R_2$ and $R_3$ are each independently hydrogen, nitro, halogen, $C_1$-$C_3$-alkyl, $C_1$-$C_3$-alkoxy, or $C_1$-$C_3$-haloalkyl ;

n is 0 or 1,

and in the cases in which n is 0

$R_4$ is hydrogen, $C_1$-$C_{12}$-alkyl, $C_3$-$C_6$-alkenyl, $C_3$-$C_6$-haloalkenyl, or $C_1$-$C_{12}$-alkyl substituted by $C_1$-$C_4$-alkoxy, $C_2$-$C_4$-alkenyl, $C_2$-$C_4$-haloalkenyl, $C_2$-$C_4$-alkynyl, cyano or phenyl, and each $C_2$-$C_{12}$-alkyl substituent may be interrupted by a carbonyl group,

and in the cases in which n is 1

$R_4$ is $C_1$-$C_{12}$-alkyl, phenyl, $C_1$-$C_4$-alkoxy, $C_2$-$C_6$-alkenyl, $C_3$-$C_5$-alkynyl, $C_2$-$C_6$-haloalkenyl, $C_3$-$C_7$-cycloalkyl, furyl, tetrahydrofuryl or pyridyl, or a $C_1$-$C_{12}$-alkyl group substituted by $C_1$-$C_4$-alkoxy, $C_2$-$C_4$-alkenyl, $C_2$-$C_4$-haloalkenyl, $C_2$-$C_4$-alkynyl, cyano or phenyl, each cyclic substituent being unsubstituted or mono- or polysubstituted by halogen and/or methyl, and each $C_2$-$C_{12}$-alkyl substituent may be interrupted by a carbonyl group ; and

$R_5$ is hydrogen, $C_1$-$C_4$-alkyl or phenyl, or a phenyl or benzyl group each mono- or polysubstituted by nitro, halogen and/or methyl ;

or an agriculturally acceptable acid addition salt, quaternary azolium salt, or metal complex of a compound of the formula I*.

7. A compound of the formula I* according to claim 6, wherein X is CH or N, Ar is phenyl, $R_1$, $R_2$ and $R_3$ are each independently halogen, methyl, methoxy or $CF_3$, n is 0 or 1, and $R_4$ and $R_5$ are as defined for formula I*.

8. A compound of the formula I* according to claim 7, wherein X is N, $R_1$, $R_2$ and $R_3$ are each independently fluorine, chlorine, bromine, $CH_3$, $CH_3O$ or $CF_3$, n is 0, $R_4$ is hydrogen, $C_1$-$C_6$-alkyl, $C_3$-$C_6$-alkenyl or $C_3$-$C_6$-haloalkenyl, and $R_5$ is as defined for formula I*.

9. A compound of the formula I* according to claim 6, wherein X, Ar, $R_1$, $R_2$, $R_3$ and $R_5$ are as defined for formula I*, $R_4$ is hydrogen or $C_1$-$C_3$-alkyl, and n is 0.

10. A 2-(1H-1,2,4-triazolylmethyl-1'-yl)-2-hydroxy-2,4-dichlorophenylacetic acid ester according to claim 6 of the formula

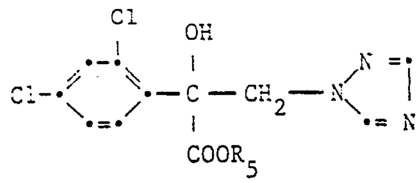

wherein $R_5$ is methyl, ethyl, n-propyl, isopropyl, n-butyl or tert-butyl.

11. A metal complex embraced by the formula I* according to claim 6, with one of the metals copper, zinc, manganese or tin.

12. A process for the preparation of a mandelic acid derivative of the formula I according to claim 1, which process comprises reacting an aryl-azolylmethyl ketone of the formula IX

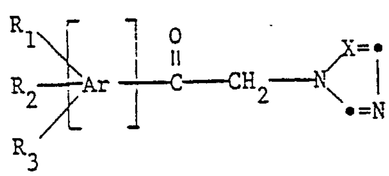

(IX)

with HCN or alkali cyanide at 0° to 100 °C, or reacting a compound of formula XI in the form its $NaHSO_3$ adduct with HCN or alkali cyanide at 0° to 100 °C, and hydrolysing the resultant mandelonitrile of the formula III

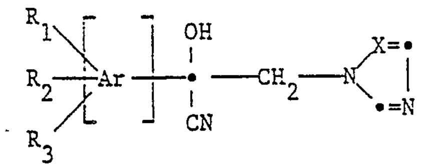

(III)

in a basic or acidic medium, to give a mandelic acid derivative of the formula Ia

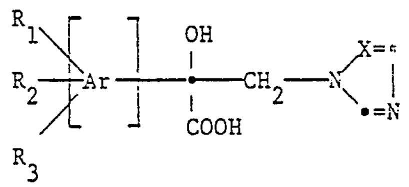

(Ia)

the substituents X, Ar, $R_1$, $R_2$ and $R_3$ in the formulae Ia, III and IX being as defined for formula I, and which process comprises, if desired, further substitution of a compound of the formula Ia at the OH group or at the COOH group, and/or, if desired, further conversion into an acid addition salt, quaternary azolium or ammonium salt or metal complex.

13. A process for the preparation of a mandelic acid ester of the formula Ib

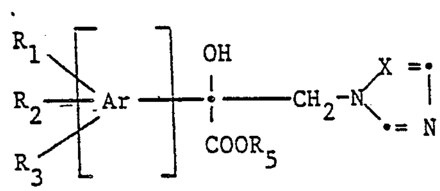

(Ib)

which process comprises reacting a corresponding α-haloacetic acid ester of the formula IV

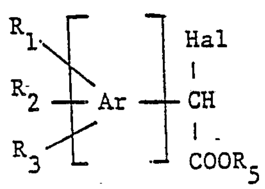

(IV)

with paraformaldehyde at 0° to 140 °C and a) with the desired azole of the formula VII

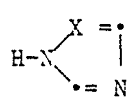

(VII)

in the presence of a base, or b) with the alkali metal salt of the azole, in an anhydrous solvent, the symbols Ar, $R_1$, $R_2$, $R_3$, X and $R_5$ in the formulae Ib, IV and VII being as defined for formula I according to claim 1, and Hal being a halogen atom.

14. A pesticidal composition for controlling microorganisms or preventing attack by said microorganisms, which composition contains as at least one active ingredient a compound of the formula I or I* according to any one of claims I to 11, together with carriers.

15. A method of controlling phytopathogenic microorganisms or of protecting cultivated plants from attack by said phytopathogenic microorganisms, which method comprises applying to the plants to be protected or to the locus thereof an effective amount of a compound of the formula I or I* according to any one of Claims 1 to 11.

**Claims** (for the Contracting State AT)

1. A pesticidal composition for controlling microorganisms or preventing attack by said microorganisms, which composition contains as at least one active ingredient a mandelic acid derivative of the formula I

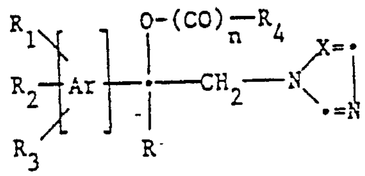

(I)

wherein

X is the bridge member —CH= or —N= ;

Ar is a phenyl, diphenyl or naphthyl group ;

$R_1$, $R_2$ and $R_3$ are each independently hydrogen, nitro, halogen, $C_1$-$C_3$-alkyl, $C_1$-$C_3$-alkoxy or $C_1$-$C_3$-haloalkyl ;

R is one of the groups —$COOR_5$ or —$COSR_6$ ;

n is 0, 1 or 2,

where in the cases in which n is 0

$R_4$ is hydrogen, $C_1$-$C_{12}$-alkyl, $C_3$-$C_6$-alkenyl, $C_3$-$C_6$-haloalkenyl, or $C_1$-$C_{12}$-alkyl substituted by $C_1$-$C_4$-alkoxy, $C_2$-$C_4$-alkenyl, $C_2$-$C_4$-haloalkenyl, $C_2$-$C_4$-alkynyl, cyano or phenyl, and each $C_2$-$C_{12}$-alkyl substituent may be interrupted by a carbonyl group or a carbamoyloxy group (—O—CONH—),

in the cases in which n is 1

$R_4$ is $C_1$-$C_{12}$-alkyl, phenyl, $C_1$-$C_4$-alkoxy, $C_1$-$C_4$-alkylthio, $C_2$-$C_6$-alkenyl, $C_3$-$C_5$-alkynyl, $C_2$-$C_6$-haloalkenyl, $C_3$-$C_7$-cycloalkyl, furyl, tetrahydrofuryl, pyridyl, 1-imidazolyl or 1-(1,2,4-triazolyl), or a phenyl group which is unsubstituted or substituted by halogen, $C_1$-$C_4$-alkyl, $C_1$-$C_4$-alkoxy, —CN or —$CF_3$, or a $C_1$-$C_{12}$-alkyl group substituted by $C_1$-$C_4$-alkoxy, $C_1$-$C_4$-alkylthio, $C_2$-$C_4$-alkenyl, $C_2$-$C_4$-haloalkenyl, $C_2$-$C_4$-alkynyl, cyano or phenyl, each heterocyclic substituent being unsubstituted or mono- or polysubstituted by halogen and/or methyl, and each $C_2$-$C_{12}$-alkyl substituent may be interrupted by a carbonyl group or a carbamoyloxy group, and $R_4$ may also be the group —N($C_1$-$C_8$-alkyl)$_2$,

and in the cases in which n is 2

$R_4$ is $C_1$-$C_{12}$-alkyl, or a benzyl group which is unsubstituted or substituted by halogen, $C_1$-$C_4$-alkyl, $C_1$-$C_4$-alkoxy, —CN or —$CF_3$ ;

and wherein

$R_5$ is hydrogen, a $C_2$-$C_{10}$-alkenyl group which is substituted by halogen, a $C_2$-$C_{10}$-alkynyl group which is unsubstituted or substituted by halogen, or it is a $C_3$-$C_8$-cycloalkyl group, or a phenyl group which is unsubstituted or substituted by halogen and/or methyl, or it is a $C_1$-$C_4$-alkyl group, or a $C_1$-$C_{12}$-alkyl chain which from $C_2$-alkyl may be interrupted by oxygen or sulfur, and/or which is substituted by one of the following atoms or groups : halogen, phenyl, —COO—($C_1$-$C_4$) alkyl, —CO—($C_1$-$C_4$) alkyl, —CO-phenyl, or an unsaturated or saturated 5- or 6- membered ring with oxygen or sulfur as hetero atom, and

$R_6$ is $C_1$-$C_{10}$-alkyl, or a phenyl or benzyl group each unsubstituted or substituted by halogen, $C_1$-$C_4$-alkyl, $C_1$-$C_4$-alkoxy, —CN or —$CF_3$ ;

or an acid addition salt, quaternary azolium or ammonium salt, or metal complex of the formula I, together with carriers.

2. A composition according to claim 1 containing a compound of the formula I, wherein X, Ar, R, $R_1$, $R_2$ and $R_3$ are as defined for formula I, n is 0, and $R_4$ is hydrogen.

3. A composition according to claim 1 containing a compound of the formula I according to claim 2, wherein Ar is a phenyl group, and R is any one of the groups —$COOR_5$ or —$COSR_6$, and X, $R_1$, $R_2$, $R_3$, $R_5$ and $R_6$ are as defined for formula I.

4. A composition according to claim 1 containing a compound of the formula I according to claim 3, wherein Ar is phenyl, $R_1$ is halogen, methyl, methoxy or $CF_3$, $R_2$ and $R_3$ are each independently of the other hydrogen, fluorine, chlorine, bromine, methyl or methoxy, and X, $R_5$ and $R_6$ are as defined for formula I.

5. A composition according to claim 1 containing a compound of the formula I according to claim 4, wherein $R_5$ is hydrogen, $C_1$-$C_4$-alkyl, a phenyl group which is unsubstituted or substituted by halogen, $C_1$-$C_4$-alkyl, $C_1$-$C_4$-alkoxy, —CN or —$CF_3$ ; and $R_6$ is $C_1$-$C_6$-alkyl or a phenyl or benzyl group.

6. A composition according to claim 1 containing a compound of formula I*

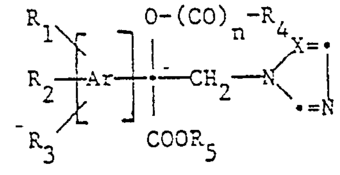

(I*)

wherein

X is the bridge member —CH= or —N= ;

Ar is a phenyl, diphenyl or naphthyl group ;

$R_1$, $R_2$ and $R_3$ are each independently hydrogen, nitro, halogen, $C_1$-$C_3$-alkyl, $C_1$-$C_3$-alkoxy, or $C_1$-$C_3$-haloalkyl ;

n is 0 or 1,

and in the cases in which n is 0

$R_4$ is hydrogen, $C_1$-$C_{12}$-alkyl, $C_3$-$C_6$-alkenyl, $C_3$-$C_6$-haloalkenyl, or $C_1$-$C_{12}$-alkyl substituted by $C_1$-$C_4$-alkoxy, $C_2$-$C_4$-alkenyl, $C_2$-$C_4$-haloalkenyl, $C_2$-$C_4$-alkynyl, cyano or phenyl, and each $C_2$-$C_{12}$-alkyl substituent may be interrupted by a carbonyl group,

and in the cases in which n is 1

$R_4$ is $C_1$-$C_{12}$-alkyl, phenyl, $C_1$-$C_4$-alkoxy, $C_2$-$C_6$-alkenyl, $C_3$-$C_5$-alkynyl, $C_2$-$C_6$-haloalkenyl, $C_3$-$C_7$-cycloalkyl, furyl, tetrahydrofuryl or pyridyl, or a $C_1$-$C_{12}$-alkyl group substituted by $C_1$-$C_4$-alkoxy, $C_2$-$C_4$-alkenyl, $C_2$-$C_4$-haloalkenyl, $C_2$-$C_4$-alkynyl, cyano or phenyl, each cyclic substituent being unsubstituted or mono- or polysubstituted by halogen and/or methyl, and each $C_2$-$C_{12}$-alkyl substituent may be interrupted by a carbonyl group ; and

$R^5$ is hydrogen, $C_1$-$C_4$-alkyl or phenyl, or a phenyl or benzyl group each mono- or polysubstituted by nitro, halogen and/or methyl ;

or an agriculturally acceptable acid addition salt, quaternary azolium salt, or metal complex of a compound of the formula I*.

7. A composition according to claim 1 containing a compound of the formula I* according to claim 6, wherein X is CH or N, Ar is phenyl, $R_1$, $R_2$ and $R_3$ are each independently halogen, methyl, methoxy or $CF_3$, n is 0 or 1, and $R_4$ and $R_5$ are as defined for formula I*.

8. A composition according to claim 1 containing a compound of the formula I* according to claim 7, wherein X is N, $R_1$, $R_2$ and $R_3$ are each independently fluorine, chlorine, bromine, $CH_3$, $CH_3O$ or $CF_3$, n is 0, $R_4$ is hydrogen, $C_1$-$C_6$-alkyl, $C_3$-$C_6$-alkenyl or $C_3$-$C_6$-haloalkenyl, and $R_5$ is as defined for formula I*.

9. A composition according to claim 1 containing a compound of the formula I* according to claim 6, wherein X, Ar, $R_1$, $R_2$, $R_3$ and $R_5$ are as defined for formula I*, $R_4$ is hydrogen or $C_1$-$C_3$-alkyl, and n is 0.

10. A composition according to claim 1 containing a 2-(1H-1,2,4-triazolylmethyl-1'-yl)-2-hydroxy-2,4-dichlorophenylacetic acid ester according to claim 6 of the formula

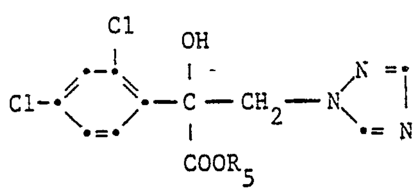

wherein $R_5$ is methyl, ethyl, n-propyl, isopropyl, n-butyl or tert-butyl.

11. A composition according to claim 1 containing a metal complex embraced by the formula I* according to claim 6, with one of the metals copper, zinc, manganese or tin.

12. A process for the preparation of a mandelic acid derivative of the formula I according to claim 1, which process comprises reacting an aryl-azolylmethyl ketone of the formula IX

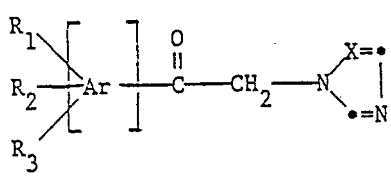

(IX)

with HCN or alkali cyanide at 0° to 100 °C, or reacting a compound of formula XI in the form its $NaHSO_3$ adduct with HCN or alkali cyanide at 0° to 100 °C, and hydrolysing the resultant mandelonitrile of the formula III

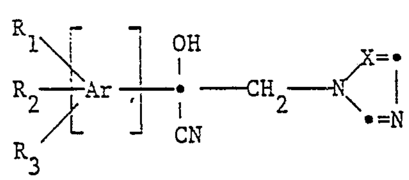

(III)

in a basic or acidic medium, to give a mandelic acid derivative of the formula Ia

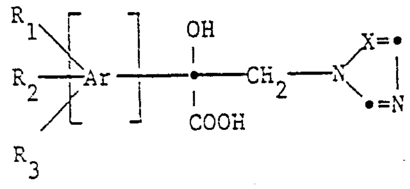

(Ia)

the substituents X, Ar, $R_1$, $R_2$ and $R_3$ in the formulae Ia, III and IX being as defined for formula I, and which process comprises, if desired, further substitution of a compound of the formula Ia at the OH group or at the COOH group, and/or, if desired, further conversion into an acid addition salt, quaternary azolium or ammonium salt or metal complex.

13. A process for the preparation of a mandelic acid ester of the formula Ib

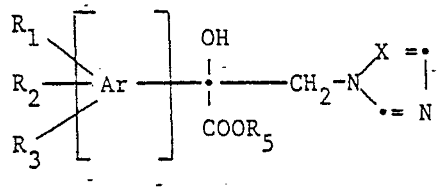

(Ib)

which process comprises reacting a corresponding α-haloacetic acid ester of the formula IV

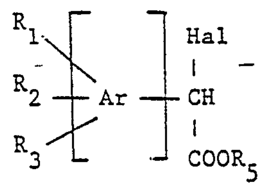

(IV)

with paraformaldehyde at 0° to 140 °C and a) with the desired azole of the formula VII

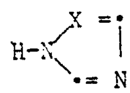

(VII)

in the presence of a base, or b) with the alkali metal salt of the azole, in an anhydrous solvent, the symbols Ar, $R_1$, $R_2$, $R_3$, X and $R_5$ in the formulae Ib, IV and VII being as defined for formula I according to claim 1, and Hal being a halogen atom.

**Revendications** (pour les Etats contractants : DE, FR, CH, LI, IT, NL, BE, SE, LU)

1. Dérivés d'acide mandélique de formule I

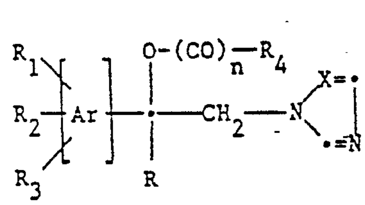

(I)

où

X représente l'élément de pont —CH= ou —N= ;

Ar représente un groupe phényle, diphényle ou naphtyle ;

$R^1$, $R^2$, $R^3$ représentent indépendamment l'un de l'autre hydrogène, nitro, halogène, alcoyle en $C_1$ à $C_3$, alcoxy en $C_1$ à $C_3$ ou haloalcoyle en $C_1$ à $C_3$ ;

R représente l'un des groupes —COOR5 ou —COSR6,

n représente les nombres 0, 1 ou 2,

où dans les cas où n représente le nombre 0,

$R_4$ représente un hydrogène, alcoyle en $C_1$ à $C_{12}$, alcényle en $C_3$ à $C_6$, haloalcényle en $C_3$ à $C_6$ ou un alcoyle en $C_1$ à $C_{12}$ substitué par alcoxy en $C_1$ à $C_4$, alcényle en $C_2$ à $C_4$, haloalcényle en $C_2$ à $C_4$, alcynyle en $C_2$ à $C_4$, cyano ou phényle, où chaque substituant alcoyle en $C_2$ à $C_{12}$ peut être éventuellement interrompu par un groupe carbonyle ou un groupe carbamoyloxy (—O—CONH—) ;

où en outre dans les cas où n représente le nombre 1,

$R_4$ représente un alcoyle en $C_1$ à $C_{12}$, phényle, alcoxy en $C_1$ à $C_4$, alcoylthio en $C_1$ à $C_4$, alcényle en $C_2$ à $C_6$, alcynyle en $C_3$ à $C_5$, haloalcényle en $C_2$ à $C_6$, cycloalcoyle en $C_3$ à $C_7$, furyle, tétrahydrofuryle, pyridyle, 1-imidazoyle, 1-(1,2,4-triazolyle) ou un groupe phényle non substitué ou substitué par halogène, alcoyle en $C_1$ à $C_4$, alcoxy en $C_1$ à $C_4$, —CN ou —$CF_3$ ou un alcoyle en $C_1$ à $C_{12}$ substitué par alcoxy en $C_1$ à $C_4$, alcoylthio en $C_1$ à $C_4$, alcényle en $C_2$ à $C_4$, haloalcényle en $C_2$ à $C_4$, alcynyle en $C_2$ à $C_4$, cyano ou phényle, où chaque substituant hétérocyclique est non substitué ou mono- ou polysubstitué par halogène et/ou méthyle et où en outre chaque substituant alcoyle en $C_2$ à $C_{12}$ peut être éventuellement interrompu par un groupe carbonyle ou un groupe carbamoyloxy ; et où $R_4$ peut en outre représenter le groupe —N(alcoyle en $C_1$ à $C_8$)$_2$ ;

où en outre dans les cas où n représente le nombre 2,

$R_4$ représente un alcoyle en $C_1$ à $C_{12}$ ou un groupe benzyle substitué par halogène, alcoyle en $C_1$ à $C_4$, alcoxy en $C_1$ à $C_4$, —CN ou —$CF_3$,

et où en outre

$R_5$ représente un hydrogène, un alcényle en $C_2$ à $C_{10}$ substitué par halogène, un alcynyle en $C_2$ à $C_{10}$ non substitué ou substitué par halogène, ou un groupe cycloalcoyle en $C_3$ à $C_8$, un groupe phényle non substitué ou substitué par halogène et/ou méthyle ou un groupe alcoyle en $C_1$ à $C_4$, ou encore représente une chaîne alcoyle en $C_1$ à $C_{12}$, qui est interrompue à partir d'alcoyle en $C_2$ par hydrogène ou soufre et/ou est substituée par l'un des atomes ou groupes suivants : halogène, phényle, —COO alcoyle($C_1$-$C_4$), —CO alcoyle($C_1$-$C_4$), —CO phényle, un noyau à 5 ou 6 chaînons non saturé ou saturé avec l'oxygène ou le soufre comme hétéroatome, et

$R_6$ représente un alcoyle en $C_1$ à $C_{10}$ ou un groupe phényle ou benzyle non substitué ou substitué par halogène, alcoyle en $C_1$ à $C_4$, alcoxy en $C_1$ à $C_4$, —CN ou —$CF_3$, et

où les sels d'addition d'acides, sels d'azolium et d'ammonium quaternaires ainsi que complexes métalliques de formule I sont compris, avec des supports.

2. Composés de formule I selon la revendication 1, où X, Ar, R, $R_1$, $R_2$ et $R_3$ ont les significations données par la formule 1, tandis que n = 0 et $R_4$ = hydrogène.

3. Composés de formule I selon la revendication 2, où Ar représente un groupe phényle et R représente l'un des groupes —COOR5 ou —COSR6, où X, R1, R2, R3, R5 et R6 ont la signification donnée pour la formule I.

4. Composés de formule I selon la revendication 3, où Ar représente un phényle, R1 représente un halogène, méthyle, méthoxy ou $CF_3$ et R2 et R3 représentent indépendamment l'un de l'autre un hydrogène, fluor, chlore, brome, méthyle ou méthoxy, tandis que X, R5 et R6 ont la signification donnée pour la formule I.

5. Composés de formule I selon la revendication 4, où R5 représente un hydrogène, un alcoyle en C1 à C4, un groupe phényle non substitué ou substitué par un halogène, alcoyle en C1 à C4, alcoxy en C1 à C4, —CN ou $CF_3$ ; et R6 représente un alcoyle en C1 à C6 ou un groupe phényle ou benzyle.

6. Dérivés de l'acide mandélique de formule I*

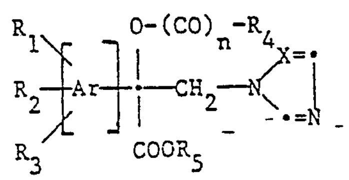

(I*)

où

X représente l'élément de pont —CH= ou —N=

Ar représente un groupe phényle, diphényle ou naphtyle ;

R1, R2, R3 représentent indépendamment l'un de l'autre hydrogène, nitro, halogène, alcoyle en C1 à C3, alcoxy en C1 à C3 ou halo-alcoyle en C1 à C3 ;

n représente les nombres 0 ou 1

et dans les cas où n représente le nombre 0,

R4 représente un hydrogène, alcoyle en C1 à C12, haloalcényle en C3 à C6 ou un alcoyle en C1 à C12 substitué par alcoxy en C1 à C4, alcényle en C2 à C4, haloalcényle en C2 à C4, alcynyle en C2 à C4, cyano ou phényle, où chaque substituant alcoyle en C2 à C12 est éventuellement interrompu par un groupe carbonyle ;

et dans les cas où n représente le nombre 1,

R4 représente un alcoyle en C1 à C12, phényle, alcoxy en C1 à C4, alcényle en C2 à C6, alcynyle en C3 à C5, haloalcényle en C2 à C6, cycloalcoyle en C3 à C7, furyle, tétrahydrofuryle, pyridyle ou un alcoyle en C1 à C12 substitué par un alcoxy en C1 à C4, alcényle en C2 à C4, haloalcényle en C2 à C4, alcynyle en C2 à C4, cyano ou phényle, où chaque substituant cyclique est non substitué ou mono- ou polysubstitué par un halogène et/ou un méthyle et où en outre chaque substituant alcoyle en C2 à C12 est éventuellement interrompu par un groupe carbonyle ; et

R5 représente un hydrogène, un alcoyle en C1 à C4, un phényle, un phényle ou benzyle mono- ou

polysubstitué par nitro, halogène et/ou méthyle ; y compris les sels d'addition d'acides phytocompatibles, sels d'azolium quaternaires ainsi que complexes métalliques des composés de formule I*.

7. Composés de formule I* selon la revendication 6, où X représente CH ou N ; Ar représente un phényle ; R1, R2, R3 représentent indépendamment l'un de l'autre halogène, méthyle, méthoxy ou $CF_3$, n = 0 ou 1 et R4 et R5 ont les significations indiquées pour la formule I*.

8. Composés de formule I* selon la revendication 7, où X représente N ; R1, R2, R3 représentent indépendamment l'un de l'autre un fluor, chlore, brome, $CH_3$, $CH_3O$ ou $CF_3$, n représente le nombre 0, R4 représente un hydrogène, alcoyle en C1 à C6, alcényle en C3 à C6 ou haloalcényle en C3 à C6 et R5 a les significations données pour la formule I*.

9. Composés de formule I* selon la revendication 6, où X, Ar, R1, R2, R3 et R5 ont les significations données sous la formule I*, R4 représente un hydrogène ou un alcoyle en C1 à C3 et n représente le nombre 0.

10. Ester de l'acide 2-(1H-1,2,4-triazolylméthyl-1'-yl)-2-hydroxy-2,4-dichlorophénylacétique selon la revendication 6 de formule

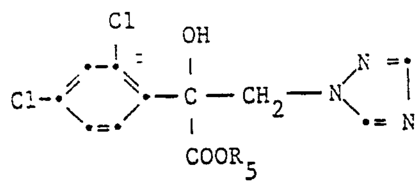

où R5 représente un méthyle, éthyle, n-propyle, isopropyle, n-butyle ou tert.-butyle.

11. Les complexes métalliques dans le cadre de la formule I* selon la revendication 6 avec les métaux cuivre, zinc, manganèse ou étain.

12. Procédé de préparation de dérivés d'acide mandélique de formule I selon la revendication 1, caractérisé en ce qu'on fait réagir des arylazolylméthylcétones de formule IX

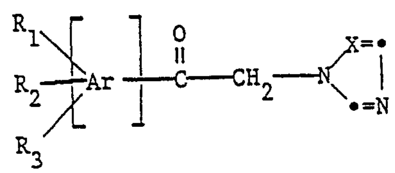

(IX)

avec HCN ou un cyanure alcalin entre 0° et 100 °C, ou encore en ce qu'on fait réagir des composés de formule IX sous la forme de leurs produits d'addition de $NaHSO_3$ avec HCN ou un cyanure d'alcoyle entre 0° et 100° C et en ce qu'on hydrolyse le mandélonitrile ainsi obtenu de formule III

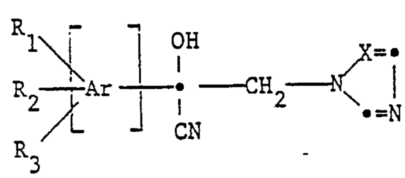

(III)

dans un milieu basique ou acide en dérivés d'acide mandélique de formule Ia

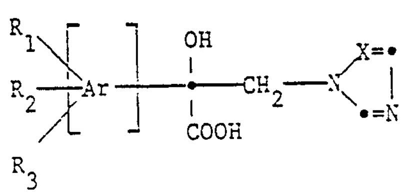

(Ia)

où dans les formules IX, III et Ia les substituants X, Ar, R1, R2 et R3 ont les significations données pour la formule I, et, si on le désire, en ce qu'on procède à une transformation ultérieure des composés de formule Ia sur le groupe OH ou sur le groupe COOH, et/ou, si on le désire, à des transformations ultérieures en sels d'addition d'acides, sels d'azolium et d'ammonium quaternaires ou complexes métalliques.

13. Procédé de préparation d'esters d'acide mandélique de formule Ib

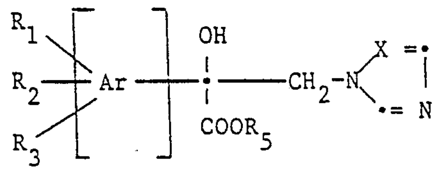

(Ib)

caractérisé en ce qu'on fait réagir un ester α-halogénacétique correspondant de formule IV

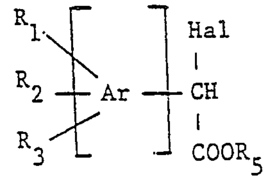

(IV)

avec du paraformaldéhyde entre 0° et 140 °C et a) l'azole désiré de formule VII

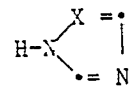

(VII)

en présence d'une base ou b) avec le sel alcalin de l'azole dans un solvant anhydre, où dans les formules Ib, IV et VII Ar, R1, R2, R3, X et R5 ont la signification donnée pour la formule I selon la revendication 1, et Hal représente un atome d'halogène.

14. Agent de lutte antiparasitaire pour combattre ou prévenir une attaque par des microorganismes, caractérisé en ce qu'il contient au moins un composant actif, à savoir un composé de formule I ou selon les cas I* selon l'une des revendications 1 à 11, avec des supports.

15. Procédé pour combattre ou prévenir une attaque de plantes cultivées par des microorganismes phytopathogènes, caractérisé en ce qu'on applique un composé de formule I ou selon les cas I* sur les plantes ou l'endroit où elles se trouvent.

**Revendications** (pour l'Etat contractant AT)

1. Agent de lutte antiparasitaire pour combattre ou prévenir une attaque par des microorganismes, caractérisé en ce qu'il contient au moins un composant actif, à savoir un dérivé d'acide mandélique de formule I

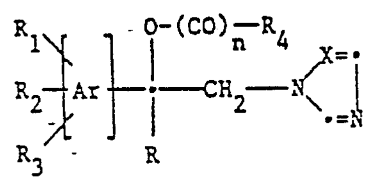

(I)

où

X représente l'élément de pont —CH= ou —N= ;

Ar représente un groupe phényle, diphényle ou naphtyle ;

R1, R2, R3 représentent indépendamment l'un de l'autre hydrogène, nitro, halogène, alcoyle en C1 à C3, alcoxy en C1 à C3 ou haloalcoyle en C1 à C3 ;

R représente l'un des groupes —COOR5 ou —COSR6,

n représente les nombres 0, 1 ou 2,

où dans les cas où n représente le nombre 0,

R4 représente un hydrogène, alcoyle en C1 à C12, alcényle en C3 à C6, haloalcényle en C3 à C6 ou un alcoyle en C1 à C12 substitué par alcoxy en C1 à C4, alcényle en C2 à C4, haloalcényle en C2 à C4, alcynyle en C2 à C4, cyano ou phényle, où chaque substituant alcoyle en C2 à C12 peut être éventuellement interrompu par un groupe carbonyle ou un groupe carbamoyloxy (—O—CONH—) ;

où en outre dans les cas où n représente le nombre 1,

R4 représente un alcoyle en C1 à C12, phényle, alcoxy en C1 à C4, alcoylthio en C1 à C4, alcényle en C2 à C6, alcynyle en C3 à C5, haloalcényle en C2 à C6, cycloalcoyle en C3 à C7, furyle, tétrahydrofuryle, pyridyle, 1-imidazolyle, 1-(1,2,4-triazolyle) ou un groupe phényle non substitué ou substitué par halogène, alcoyle en C1 à C4, alcoxy en C1 à C4, —CN ou —$CF_3$ ou un alcoyle en C1 à C12 substitué par alcoxy en C1 à C4, alcoylthio en C1 à C4, alcényle en C2 à C4, haloalcényle en C2 à C4, alcynyle en C2 à C4, cyano

ou phényle, où chaque substituant hétérocyclique est non substitué ou mono- ou polysubstitué par halogène et/ou méthyle et où en outre chaque substituant alcoyle en C2 à C12 peut être éventuellement interrompu par un groupe carbonyle ou un groupe carbamoyloxy ; et où R4 peut en outre représenter le groupe —N(alcoyle en C1 à C8)$_2$ ;

où en outre dans les cas où n représente le nombre 2,

R4 représente un alcoyle en C1 à C12 ou un groupe benzyle substitué par halogène, alcoyle en C1 à C4, alcoxy en C1 à C4, —CN ou —$CF_3$,

et où en outre

R5 représente un hydrogène, un alcényle en C2 à C10 substitué par halogène, un alcynyle en C2 à C10 non substitué ou substitué par halogène, un groupe cycloalcoyle en C3 à C8, un groupe phényle non substitué ou substitué par halogène et/ou méthyle ou un groupe alcoyle en C1 à C4, ou encore représente une chaîne alcoyle en C1 à C12, qui est interrompue à partir d'alcoyle en C2 par hydrogène ou soufre et/ou est substituée par l'un des atomes ou groupes suivants : halogène, phényle, —COO alcoyle(C1-C4), —CO alcoyle(C1-C4), —CO phényle, un noyau à 5 ou 6 chaînons non saturé ou saturé avec l'oxygène ou le soufre comme hétéroatome ; et

R6 représente un alcoyle en C1 à C10 ou un groupe phényle ou benzyle non substitué ou substitué par halogène, alcoyle en C1 à C4, alcoxy en C1 à C4, —CN ou —$CF_3$, et

où les sels d'addition d'acides, sels d'azolium et d'ammonium quaternaires ainsi que complexes métalliques de formule I sont compris.

2. Agent selon la revendication 1 contenant un composé de formule I selon la revendication 1, où X, Ar, R, R1, R2, R3 ont les significations données pour la formule I, tandis que n = 0 et R4 = hydrogène.

3. Agent selon la revendication 1 contenant un composé de formule I selon la revendication 2, où Ar représente un groupe phényle et R représente l'un des groupes —COOR5 ou —COSR6, où X, R1, R2, R3, R5 et R6 ont la signification donnée pour la formule I.

4. Agent selon la revendication 1 contenant un composé de formule I selon la revendication 3, où Ar représente un phényle, R1 représente un halogène, méthyle, méthoxy ou $CF_3$ et R2 et R3 représentent indépendamment l'un de l'autre un hydrogène, fluor, chlore, brome, méthyle ou méthoxy, tandis que X, R5 et R6 ont la signification donnée pour la formule I.

5. Agent selon la revendication 1 contenant un composé de formule I selon la revendication 4, où R5 représente un hydrogène, un alcoyle en C1 à C4, un groupe phényle non substitué ou substitué par un halogène, alcoyle en C1 à C4, alcoxy en C1 à C4, —CN ou $CF_3$ ; et R6 représente un alcoyle en C1 à C6 ou un groupe phényle ou benzyle.

6. Agent selon la revendication 1 contenant un composé de formule I*

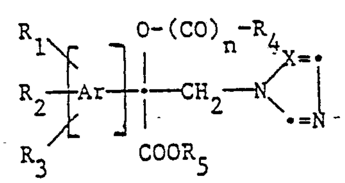

(I*)

où

X représente l'élément de pont —CH= ou —N=

Ar représente un groupe phényle, diphényle ou naphtyle ;

R1, R2, R3 représentent indépendamment l'un de l'autre hydrogène, nitro, halogène, alcoyle en C1 à C3, alcoxy en C1 à C3 ou haloalcoyle en C1 à C3 ;

n représente les nombres 0 ou 1 et dans les cas où n représente le nombre 0, R4 représente un hydrogène, alcoyle en C1 à C12, haloalcényle en C3 à C6 ou un alcoyle en C1 à C12 substitué par alcoxy en C1 à C4, alcényle en C2 à C4, haloalcényle en C2 à C4, alcynyle en C2 à C4, cyano ou phényle, où chaque substituant alcoyle en C2 à C12 est éventuellement interrompu par un groupe carbonyle ;

et dans les cas où n représente le nombre 1,

R4 représente un alcoyle en C1 à C12, phényle, alcoxy en C1 à C4, alcényle en C2 à C6, alcynyle en C3 à C5, haloalcényle en C2 à C6, cycloalcoyle en C3 à C7, furyle, tétrahydrofuryle, pyridyle ou un alcoyle en C1 à C12 substitué par un alcoxy en C1 à C4, alcényle en C2 à C4, haloalcényle en C2 à C4, alcynyle en C2 à C4, cyano ou phényle, où chaque substituant cyclique est non substitué ou mono- ou polysubstitué par un halogène et/ou un méthyle et où en outre chaque substituant alcoyle en C2 à C12 est éventuellement interrompu par un groupe carbonyle ; et

R5 représente un hydrogène, un alcoyle en C1 à C4, un phényle, un phényle ou benzyle mono- ou polysubstitué par nitro, halogène et/ou méthyle ; y compris les sels d'addition d'acides phytocompatibles, sels d'azolium quaternaires ainsi que complexes métalliques des composés de formule I*.

7. Agent selon la revendication 1 contenant un composé de formule I* selon la revendication 6, où X représente CH ou N ; Ar représente un phényle ; R1, R2, R3 représentent indépendamment l'un de l'autre halogène, méthyle, méthoxy ou $CF_3$, n = 0 ou 1 et R4 et R5 ont les significations indiquées pour la formule I*.

8. Agent selon la revendication 1 contenant un composé de formule I* selon la revendication 7, où X

représente N ; R1, R2, R3 représentent indépendamment l'un de l'autre un fluor, chlore, brome, $CH_3$, $CH_3O$ ou $CF_3$, n représente le nombre 0, R4 représente un hydrogène, alcoyle en C1 à C6, alcényle en C3 à C6 ou haloalcényle en C3 à C6 et R5 a les significations données pour la formule I*.

9. Agent selon la revendication 1 contenant un composé de formule I* selon la revendication 6, où X, Ar, R1, R2, R3 et R5 ont les significations données sous la formule I*, R4 représente un hydrogène ou un alcoyle en C1 à C3 et n représente le nombre 0.

10. Agent selon la revendication 1 contenant un ester de l'acide 2-(1H-1,2,4-triazolylméthyl-1'-yl)-2-hydroxy-2,4-dichlorophénylacétique selon la revendication 6 de formule

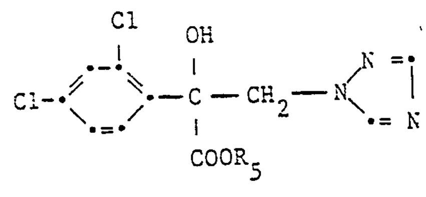

où R5 représente un méthyle, éthyle, n-propyle, isopropyle, n-butyle ou tert.-butyle.

11. Agent selon la revendication 1 contenant un des complexes métalliques dans le cadre de la formule I* selon la revendication 6 avec les métaux cuivre, zinc, manganèse ou étain.

12. Procédé de préparation de dérivés d'acide mandélique de formule I selon la revendication 1, caractérisé en ce qu'on fait réagir des arylazolylméthylcétones de formule IX

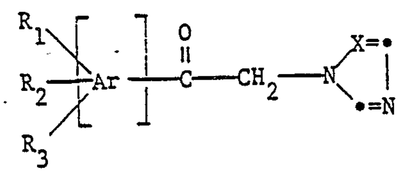

(IX)

avec HCN ou un cyanure alcalin entre 0° et 100 °C, ou encore en ce qu'on fait réagir des composés de formule IX sous la forme de leurs produits d'addition de $NaHSO_3$ avec HCN ou un cyanure d'alcoyle entre 0° et 100 °C et en ce qu'on hydrolyse le mandélonitrile ainsi obtenu de formule III

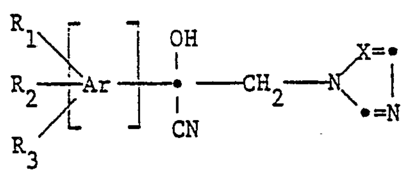

(III)

dans un milieu basique ou acide en dérivés d'acide mandélique de formule Ia

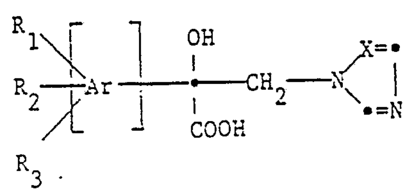

(Ia)

où dans les formules IX, III et Ia les substituants X, Ar, R1, R2 et R3 ont les significations données pour la formule I, et, si on le désire, en ce qu'on procède à une transformation ultérieure des composés de formule Ia sur le groupe OH ou sur le groupe COOH, et/ou, si on le désire, à des transformations ultérieures en sels d'addition d'acides, sels d'azolium et d'ammonium quaternaires ou complexes métalliques.

13. Procédé de préparation d'esters d'acide mandélique de formule Ib

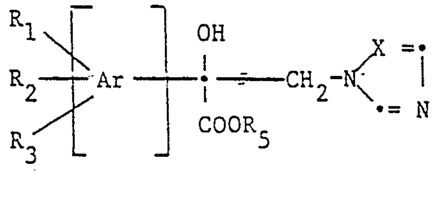

(Ib)

caractérisé en ce qu'on fait réagir un ester α-halogénacétique correspondant de formule IV

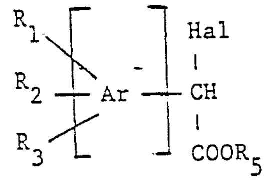

(IV)

avec du paraformaldéhyde entre 0° et 140 °C et a) l'azole désiré de formule VII

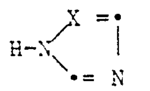

(VII)

en présence d'une base ou b) avec le sel alcalin de l'azole dans un solvant anhydre, où dans les formules Ib, IV et VII Ar, R1, R2, R3, X et R5 ont la signification donnée pour la formule I selon la revendication 1, et Hal représente un atome d'halogène.